# EUROPEAN PATENT SPECIFICATION

(11) **EP 1 820 854 B1**
(45) Date of publication and mention of the grant of the patent: **28.07.2010**
(21) Application number: 07008869.5
(22) Date of filing: 05.10.1998
(51) Int. Cl.: C12N 15/00, C12N 15/09, C12N 15/63, C12N 5/00, A01N 43/04, A61K 31/70

(54) **JNK3 modulators and methods of use**
JNK3-Modulatoren und Verwendungsverfahren dafür
Modulateurs JNK3 et procédés d'utilisation

(30) Priority: 03.10.1997 US 60995 P
(43) Date of publication of application: 22.08.2007
(62) Divisional of application: 98954937.3
(73) Proprietor: University of Massachusetts, Boston, MA 02110 (US)
(72) Inventor: Davis, Roger J., Princeton MA 01541 (US); Flavell, Richard A., Guilford CT 06437 (US); Rakic, Pasko, New Haven CT 06511-2313 (US); Whitmarsh, Alan J., Manchester M14 5LX (GB); Kuan, Chia-Yin c/o Children`s Hospital Research Foundation, Cincinnati, OH 45229 (US); Yang, Derek Di, Carmel IN 46033 (US)
(74) Representative: Grund, Martin

(56) References cited:
- US-A- 5 605 808
- GUPTA S ET AL: "SELECTIVE INTERACTION OF JNK PROTEIN KINASE ISOFORMS WITH TRANSCRIPTION FACTORS" EMBO JOURNAL, OXFORD UNIVERSITY PRESS, SURREY, GB, vol. 15, no. 11, 27 June 1997 (1997-06-27), pages 2760-2770, XP002915273 ISSN: 0261-4189
- MOHIT A A ET AL: "P49SF12 KINASE: A NOVEL MAP KINASE EXPRESSED IN A SUBSET OF NEURONS IN THE HUMAN NERVOUS SYSTEM" NEURON, CAMBRIDGE, MA, US, vol. 14, no. 1, January 1995 (1995-01), pages 67-78, XP008006032
- MARTIN J H ET AL: "Developmental expression in the mouse nervous system of the p493F12 SAP kinase." BRAIN RESEARCH. MOLECULAR BRAIN RESEARCH JAN 1996, vol. 35, no. 1-2, January 1996 (1996-01), pages 47-57, XP009089599 ISSN: 0169-328X
- WOODGETT J R ET AL: "The stress activated protein kinase pathway." CANCER SURVEYS, vol. 27, 1996, pages 127-138, XP001105173 1996 Cold Spring Harbor Laboratory Press 10 Skyline Drive, Plainview, New York 11803, USA ISBN: 0-87969-484-X
- YAN ZHANG ET AL: "A splicing variant of a death domain protein that is regulated by a mitogen-activated kinase is a substrate for c-Jun N-terminal kinase in the human central nervous system." PROCEEDINGS OF THE NATIONAL ACADEMY OF SCIENCES OF THE UNITED STATES, vol. 95, no. 5, 3 March 1998 (1998-03-03), pages 2586-2591, XP002214181 March 3, 1998 ISSN: 0027-8424
- YANG DEREK D ET AL: "Absence of excitotoxicity-induced apoptosis in the hippocampus of mice lacking the Jnk3 gene." NATURE (LONDON), vol. 389, no. 6653, 23 October 1997 (1997-10-23), pages 865-870, XP002214182 ISSN: 0028-0836

## Description

### Field of the Invention

The invention relates to screening assays for the detection of inhibitors of protein kinase expression or activity.

### Background of the Invention

Apoptosis, or programmed cell death, is a prominent feature of the nervous system during normal development and in adult brain exposed to environmental stress (Kuida et al., Nature, 384:368-372, 1996; Ratan et al., Neurochem., 62:376-379, 1994; Raff et al., Science, 262:695-700, 1993). Stress-induced apoptosis has been implicated in a variety of neurological diseases (Thompson, Science, 267:1456-1462, 1995) and requires de novo protein and RNA synthesis (Martin et al., J. Cell. Biol., 106:829-844, 1988; Oppenheim et al., Dev. Biol., 138:104-113, 1990). Increased expression of c-Jun protein is associated with neuronal damage following global ischemia (Neumann-Haefelin et al., Cerebral Flow Metab,, 14:206-216, 1994) or transection of nerve axons *in vivo* (Neumann-Haefelin, *supra*). Increased expression and phosphorylation of c-Jun have been observed *in vitro* prior to the apoptotic death of sympathetic neurons deprived of nerve growth factor (NGF) (Ham et al., Neuron, 14:927-939, 1995). Moreover, expression of a dominant negative mutant c-Jun, or treatment with c-Jun antibody protects NGF-deprived sympathetic neurons from apoptosis (Ham et al., *supra;* Estus et al., J. Cell. Biol., 127:1717-1727, 1994). However, the requirement of c-Jun for stress-induced neuronal apoptosis has not been tested *in vivo* since c-Jun deficient mice die during mid-gestation (Hilberg et al., Nature, 365:179-181, 1993).

Protein phosphorylation is one important mechanism involved in the activation of c-Jun in response to environmental stress signals (Whitmarsh et al., J. Mol. Med., 74:589-607, 1996). c-Jun N-terminal kinase (JNK, also known as SAPK) is a serine/threonine protein kinase that phosphorylates two residues (Ser-63 and Ser-73) on the NH₂-terminal activation domain of c-Jun (Whitmarsh et al., *supra;* Dèrijard et al., Cell, 76:1025-1037, 1994; Kyriakis et al., Nature, 369:156-160, 1994 US 5,605,808). Map kinase kinase
(MKK) 4 (also known as SEK1) is a direct activator of JNK in response to environmental stresses and mitogenic factors (Whitmarsh et al, *supra*; Dèrijard et al, *supra;* Nishina et al., Nature, 385:350-353, 1997; Yang et al., Proc. Nat. Acad. Sci. USA, 94:3004-3009, 1997; Sanchez et al., Nature, 372:794-798, 1994). JNK also phosphorylates ATF2 and other Jun-family proteins which function as components of the AP-1 transcription factor complex (Whitmarsh et al., *supra;* Gupta et al., Science, 267:389-393, 1995; Gupta et al., EMBO J., 15:2760-2770, 1996). The phosphorylation of these transcription factors by JNK leads to increased AP-1 transcriptional activity (Whitmarsh et al., *supra*). Conversely, the induction of AP-1 transcriptional activity is selectively blocked in cells lacking MKK4 (Yang et al., *supra).*

JNK has been implicated in the apoptosis of NGF-differentiated PC12 pheochromocytoma cells (Xia et al., Science, 270:1326-1311, 1995), one model system of neuronal cell death *in vivo* (Batistatou et al., J. Cell. Biol., 122:523-532, 1993). When differentiated PC12 cells are deprived of nerve growth factor (NGF), JNK activation is observed prior to apoptotic death (Xia et al., *supra*). Transfection studies using constitutively activated and dominant negative mutant components of the JNK signaling pathway established that JNK is involved in NGF withdrawal-induced apoptosis of PC12 cells (Xia et al., *supra*).

Ten JNK isoforms, resulting from alternative splicing of three different genes have been identified (Dèrijard et al., *supra;* Kyriakis et al., *supra;* Gupta et al., *supra*; Martin et al., Brain Res. Mol. Brain Res., 35:47-57, 1996). Although the JNK1 and JNK2 isoforms are widely expressed in murine tissues, including the brain, the JNK3 isoforms are predominantly expressed in the brain and, to a lesser extent, in the heart and testis.

### Summary of the Invention

The invention relates to methods as defined in the appended claims. The invention is based on the discovery that mice lacking the JNK3 gene (JNK3(-/-)) develop normally and are resistant to excitotoxic damage, and that JNK3 plays a role in stress-induced seizure activity, AP-1 transcriptional activation, and kainate-induced apoptosis of hippocampal neurons. Thus, JNK3 is a mediator of kainate/glutamate excitotoxicity and a target for limiting or preventing excitotoxic damage.

The description also features a method of identifying a candidate compound that modulates JNK3 expression. The method includes the steps of incubating a cell that can express a JNK3 protein with a compound under conditions and for a time sufficient for the cell to express the JNK3 protein when the candidate compound is not present. The expression of JNK3 is then measured in the cell in the presence of the compound. The expression of JNK3 is also measured in a control cell under the same conditions and for the same time. The amount of JNK3 expression in the cell incubated in the presence of the compound and in the control cell is compared. A difference in JNK3 expression indicates that the compound modulates JNK3 expression. In an embodiment of this method, the compound decreases JNK3 expression. Disclosed is also a method of identifying a candidate compound that modulates JNK3 activity. The method includes the steps of incubating a cell that has JNK3 activity with a compound under conditions and for a time sufficient for the cell to express JNK3 activity when the candidate compound is not present. The activity of JNK3 is then measured in the cell in the presence of the compound. The activity of JNK3 is also measured in a control cell under the same conditions and for the same time. The amount of JNK3 activity in the cell incubated in the presence of the compound and in the control cell is compared. A difference in JNK3 activity indicates that the compound modulates JNK3 activity. In an embodiment of this method, the compound decreases JNK3 activity.

Disclosed is also a method of identifying a compound that modulates the binding of a JNK3 polypeptide to a substrate. The method involves comparing the amount of a JNK3 polypeptide bound to a substrate in the presence and absence of a selected compound. A difference in the amount of binding of a JNK3 polypeptide to the substrate indicates that the selected compound modulates the binding of a JNK3 polypeptide. In an embodiment of this method, the binding of a JNK3 polypeptide to a substrate is decreased.

Disclosed is also a method for generating a totipotent mouse cell comprising at least one inactivated JNK3 gene. The method includes: a) providing a plurality of totipotent mouse cells; b) introducing into the cells a DNA construct that includes a mouse JNK3 gene disrupted by the insertion of a sequence into the gene, thus the disruption prevents expression of functional JNK3; c) incubating the cells so that homologous recombination occurs between the chromosomal sequence encoding JNK3 and the introduced DNA construct; and d) identifying a totipotent mouse cell that has at least one inactivated JNK gene.

Disclosed is also a method for generating a mouse homozygous for an inactivated JNK3 gene. The method includes the steps of: a) providing a totipotent mouse cell that contains at least one inactivated JNK3 gene; b) inserting the cell into a mouse embryo and implanting the embryo into a female mouse; c) permitting the embryo to develop into a neonatal mouse; d) permitting the neonatal mouse to reach sexual maturity; e) mating two of the sexually mature mice to obtain a mouse homozygous for the inactivated JNK3 gene. Such a mouse (homozygous JNK3(-/-)) is resistant to excitotoxic damage.

Disclosed are also methods of treating a patient having or at risk for a disorder of the nervous system involving excitotoxicity. The methods include administering to the patient a therapeutically effective amount of a compound that inhibits JNK3 expression, or a therapeutically effective amount of a compound that inhibits JNK3 activity. An antisense nucleic acid molecule or ribozyme can be used as the inhibitory compound. Disorders that can be treated by these methods include dementias including Alzheimer's disease, neurodegenerative diseases such as Huntington disease, cerebrovascular disorders such as ischemia, amyotrophic lateral sclerosis, trauma including that caused by heat or cold, motor neuron disease, Parkinson's disease, or seizure disorders including epilepsy. Neuroendocrine disorders such as those that affect pituitary glands, adrenal glands, testis, or pancreas (e.g., β-cells) can be treated with JNK3 modulators.

Disclosed is also a transgenic non-human mammal having a transgene disrupting expression of a JNK3 gene, the transgene being chromosomally integrated into germ cells of the mammal. The mammal may be a mouse. The germ cells of the mammal can be homozygous for the transgene and the disruption of JNK3 gene expression can be the result of a null mutation. Disclosed is also a cell line descended from a cell of the mammal having the transgene disrupting expression of a JNK3 gene.

A DNA construct comprising a disrupted mouse JNK3 gene is also featured. The disruption is by insertion of a sequence into the gene such that the disruption prevents or modifies the expression of functional JNK3.

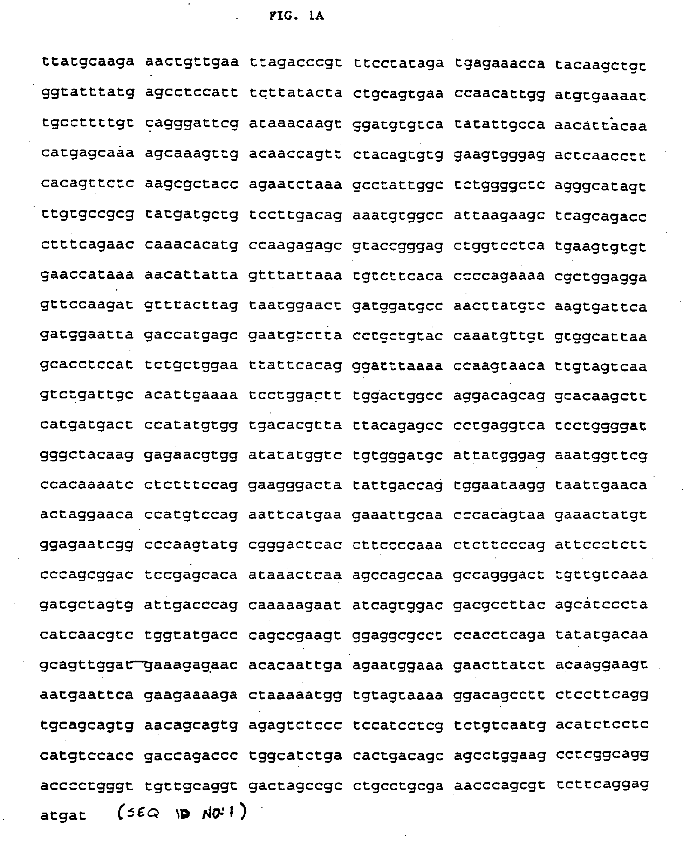

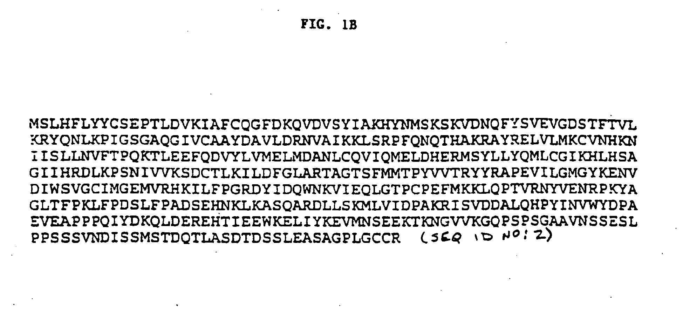

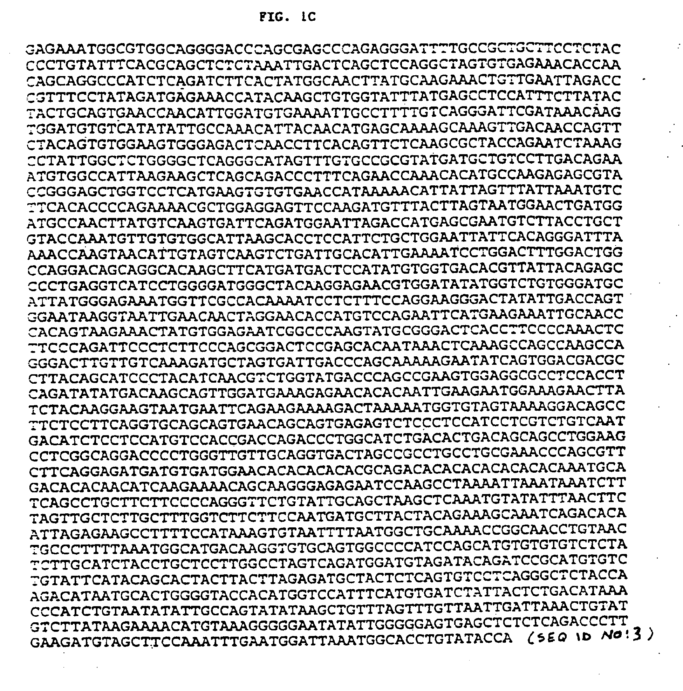

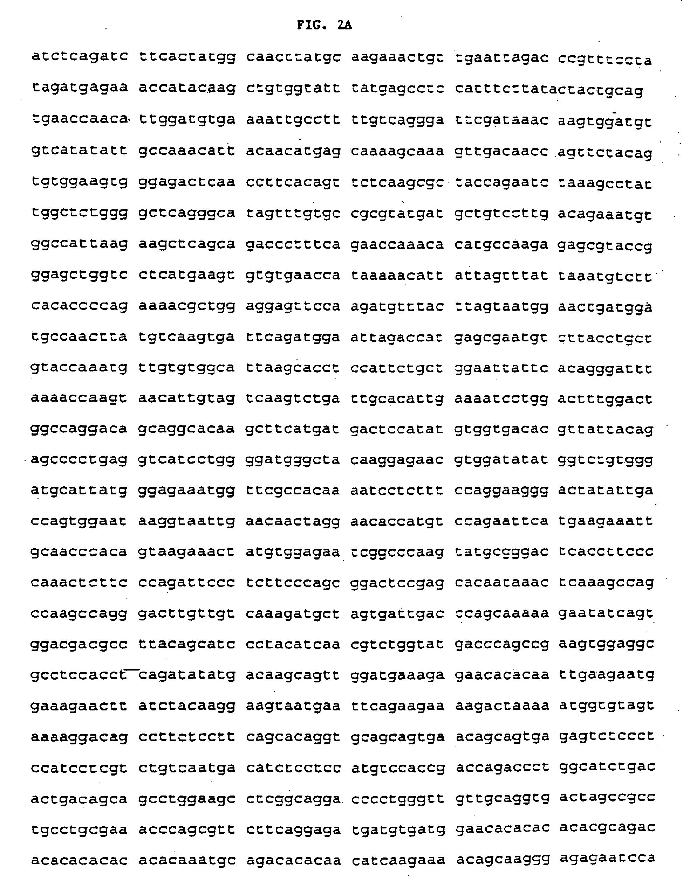

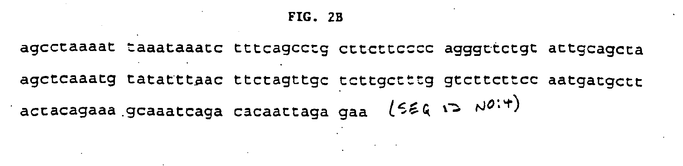

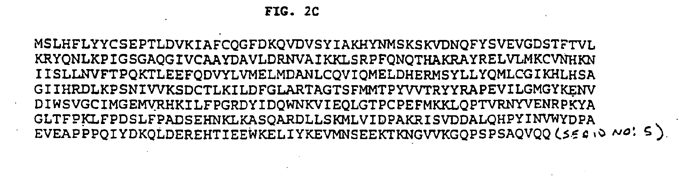

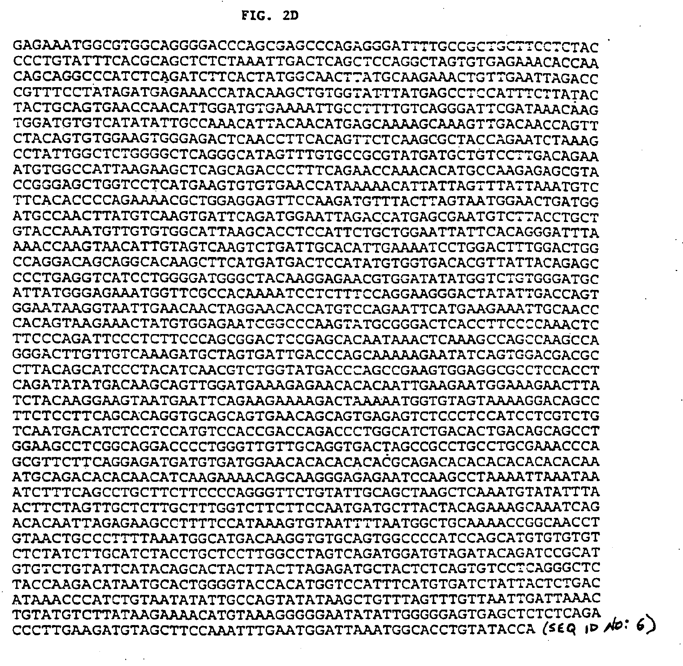

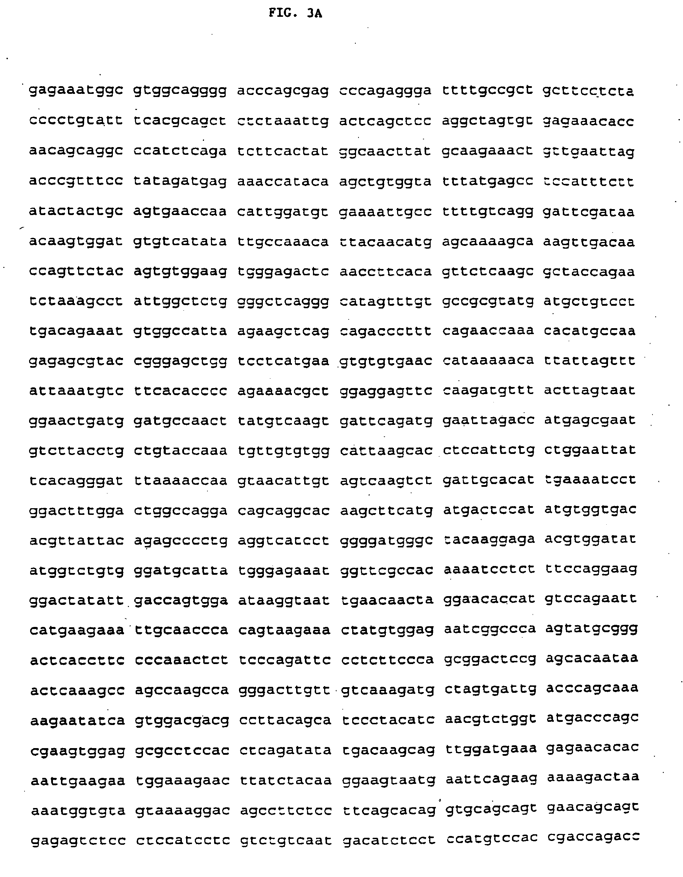

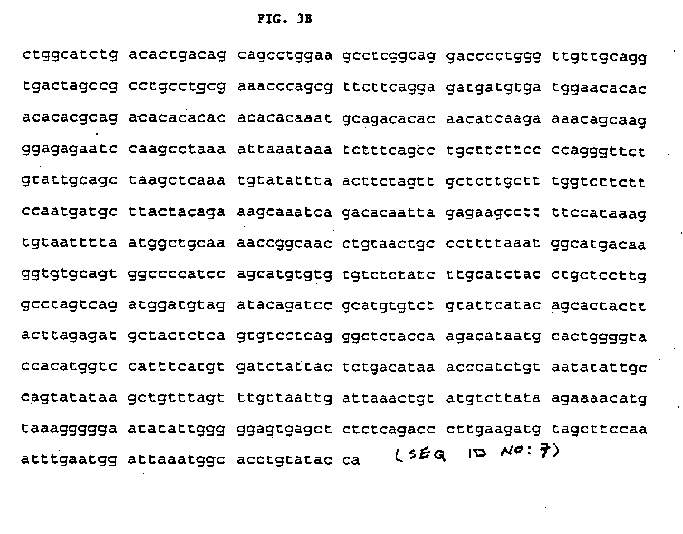

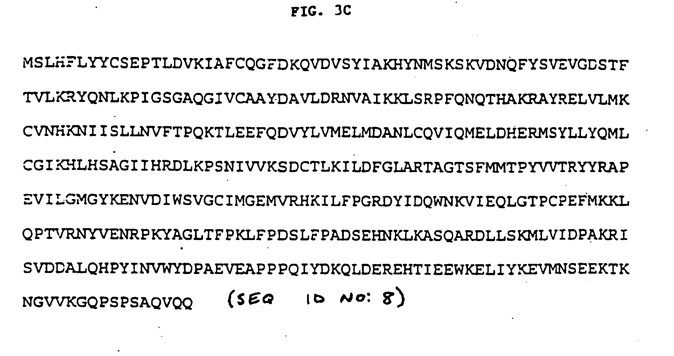

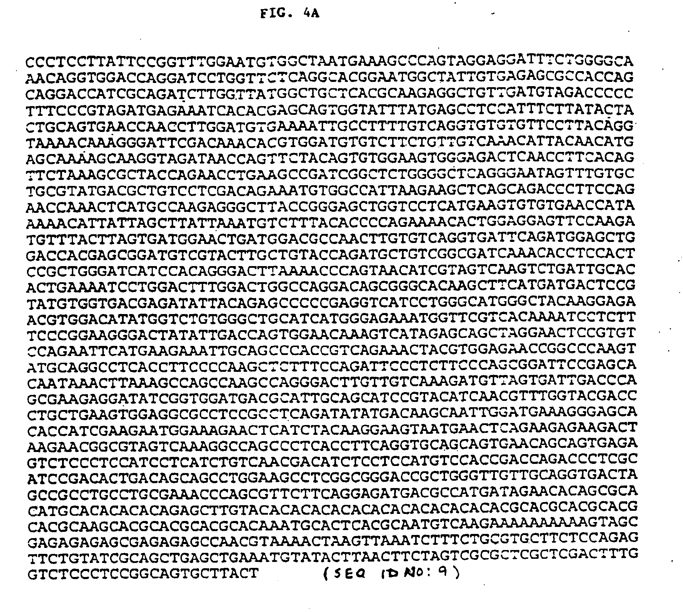

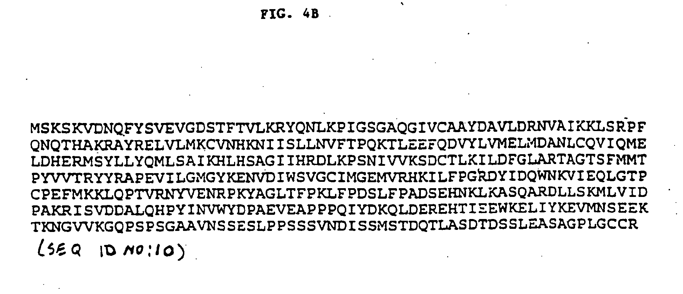

Unless otherwise specified, "JNK3" can refer both to nucleic acids and polypeptides, such as the sequences shown in Figs. 1A-5B (SEQ ID NOS:1-12; see also, GenBank accession number: U34819 which corresponds to SEQ ID NO:1 and SEQ ID NO:2; U34820 which corresponds to SEQ ID NO:4 and SEQ ID NO:5; U07620 which corresponds to SEQ ID NO:7 and SEQ ID NO:8; L27128 which corresponds to SEQ ID NO:9 and SEQ ID NO:10; and L35236 which corresponds to SEQ ID NO:11 and SEQ ID NO:12). SEQ ID NO:3 and SEQ ID NO:6 represent deduced nucleotide sequences based on the presumed overlap between the sequences represented by SEQ ID NOS:1 and 4 with the sequence represented by SEQ ID NO:7. JNK3 also refers to polypeptides that are at least 85% identical to the amino acid sequences listed above, and to the nucleic acids encoding those polypeptides. Examples of these sequences and methods of isolating them are found in Gupta et al., *supra,* 1996; Kyriakis et al., *supra*; Martin et al., Brain Res. Mol. Brain Res., 35:45-57, 1996; and Mohit et al., Neuron, 14:67-78, 1995.

A "control" cell is a cell that is generally the same, e.g., genotypically and phenotypically, as the cell to which it is being compared (e.g., the cells can be sister cells), but which is not exposed to a test compound.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description and from the claims.

### Brief Description of the Drawings

Fig. 1A is a schematic representation of the nucleic acid sequence of GenBank Accession No. U34819 (SEQ ID NO:1) .
Fig. 1B is a schematic representation of the amino acid sequence of GenBank Accession No. U34819 (SEQ ID NO:2) .
Fig. 1C is a schematic representation of the nucleic acid sequence of SEQ ID NO:3.
Fig. 2A-B is a schematic representation of the nucleic acid sequence of GenBank Accession No. U34820 (SEQ ID NO:4) .
Fig. 2C is a schematic representation of the amino acid sequence of GenBank Accession No. U34820 (SEQ ID NO:5).
Fig. 2D is a schematic representation of the nucleic acid sequence of SEQ ID NO:6.
Fig. 3A-B is a schematic representation of the nucleic acid sequence of GenBank Accession No. U07620 (SEQ ID NO:7).
Fig. 3C is a schematic representation of the amino acid sequence of GenBank Accession No. U07620 (SEQ ID N0:8) .
Fig. 4A is a schematic representation of the nucleic acid sequence of GenBank Accession No. L27128 (SEQ ID NO:9).
Fig. 4B is a schematic representation of the amino acid sequence of GenBank Accession No. L27128 (SEQ ID NO:10).
Fig. 5A is a schematic representation of the nucleic acid sequence of GenBank Accession No. L35236 (SEQ ID NO:11)
Fig. 5B is a schematic representation of the amino acid sequence of GenBank Accession No. L35236 (SEQ ID NO:12).
Fig. 6 is a diagram of the wild type JNK3 gene locus, targeting vector, and the mutated or disrupted JNK3 gene locus.
Fig. 7 is a bar graph showing the temporal responses of wild type and JNK3(-/-) mice to kainic acid (KA) injection.
Fig. 8 is a bar graph showing the temporal responses of wild type and JNK3(-/-) mice to pentetrazole (PTZ) injection.
Fig. 9A is a schematic representation of the nucleic acid sequence of murine c-Jun (GenBank Accession No. X12740; SEQ ID NO:13).
Fig. 9B is a schematic representation of the amino acid sequence of murine c-Jun (GenBank Accession No. X12740; SEQ ID NO:14).
Fig. 10A-B is a schematic representation of the nucleic acid sequence of murine c-Fos (GenBank Accession No. V00727; SEQ ID NO:15).
Fig. 10C is a schematic representation of the amino acid sequence of murine c-Fos (GenBank Accession No. V00727; SEQ ID NO:16)
Fig. 11 is a bar graph showing the level of KA-induced AP-1 activity at various times after KA induction as reflected by luciferase activity in JNK3(-/-) mice crossed with transgenic AP-1 luciferase mice.
Fig. 12 is a bar graph showing the level of KA-induced AP-1 activity as reflected by the relative level of luciferase activity in hippocampus (HP) and cerebellum (CB) of JNK3(-/-) mice and wild type(+/+) mice.
Fig. 13 is a diagram of the proposed chain of molecular events caused by KA leading to neuronal apoptosis.
Fig. 14 is a diagram of the trisynaptic connection within the hippocampal formation.

### Detailed Description

JNK protein kinase phosphorylates c-Jun and subsequently increases AP-1 transcriptional activity in response to a specific group of stress signals (Whitmarsh et al, *supra*; Yang et al., *supra*)*.* The neural-specific expression of JNK3 may render neurons particularly susceptible to physiological stress. In the experiments described herein, a remarkable resistance to kainic acid (KA)-induced seizures and apoptosis has been observed in JNK3-deficient mice. The resistance to KA neurotoxicity may be due to the elimination of a specific stress-response pathway mediated by the JNK3 isoform of JNK protein kinase. First, the administration of KA caused the phosphorylation of the NH₂-terminal activation domain of c-Jun and markedly increased AP-1 transcriptional activity in wild-type, but not in JNK3-deficient mice. Second, there was prolonged expression of phosphorylated c-Jun within the most vulnerable area of the hippocampus, further indicating that JNK activity may lead to neuronal apoptosis.

The findings reported herein are consistent with the dependence of KA neurotoxicity on excitatory circuitry (Nadler et al., Brain Res. 195:47-56, 1980). Since JNK3 is widely expressed in the nervous system and its activity is increased by many different stress signals (Gupta et al., *supra),* JNK3 may be involved in stress-induced apoptosis caused by a wide range of environmental insults.

The identification of JNK3 as a critical mediator of KA-induced excitatory neurotoxicity has clinical implications. The amino acid sequence of mouse, rat and human JNK3 is highly conserved (Kyriakis et al., *supra*; Gupta et al., *supra;* Martin et al., *supra*; Mohit et al., Neuron. 14:67-78, 1995). Moreover, the expression of the human JNK3 gene is also restricted to the nervous system and neuroendocrine system, is widely expressed in many brain subregions (Gupta et al., *supra;* Mohit et al., *supra*). It is therefore likely that the human and rodent JNK3 protein kinases have related or identical physiological functions. Neurotoxicity of the excitatory amino acids has been implicated in many neurological disorders ranging from acute ischemia to chronic neurodegenerative diseases (Choi, Neuron, 1:623-634, 1988; Lipton et al., N. Engl. J. Med. 330:613-622, 1994; Rothman et al., Annu. Neurol. 19:105-111, 1986). Previous therapeutic strategies have been focused on the prevention of calcium influx through cell surface channels, such as the NMDA-type glutamate receptor. To date, these approaches have only met with mixed results (Lipton et al., *supra*). JNK3 is therefore a target for therapeutic interventions when excitatory neurotoxicity involves JNK3-mediated apoptosis.

In the experiments described infra, homologous recombination was used to generate JNK3-deficient mice, and their responses to noxious stimuli were examined. KA, a potent excitotoxic chemical, elicits limbic seizures and neuronal cell death. The neurotoxicity of KA derives from the direct stimulation of the glutamate receptor at postsynaptic sites, and an indirect increase in the release of excitatory amino acids from presynaptic sites. It is well-documented that systemic application of KA induces the expression of various cellular immediate early genes (cIEGs), including c-Jun and c-Fos. Thus, the application of KA triggers a stress-response pathway in the brain *in vivo*. The experiments detailed *infra* demonstrate that KA induces phosphorylation of c-Jun and an increase in AP-1 transcriptional activity in the brain of wild-type mice. However, these effects of KA are markedly suppressed in the brains of JNK3-deficient mice. Moreover, JNK3-deficient mice exhibit a remarkable resistance to KA-induced seizures and apoptosis of hippocampal neurons. These normal mice treated with KA represent a useful model of human disorders of the nervous system involving excitotoxicity.

Based on these experimental results, JNK3 was found to be an exceptional target for limiting excitotoxic damage. In particular, JNK3 is a target in screening protocols including protocols to screen for molecules that regulate JNK3 gene expression, JNK3 binding to its substrates, and JNK3 activity, as described below. The molecules found in these screens that effectively decrease JNK3 expression or activity are candidate drugs to be used to treat disorders of the nervous system involving excitotoxicity, including seizure disorders such as epilepsy, cerebrovascular disorders including ischemia, metabolic imbalance (e.g., hypoglycemia), injury due to extreme heat or cold, trauma (e.g., irradiation, spinal cord injury, pressure, and ionic imbalance), dementias such as Alzheimer's disease, Parkinson's disease, and neurodegenerative disorders (e.g., Huntington disease), and motoneuron disease (including amyotrophic lateral sclerosis) (Thompson, Science, 267:1456-1462, 1995; Coyle et al., Science, 262:689-695, 1993).

### Methods of Screening for Molecules that Inhibit JNK3 Activity

The following assays and screens can be used to identify compounds that are effective inhibitors of JNK3 activity. The assays and screens can be done by physical selection of molecules from libraries, and computer comparisons of digital models of compounds in molecular libraries and a digital model of the JNK3 active site. The inhibitors identified in the assays and screens may act by, but are not limited to, binding to JNK3 (e.g., from mouse or human), binding to intracellular proteins that bind to JNK3, compounds that interfere with the interaction between JNK3 and its substrates, compounds that modulate the activity of a JNK3 gene, or compounds that modulate the expression of a JNK3 gene or a JNK3 protein.

Assays can also be used to identify molecules that bind to JNK3 regulatory sequences (e.g., promoter sequences), thus modulating gene expression. See, e.g., Platt, J. Biol. Chem., 269:28558-28562, 1994.

The compounds that can be screened by the methods described herein include, but are not limited to, peptides and other organic compounds (e.g., peptidomimetics) that bind to a JNK3 protein or inhibit its activity in any way.
Such compounds may include, but are not limited to, peptides; for example, soluble peptides, including but not limited to members of random peptide libraries (see, e.g., Lam et al., Nature 354:82-84, 1991; Houghten et al., Nature 354:84-86, 1991), and combinatorial chemistry-derived molecular libraries made of D-and/or L-amino acids, phosphopeptides (including, but not limited to, members of random or partially degenerate, directed phosphopeptide libraries; see, e.g., Songyang et al., Cell 72:767-778, 1993), and small organic or inorganic molecules.

Compounds and molecules are screened to identify those that affect expression of a JNK3 gene or some other gene involved in regulating the expression of JNK3 (e.g., by interacting with the regulatory region or transcription factors of a gene). Compounds are also screened to identify those that affect the activity of such proteins (e.g., by inhibiting JNK3 activity) or the activity of a molecule involved in the regulation of JNK3.

Computer modeling or searching technologies are used to identify compounds, or identify modified compounds that modulate or are candidates to modulate the expression or activity of a JNK3 protein. For example, compounds likely to interact with the active site of the JWK3 protein are identified. The active site of JNK3 can be identified using methods known in the art including, for example, analysis of the amino acid sequence of a molecule, and from a study of complexes formed by JNK3 with a native ligand (e.g., ATF2 or c-Jun). Chemical or X-ray crystallographic methods can be used to identify the active site of JNK3 by the location of a bound ligand such as c-Jun or ATF2.

The three-dimensional structure of the active site can be determined. This can be done using known methods, including X-ray crystallography, which can be used to determine a complete molecular structure. Solid or liquid phase NMR can be used to determine certain intramolecular distances. Other methods of structural analysis can be used to determine partial or complete geometrical structures. Geometric structure can be determined with a JNK3 protein bound to a natural (e.g., c-Jun or ATF2) or artificial ligand which may provide a more accurate active site structure determination.

Computer-based numerical modeling can be used to complete an incomplete or insufficiently accurate structure. Modeling methods that can be used are, for example, parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups are necessary, and can be selected from force fields known in physical chemistry. Information on incomplete or less accurate structures determined as above can be incorporated as constraints on the structures computed by these modeling methods.

Having determined the structure of the active site of a JNK3 protein, either experimentally, by modeling, or by a combination of methods, candidate modulating compounds can be identified by searching databases containing compounds along with information on their molecular structure. The compounds identified in such a search are those that have structures that match the active site structure, fit into the active site, or interact with groups defining the active site. The compounds identified by the search are potential JNK3 modulating compounds.

These methods may also be used to identify improved modulating compounds from an already known modulating compound or ligand. The structure of the known compound is modified and effects are determined using experimental and computer modeling methods as described herein. The altered structure is compared to the active site structure of a JNK3 protein to determine or predict how a particular modification to the ligand or modulating compound will affect its interaction with that protein. Systematic variations in composition, such as by varying side groups, can be evaluated to obtain modified modulating compounds or ligands of preferred specificity or activity.

Given the teachings herein, additioanl experimental and computer modeling methods useful to identify modulating compounds based on identification of the active sites of a JNK3 protein and related transduction and transcription factors an be developed by those skilled in the art.

Examples of molecular modeling systems are the QUANTA programs, e.g., CHARMm, MCSS/HOOK, and X-LIGAND, (Molecular Simulations, Inc., San Diego, CA). QUANTA provides a modeling environment for two dimensional and three dimensional modeling, simulation, and analysis of macromolecules and small organics. Specifically, CHARMm analyzes energy minimization and molecular dynamics functions. MCSS/HOOK characterizes the ability of an active site to bind a ligand using energetics calculated via CHARMm. X-LIGAND fits ligand molecules to electron density patterns of protein-ligand complexes. The program also allows interactive construction, modification, visualization, and analysis of the behavior of molecules with each other.

Articles reviewing computer modeling of compounds interacting with specific proteins can provide additional guidance. For example, see, Rotivinen et al., Acta Pharmaceutical Fennica 97:159-166, 1988; Ripka, New Scientist 54-57 (June 16, 1988); McKinaly and Rossmann, Ann. Rev. Pharmacol. Toxicol. 29:111-122, 1989; Perry and Davies, OSAR: Quantitative Structure-Activity Relationships in Drug Design pp.189-193 (Alan R. Liss, Inc., 1989); Lewis and Dean, Proc. R. Soc. Lond. 236:125-140, 141-162, 1989; and, regarding a model receptor for nucleic acid components, see Askew et al., Am. J. Chem. Soc. 111:1082-1090. Computer programs designed to screen and depict chemicals are available from companies such as MSI (*supra*), Allelix, Inc. (Mississauga, Ontario, Canada), and Hypercube, Inc. (Gainesville, FL). These applications are largely designed for drugs specific to particular proteins; however, they may be adapted to the design of drugs specific to identified regions of DNA or RNA. Commercial sources of chemical libraries can be used as sources of candidate compounds. Such chemical libraries can be obtained from, for example, ArQule, Inc. (Medford, MA).

In addition to designing and generating compounds that alter binding, as described above, libraries of known compounds, including natural products, synthetic chemicals, and biologically active materials including peptides, can be screened for compounds that are inhibitors or activators.

Compounds identified by methods described above may be useful, for example, for elaborating the biological function of JNK3 gene products and in treatment of disorders in which JNK3 activity is deleterious. Assays for testing the effectiveness of compounds such as those described herein are further described below.

### In Vitro Screening Assays for Compounds that Bind to JNK3 Proteins and Genes

*In vitro* systems can be used to identify compounds that can interact (e.g., bind) to JNK3 proteins or genes encoding those proteins. Such compounds may be useful, for example, for modulating the activity of JNK3 polypeptides or nucleic acids, elaborating their biochemistry, or treating disorders caused or exacerbated by JNK3 expression. These compounds may themselves disrupt normal function or can be used in screens for compounds that disrupt normal function.

Assays to identify compounds that bind to JNK3 proteins involve preparation of a reaction mixture of the protein and the test compound under conditions sufficient to allow the two components to interact and bind, thus forming a complex that can be detected and/or isolated.

Screening assays for molecules that can bind to a JNK3 protein or nucleic acid can be performed using a number of methods. For example, a JNK3 protein, peptide, or fusion protein can be immobilized onto a solid phase, reacted with the test compound, and complexes detected by direct or indirect labeling of the test compound. Alternatively, the test compound can be immobilized, reacted with JNK3 polypeptide, and any complexes detected. Microtiter plates can be used as the solid phase and the immobilized component anchored by covalent or noncovalent interactions. Non-covalent attachment may be achieved by coating the solid phase with a solution containing the molecule, and drying. Alternatively, an antibody specific for JNK3 is used to anchor the molecule to the solid surface. Such surfaces may be prepared in advance of use, and stored. JNK3 antibodies can be produced using conventional methods such as those described in Coligan et al. (Current Protocols in Immunology, John Wiley & Sons, Inc., 1994, see Volume 1, chapter 2).

In the assay, the non-immobilized component is added to the coated surface containing the immobilized component under conditions that permit interaction and binding between the two components. The unreacted components are then removed (e.g., by washing) under conditions such that any complexes formed will remain immobilized on the solid phase. The detection of the complexes can be accomplished by a number of methods known to those in the art. For example, the nonimmobilized component of the assay may be prelabeled with a radioactive or enzymatic label and detected using appropriate means. If the non-immobilized entity was not prelabeled, an indirect method is used. For example, if the non-immobilized entity is a JNK3 polypeptide, an antibody against JNK3 is used to detect the bound molecule, and a secondary, labeled antibody is used to detect the entire complex.

Alternatively, a reaction can be conducted in a liquid phase, the reaction products separated from unreacted components, and complexes detected (e.g., using an immobilized antibody specific for a JNK3 protein).

Cell-based assays can be used to identify compounds that interact with JNK3 proteins. Cell lines that naturally express such proteins or have been genetically engineered to express such proteins (e.g., by transfection or transduction with JNK3 DNA) can be used. For example, test compounds can be administered to cell cultures and the phosphorylation of ATF2 or c-Jun measured as described infra. A decrease in the amount of phosphorylation of a JNK3 substrate in the presence of the test compound compared to controls that do not contain the test compound indicates that the test compound is an inhibitor of JNK3 activity.

Inhibitors of JNK3 expression that act on the JNK3 promoter can be identified using a chimeric gene in which genomic sequences including the JNK3 promoter are fused to a reporter, for example firefly luciferase. Cultured cells (including neurons) transformed with this DNA are screened for the expression of luciferase activity. Compounds that inhibit luciferase activity in this high throughput assay can be confirmed by direct measurement of the endogenous JNK3 protein (by Western blotting) and JNK3 mRNA (by Northern blotting) using methods known in the art (for example, see Ausubel et al., Current Protocols in Molecular Biology, John Wiley & Sons, 1994).

Candidate inhibitory compounds can be tested further in cell or tissue cultures as well as animal models. For example, cells expressing JNK3 are incubated with a test compound. Lysates are prepared from treated and untreated cells and Western blotted according to known methods. The blots are probed with antibodies specific for JNK3. A decrease in the amount of JNK3 expression in cultures treated with the test compound compared to untreated controls indicates that the test compound is a candidate for a drug to treat disorders associated with JNK3 expression.

### Assays for Compounds that Interfere with JNK3/JNK3

### Substrate Interactions

Molecules that disrupt the interaction between JNK3 and its substrates can be identified using assays that detect protein-protein interactions. For example, the yeast two-hybrid method detects protein interactions *in vivo*. However, an *in vitro* assay is preferable because candidate molecules may not be permeable to the yeast cell wall. An example of an *in vitro* assay for such test molecules that disrupt the interaction between JNKC3 and a substrate includes the use of immobilized JNK3 or immobilized substrate (e.g., c-Jun) and incubation of the immobilized component with cell lysates or purified proteins in the presence and absence of a test molecule. In general, the test molecule is tested over a range of a 100 fold molar excess over the most abundant component (e.g., the component immobilized or in solution). If the test molecule is predicted to interact with the immobilized component of the assay, then it can be pre-incubated with that component before adding the cell lysate or purified protein. After washing away unbound material, the bound proteins are detected with antibodies (e.g., ELISA or Western blot) or through the use of labeled proteins (e.g. radioactive or fluorescent) using methods known in the art. Test molecules that decrease the amount of substrate bound to JNK3 are thus identified as molecules that interfere with JNK3/JNK3 substrate interactions.

### Assays for Compounds that Ameliorate the Effects of JNK3 in vivo

Compounds identified as above, or other candidate compounds that inhibit JNK3 activity *in vitro* may be useful for treating disorders involving JNK3 activity. These compounds can be tested in *in vivo* assays, for example, in animal models of disorders involving JNK3 activity. For example, transgenic mouse models of ALS have been described (Bruijn and Cleveland, Neuropathol. Appl. Neurobiol. 22:373-387, 1996; Dal Canto and Gurney, Brain Res. 676: 25-40, 1995; Cleveland et al., Neurology 47: Suppl 2, S54-61) as have transgenic models of Alzheimer's disease such as the PDAPP mouse and others (for examples, see Loring et al., Neurobiol. Aging 17:173-182, 1996). MPTP (1-methyl-4-phenyl-1,2,3,6-tetrahydropyridine)-induced dopaminergic neurotoxicity has been used as a model for Parkinson's disease in rodents and nonhuman primates (for example, Przedborski et al., Proc. Nat'1. Acad. Sci. USA 93:4565-4571, 1996).

Test compounds predicted to inhibit JNK3 activity are administered to animals, e.g., as described above, that serve as models for the various disease paradigms. Treated animals are then assayed for inhibition of JNK3 activity. Such assays may be indirect or inferential, for example, improved health or survival of the animal indicates the efficacy of a test compound. Assays can also be direct, for example, a decrease in JNK3 or c-Jun expression can be measured by Northern analysis of neural tissue removed from an animal treated with a test compound. A decrease in the amount of JNK3 mRNA present in the sample from treated animals compared to untreated controls indicates that the test compound is inhibiting JNK3 expression. A decrease in the amount of c-Jun indicates that the test compound is inhibiting JNK3 expression or activity.

### Antisense Constructs and Therapies

Treatment regimes based on an "antisense" approach involve the design of oligonucleotides (either DNA or RNA) that are complementary to JNK3 mRNAs. These oligonucleotides bind to the complementary mRNA transcripts and prevent translation. Absolute complementarily, although preferred, is not required. A sequence "complementary" to a portion of an RNA, as referred to herein, is a sequence sufficiently complementary to be able to hybridize with the RNA, forming a stable duplex; in the case of double-stranded antisense nucleic acids, a single strand of the duplex DNA may be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the message, e.g., the 5' untranslated sequence, up to and including the AUG initiation codon, are generally most efficient for inhibiting translation. However, sequences complementary to the 3' untranslated sequences of mRNAs have also been shown to be effective for inhibiting translation (Wagner, Nature, 372:333, 1984). Thus, oligonucleotides complementary to either the 5' or 3' non-translated, non-coding regions of a JNK3 could be used in an antisense approach to inhibit translation of the endogenous human homolog of JNK3 mRNA. Oligonucleotides complementary to the 5' untranslated region of the mRNA should include the complement of the AUG start codon. Examples of candidate antisense sequences for the 5' and 3' regions are; 5'-AAG AAA TGG AGG CTC ATA AAT ACC ACA GCT-3' (SEQ ID NO:17) and 5'-ATT GGA AGA AGA CCA AAG CAA GAG CAA CTA-3' (SEQ ID NO:18), respectively.

While antisense nucleotides complementary to the coding region of a JNK3 gene could be used, those complementary to transcribed untranslated regions are most preferred. Examples of this type of candidate sequence are 5'-TAA GTA AGT AGT GCT GTA TGA ATA CAG ACA-3' (SEQ ID NO:19) and 5'-TAC TGG CAA TAT ATT ACA GAT GGG TTT ATG-3' (SEQ ID NO:20).

Antisense oligonucleotides complementary to mRNA coding regions are less efficient inhibitors of translation, but could be used in accordance with the invention. Whether designed to hybridize to the 5', 3', or coding region of a JNK3 mRNA, antisense nucleic acids should be at least six nucleotides in length, and are preferably oligonucleotides ranging from 6 to about 50 nucleotides in length. In specific aspects, the oligonucleotide is at least 10 nucleotides, or at least 50 nucleotides in length.

Regardless of the choice of target sequence, *in vitro* studies are usually performed first to assess the ability of an antisense oligonucleotide to inhibit gene expression. In general, these studies utilize controls that distinguish between antisense gene inhibition and nonspecific biological effects of oligonucleotides. In these studies levels of the target RNA or protein are usually compared with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide, and that the nucleotide sequence of the oligonucleotide differs from the antisense sequence no more than is necessary to prevent specific hybridization to the target sequence.

The oligonucleotides can be DNA or RNA, or chimeric mixtures or derivatives or modified versions thereof, single-stranded or double-stranded. The oligonucleotide can be modified at the base moiety, sugar moiety, or phosphate backbone, for example, to improve stability of the molecule or hybridization. The oligonucleotide may include other appended groups such as peptides (e.g., for targeting host cell receptors *in vivo*), or agents facilitating transport across the cell membrane (as described, e.g., in Letsinger et al., Proc. Natl. Acad. Sci. USA 86:6553, 1989; Lemaitre et al., Proc. Natl. Acad. Sci. USA 84:648, 1987; PCT Publication No. WO 88/09810) or the blood-brain barrier (see, for example, PCT Publication No. WO 89/10134), or hybridization-triggered cleavage agents (see, for example, Krol et al., BioTechniques 6:958, 1988), or intercalating agents (see, for example, Zon, Pharm. Res. 5:539, 1988). To this end, the oligonucleotide can be conjugated to another molecule, e.g., a peptide, hybridization-triggered cross-linking agent, transport agent, or hybridization-triggered cleavage agent.

The antisense oligonucleotide may comprise at least one modified base moiety which is selected from the group including, but not limited to, 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-theouracil, 2-thiouracil, 4-thiouracil, 5-methyluracil, uracil-5-oxyacetic acid methylester, uracil-5-oxyacetic acid (v), 5-methyl-2-thiouracil, 2-(3-amino-3-N-2-carboxypropl) uracil, (acp3)w, and 2,6-diaminopurine.

The antisense oligonucleotide can also comprise at least one modified sugar moiety selected from the group including, but not limited to, arabinose, . 2-fluoroarabinose, xylulose, and hexose.

The antisense oligonucleotide can also include at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal, or an analog of any of these backbones.

The antisense oligonucleotide can include an α-anomeric oligonucleotide. An α-anomeric oligonucleotide forms specific double-stranded hybrids with complementary RNA in which, contrary to the usual β-units, the strands run parallel to each other (Gautier et al., Nucl. Acids. Res. 15:6625, 1987). The oligonucleotide is a 2'-0-methylribonucleotide (Inoue et al., Nucl. Acids Res. 15:6131, 1987), or a chimeric RNA-DNA analog (Inoue et al., FEBS Lett. 215:327, 1987).

Antisense oligonucleotides of the invention can be synthesized by standard methods known in the art, e.g., by use of an automated DNA synthesizer (such as are commercially available from Biosearch, Applied Biosystems, etc.). As examples, phosphorothioate oligonucleotides can be synthesized by the method of Stein et al. (Nucl. Acids Res. 16:3209, 1988), and methylphosphonate oligonucleotides can be prepared by use of controlled pore glass polymer supports (Sarin et al., Proc. Natl. Acad. Sci. USA 85:7448, 1988).

The antisense molecules should be delivered to cells that express JNK3 proteins *in vivo*. A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense molecule sufficient to suppress translation of endogenous mRNAs. Therefore, an approach may be used in which a recombinant DNA construct comprises an antisense oligonucleotide placed under the control of a strong *pol* III or *pol* II promoter. The use of such a construct to transfect target cells in a patient will result in the transcription of sufficient amounts of single stranded RNAs that will form complementary base pairs with the endogenous JNK3 transcripts and thereby prevent translation of that mRNA. For example, a vector can be introduced *in vivo* such that it is taken up by a cell and directs the transcription of an antisense RNA. Such a vector can remain episomal or become chromosomally integrated, as long as it can be transcribed to produce the desired antisense RNA.

Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Suitable promoters include, but are not limited to: the SV40 early promoter region (Bernoist et al., Nature 290:304, 1981); the promoter contained in the 3' long terminal repeat of Rous sarcoma virus (Yamamoto et al., Cell 22:787-797, 1988); the herpes thymidine kinase promoter (Wagner et al., Proc. Natl. Acad. Sci. USA 78:1441, 1981); and the regulatory sequences of the metallothionein gene (Brinster et al., Nature 296:39, 1988). Constructs may also be contained on an artificial chromosome (e.g., mammalian artificial chromosome; MAC; Harrington et al., Nature Genet. 15:345-355, 1997) .

The production of a JNK3 antisense nucleic acid molecule by any gene therapeutic approach described above results in a cellular level of JNK3 protein that is less than the amount present in an untreated individual.

### Ribozymes

Ribozyme molecules designed to catalytically cleave JNK3 mRNAs can also be used to prevent translation of these mRNAs and expression of JNK3 mRNAs (see, e.g., PCT Publication WO 90/11364; Saraver et al., Science 247:1222, 1990). While various ribozymes that cleave mRNA at site-specific recognition sequences can be used to destroy specific mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art (Haseloff et al., Nature 334:585, 1988). Preferably, the ribozyme is engineered so that the cleavage recognition site is located near the 5' end of the JNK3 mRNA, i.e., to increase efficiency and minimize the intracellular accumulation of non-functional mRNA transcripts.

Examples of potential ribozyme sites in human JNK3 include 5'-UG-3' sites which correspond to the initiator methionine codon at, for example, in human JNK3, about nucleotides 224-226, the codon for a downstream potential initiation site (nucleotides 338-340), and additional codons in the coding region, including nucleotides 698-670; 740-742; and 935-937.

The ribozymes of the present invention also include RNA endoribonucleases (hereinafter "Cech-type ribozymes"), such as the one that occurs naturally in *Tetrahymena Thermophila* (known as the IVS or L-19 IVS RNA), and which has been extensively described by Cech and his collaborators (Zaug et al., Science 224:574, 1984; Zaug et al., Science, 231:470, 1986; Zug et al., Nature 324:429, 1986; PCT Application No. WO 88/04300; and Been et al., Cell 47:207, 1986). The Cech-type ribozymes have an eight base-pair sequence that hybridizes to a target RNA sequence, whereafter cleavage of the target RNA takes place. The invention encompasses those Cech-type ribozymes that target eight base-pair active site sequences present in JNK3 proteins.

As in the antisense approach, the ribozymes can be composed of modified oligonucleotides (e.g., for improved stability, or targeting), and should be delivered to cells which express a JNK3 gene *in vivo*, e.g., the brain and spinal cord. A preferred method of delivery involves using a DNA construct "encoding" the ribozyme under the control of a strong constitutive *pol* III or *pol* II promoter, so that transfected cells will produce sufficient quantities of the ribozyme to destroy endogenous JNK3 mRNAs and inhibit translation. Because ribozymes, unlike antisense molecules, are catalytic, a lower intracellular concentration is required for efficiency.

For any of the above approaches, the therapeutic JNK3 antisense or ribozyme nucleic acid molecule construct is preferably applied directly to the target area (e.g., the focal site of activity in a seizure disorder, the hippocampus in Alzheimer's disease, the *substantia nigra* in patients with Parkinson's disease), but can also be applied to tissue in the vicinity of the target area or even to a blood vessel supplying the target area.

For gene therapy, antisense, or ribozyme JNK3 expression is directed by any suitable promoter (e.g., the human cytomegalovirus, simian virus 40, or metallothionein promoters), and its production is regulated by any desired mammalian regulatory element. For example, if desired, enhancers that direct preferential gene expression in cells under excitotoxic induction can be used to direct antisense JNK3 expression in a patient with a seizure disorder.

JNK3 antisense or ribozyme therapy is also accomplished by direct administration of the antisense JNK3 or ribozyme RNA to a target area. This mRNA can be produced and isolated by any standard technique, but is most readily produced by *in vitro* transcription using an antisense JNK3 DNA under the control of a high efficiency promoter (e.g., the T7 promoter). Administration of antisense JNK3 RNA to target cells is carried out by any of the methods for direct administration of therapeutic compounds described herein.

### Methods of Treating Disorders Involving JNK3 Expression or Activity

The invention also encompasses the treatment of disorders, especially in mammals, such as humans, in which JNK3 plays a damaging role. A number of disorders or the nervous system involving excitotoxicity, such as seizure disorders (e.g., epilepsy), dementias such as meurodegenerative disorders (e.g., Alzheimer's disease, Huntington disease), cerebrovascular disorders such as ischemia, motor neuron disease (including ALS), injuries caused by extreme heat or cold, trauma (e.g., irradiation, spinal cord injury, pressure, and ionic imbalance), metabolic imbalance (e.g., hypoglycemia) and Parkinson's disease, can be treated by the methods described herein. Without limiting the invention by committing to any particular theory, a substantial number of neurologic disorders are attributable, at least in part, to excitotoxicity which is mediated by the JNK3 pathway. Thus, inhibitors of this pathway, identified as described above, are useful for treatment of disorders involving excitotoxicity.

Therapy can be designed to reduce the level of endogenous JNK3 gene expression, e.g., using antisense or ribozyme approaches to inhibit or prevent translation of a JNK3 mRNA; triple helix approaches to inhibit transcription of the gene; or targeted homologous recombination to inactivate or "knock out" a gene or its endogenous promoter. The antisense, ribozyme, or DNA constructs described herein can be administered directly to the site containing the target cells; e.g., specific regions of the brain or the spinal cord. Antibodies or fragments of antibodies that recognize JNK3 or a JNK3 substrate, and that have been modified to be expressed or otherwise enter the cell can also be used therapeutically.

### Effective Dose

Toxicity and therapeutic efficacy of the compounds of the invention, e.g., compounds that modulate JNK3 expression or activity, can be determined by standard pharmaceutical procedures, using either cells in culture or experimental animals to determine the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Polypeptides or other compounds that exhibit large therapeutic indices are preferred. While compounds that exhibit toxic side effects may be used, care should be taken to design a delivery system that targets such compounds to the site of affected tissue to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (that is, the concentration of the test compound which achieves a half-maximal inhibition of symptoms) as determined in cell culture. Such information can be used to more accurately determine useful doses in humans. Levels in plasma can be measured, for example, by high performance liquid chromatography.

### Formulations and Use

Pharmaceutical compositions for use in accordance with the present invention can be formulated in conventional manner using one or more physiologically acceptable carriers or excipients.

Thus, the compounds and their physiologically acceptable salts and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (for example, pregelatinised maize starch, polyvinylpyrrolidone or hydroxypropyl methylcellulose); fillers (for example, lactose, microcrystalline cellulose or calcium hydrogen phosphate); lubricants (for example, magnesium stearate, talc or silica); disintegrants (for example, potato starch or sodium starch glycolate); or wetting agents (for example, sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (for example, sorbitol syrup, cellulose derivatives or hydrogenated edible fats); emulsifying agents (for example, lecithin or acacia); non-aqueous vehicles (for example, almond oil, oily esters, ethyl alcohol or fractionated vegetable oils); and preservatives (for example, methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For buccal administration the compositions may take the form of tablets or lozenges formulated in conventional manner.

The preferred methods of administering the compositions of the invention are by direct delivery of the compounds to the central nervous system, preferentially to the brain, especially near to or directly at the site of the disorder, e.g., the hippocampus in the case of Alzheimer's disease, the substantia nigra in the case of Parkinson's disease, and the focal site for seizure disorders. Accordingly, administration may be into a ventricle, intrathecal, or intracerebral ventricular. For example, an Omaya reservoir-shunt with in-line filter can be surgically placed into the cisternal space. A therapeutic compound in an appropriate excipient (e.g., phosphate-buffered saline) is instilled into the shunt by injection on a prescribed basis.

For administration by inhalation, the compounds for use according to the present invention are conveniently delivered in the form of an aerosol spray presentation from pressurized packs or a nebulizer, with the use of a suitable propellant, for example, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the compound and a suitable powder base such as lactose or starch.

The compounds can be formulated for parenteral administration by injection, for example, by bolus injection or continuous infusion. Formulations for injection may be presented in unit dosage form, for example, in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, for example, sterile pyrogen-free water, before use.

The compounds can also be formulated in rectal compositions such as suppositories or retention enemas, for example, containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

The therapeutic compositions of the invention can also contain a carrier or excipient, many of which are known to skilled artisans. Excipients which can be used include buffers (for example, citrate buffer, phosphate buffer, acetate buffer, and bicarbonate buffer), amino acids, urea, alcohols, ascorbic acid, phospholipids, proteins (for example, serum albumin), EDTA, sodium chloride, liposomes, mannitol, sorbitol, and glycerol. The nucleic acids, polypeptides, antibodies, or modulatory compounds of the invention can be administered by any standard route of administration. For example, administration can be parenteral, intravenous, subcutaneous, intramuscular, intracranial, intraorbital, opthalmic, intraventricular, intracapsular, intraspinal, intracisternal, intraperitoneal, transmucosal, or oral. The modulatory compound can be formulated in various ways, according to the corresponding route of administration. For example, liquid solutions can be made for ingestion or injection; gels or powders can be made for ingestion, inhalation, or topical application. Methods for making such formulations are well known and can be found in, for example, "Remington's Pharmaceutical Sciences." It is expected that particularly useful routes of administration will be nasal or by direct infusion into the central nervous system.

### EXAMPLES

### Example 1. JNK3 Expression

A 351-bp sequence derived from the 5' region of the mouse JNK3 cDNA (nucleotides 62-412) was labelled with [³²P] by random priming and used as a probe to determine the tissue expression pattern of the JNK3 gene. Northern blot analysis was performed by standard methods on 2 mg samples of poly(A)⁺ mRNA isolated from testis, kidney, skeletal muscle, liver, lung, spleen, brain, and heart. All Northern blots were probed with [³²P]-labelled β-actin as a control to ensure loading of similar amounts of RNA in each lane. A strong signal corresponding to a 2.7 kb transcript, as well as a weak signal corresponding to a 7.0 kb transcript, were detected in brain. A weak signal corresponding to a 2.7 kb transcript was also detected in the heart. A signal corresponding to a 2.4 kb transcript was detected in the testis. JNK3 expression was not detected in the other tissues examined.

*In situ* hybridization analysis has indicated that JNK3 is expressed in many regions of the brain (Martin et al., *supra*). Total RNA (10 mg) was therefore isolated from different regions of mouse brain (cerebellum, cerebral cortex, hippocampus, midbrain, thalamus, and brainstem) using the TRIzol reagent (Gibco-BRL), and analyzed by Northern blot using the JNK3 probe described above. A signal corresponding to a 2.7 kb transcript was detected in all sections of the brain examined, and was most abundant in the hippocampus.

### Example 2. Targeted Disruption of the JNK3 Gene

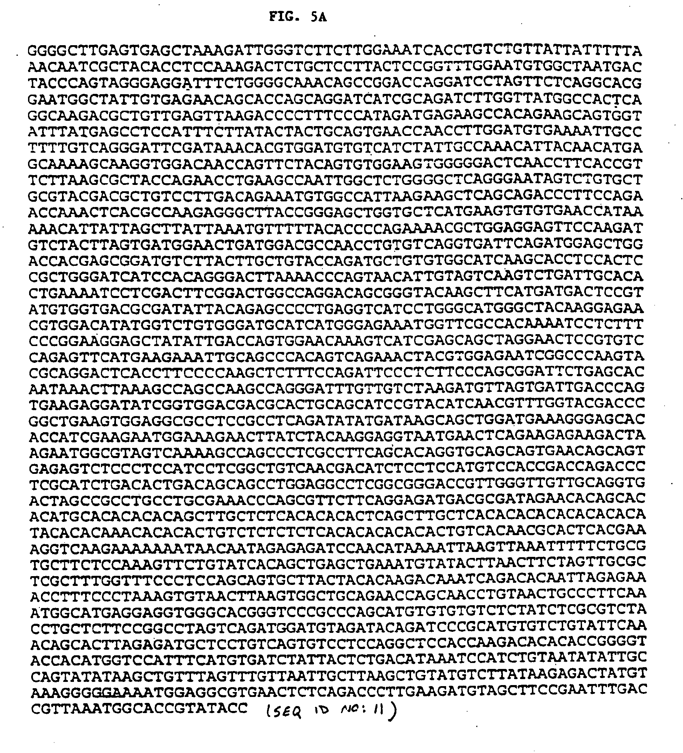

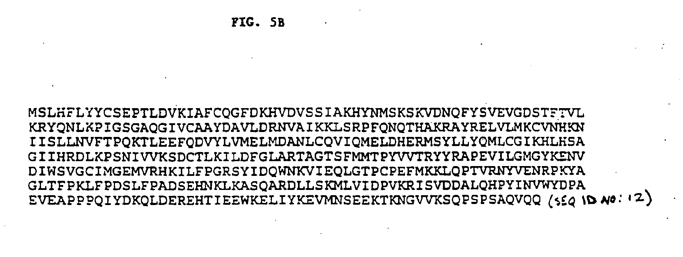

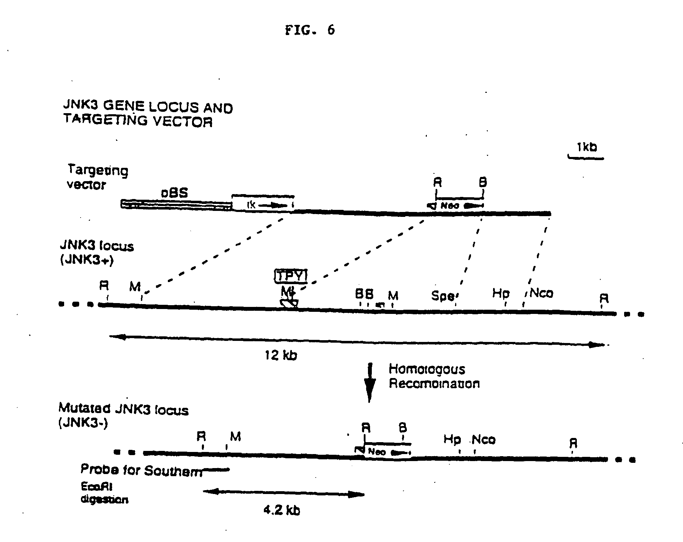

To generate JNK3-deficient mice, a targeting vector was designed to replace an internal 4 kb Mscl-Spel JNK3 genomic fragment with a PGKneo cassette. A map of the JNK3 gene, the JNK3 targeting vector, and the predicted structure of the mutated JNK3 gene are shown diagrammatically in Fig. 6. Restriction enzyme sites are indicated (B, BamHI; Hp, HpaI; M, MscI; Nco, NcoI; R, EcoRI; Spe, SpeI). A 10-kb NotI-EcoRI (the NotI site was vector-derived) JNK3 fragment was cloned from a X FixII phage library of a 129/Sv mouse strain (Stratagene Inc.). The targeting vector was constructed by inserting a 4.0 kb MscI fragment from the 5' end of the JNK3 genomic fragment, a 1.6 kb PGK-neo cassette (Negishi et al., Nature 376:435-438, 1995) and a 1.8-kb SpeI-NcoI fragment of the 3' end of the JNK3 fragment into pBluescript KS vector (Stratagene Inc.) using appropriate linkers. The targeting vector contains a 2.6-kb PGK tk cassette (Negishi et al., *supra*) flanking the 5' end of the JNK3 genomic sequence for negative-selection of mutant ES cells (Mansour et al., 336:348-352, 1988). The region replaced in the JNK3 gene by the targeting vector encompasses one and a half exons encoding amino acids 211 to 267 of JNK3 (as shown in Fig. 5B). This region includes the tripeptide dual phosphorylation motif Thr-Pro-Tyr (TPY) that is characteristic of the JNK group and required for protein kinase activity (Dèrijard et al., *supra*)*.* The two hatched boxes shown in the JNK3 locus correspond to subdomains VIII and IX (encoding amino acid residues 189-267 in the JNK3 protein shown in Fig. 5B) of JNK3.

The targeting vector was linearized with NotI and electroporated into W9.5 embryonic stem (ES) cells. Genomic DNA from transfectants resistant to G418 (200 mg/ml)(Gibco BRL) and gancyclovir (2 mM) (Syntex, Pala Alto, CA) were isolated and screened by Southern blot analysis. Southern blot analysis of 104 independent G418- and gancyclovir-resistant clones revealed three clones containing the desired homologous recombination event (targeting frequency 2.9%). Chimeric mice were generated by injecting these ES cells into C57BL/6 (B6) mouse blastocysts.

Southern blots of EcoRI-restricted DNA derived from the tails of these chimeric mice were probed with the radiolabeled 351 bp JNK3 probe. EcoRI digestion resulted in a 12 kb band corresponding to the wild-type (endogenous allele), and a 4.2 kb band corresponding to the mutant (disrupted allele).

Two clones mediated germline transmission of the disrupted JNK3 allele into the next generation of mice. Heterozygotes (+/-) were intercrossed to generate homozygous mutant mice (-/-) that were identified by Southern blot analysis of genomic DNA. Total RNA isolated from mouse brain was examined by Northern blot analysis. The blot was probed with a random-primed ³²P-labeled mouse JNK3 cDNA probe, then stripped and sequentially reprobed with mouse JNK1 and β-actin cDNA probes. The major JNK3 transcript in brain is 2.7 kb, and the JNK1 transcripts in mouse brain are 2.3 and 4.4 kb. Blots hybridized with a JNK3 cDNA probe detected transcripts in wild-type (+/+), but not in homozygous knockout (-/-) mice.

Reverse transcriptase-polymerase chain reaction (RT-PCR) analysis was used to confirm that JNK3 transcripts were absent in the homozygous JNK3 (-/-) brain. A JNK1 probe (447 bp) was amplified from mouse brain RNA by RT-PCR (Yang et al., *supra*) using the amplimers 5'-GTGTGCAGCTTATGATGCTATTCTTGAA-3' (SEQ ID NO:21) and 5' CGCGTCACCACATACGGAGTCATC-3' (SEQ ID NO:22). RT-PCR detection (Yang et al., *supra*) of JNK3 mRNA in mouse tissues was performed by RT-PCR using the amplimers: 5'-CTGGAGGAGTTCCAAGATGTCTACT-3' (SEQ ID NO:23) and 5'-TGGAAAGAGCTTGGGGAAGGTGAG-3' (SEQ ID NO:24) to yield a specific 537 bp DNA product. RNA isolated from mouse brain was amplified with primers specific for HPRT as a control. These experiments confirmed the absence of JNK3 transcripts in the homozygous JNK3 (-/-) brain.

Protein kinase assays were performed to show that JNK3 (-/-) mouse brain was deficient in JNK3 activity. In these experiments, JNK3 kinase activity in brain lysates was measured after immunodeletion of JNK1 and JNK2 by in-gel protein kinase assays using the substrate GST-cJun (Dèrijard et al., *supra*). When mouse hippocampal lysates (30 µg) from wild type (+/+) and homozygous knockout (-/-) brains were assayed, the 55 kD and 46 kD JNK3 isoforms were detected in wild type but not JNK3 (-/-) mice, confirming that JNK3 (-/-) mouse brain was deficient in JNK3 kinase activity. Together, these data demonstrate that the targeted disruption of the JNK3 gene resulted in a null allele.

The JNK3(-/-) mice were fertile and of normal size. Histological surveys of a variety of tissues revealed no apparent abnormality using hematoxylin and eosin (H & E) staining of heart, lung, thymus, spleen, lymph nodes, liver, kidney, and skeletal muscle. JNK3(-/-) and wild-type mouse brains were examined by immunocytochemical analysis of a pyramidyl neuronal marker (MAP-2), interneuronal markers calbindin and parvalbumin, an astrocyte marker (glial fibrillary acidic protein; GFAP; Hsu et al., J. Histochem. Cytochem. 29:577-580, 1981), and Nissl's stain (Hsu et al., *supra).* These studies revealed that JNK3(-/-) mice had apparently normal development and structural organization of the brain. A comparable number of motor neurons were found in the facial nucleus in wild-type and JNK3(-/-) mice (2150-2300 neurons per nucleus at postnatal day 10, n=4). The neurons were identified by morphology and were counted by a double-blind assay of serial sections throughout the facial nuclei of wild-type and JNK3(-/-) mice. Thus, there is no apparent developmental abnormality, including cell death, in JNK3 (-/-) mice.

### Example 3. JNK3 Deficient Mice are Resistant to KA-Induced Seizures

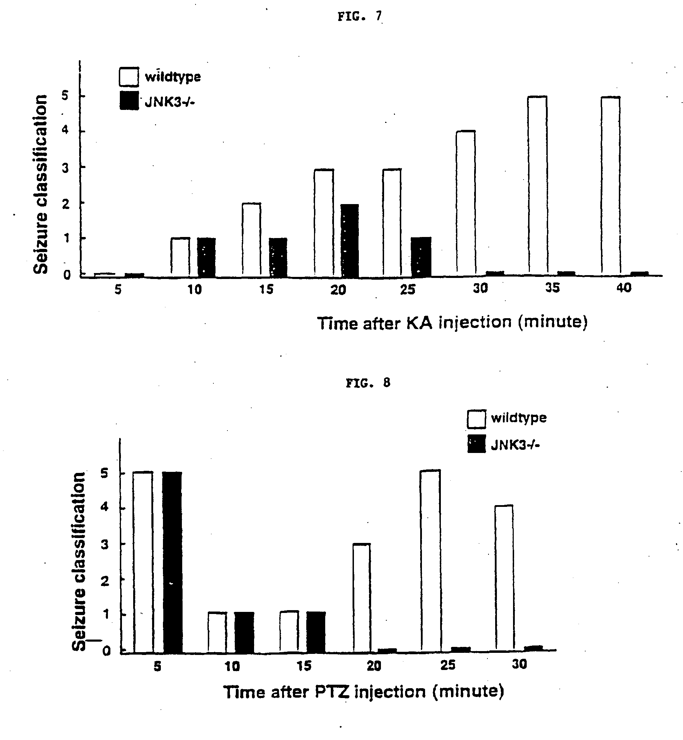

JNK3(-/-) mice and their wild-type littermates were injected intraperitoneally (i.p.) with 30 mg/kg KA to induce seizures (Ben-Ari, *supra*). In wild-type mice, the administration of KA first induced "staring spells" with abnormal body posture, then progressed to head nodding ("wet-dog shakes"), fore-paw tremor, rearing, loss of postural control, and eventually, continuous convulsions. The seizure activities typically subsided one hour after injection. Wild-type and heterozygous mice developed motor symptoms of seizures, including rearing, at 30 to 40 minutes post-injection. The JNK3 (-/-) mice, in contrast, developed much milder symptoms, mainly consisting of "staring" spells and occasional myoclonic tremors. At this dose, JNK3 (-/-) mice did not develop grand mal seizures and recovered much faster than did wild-type and heterozygous mice. JNK3(-/-) mice developed seizures of comparable severity to wild-type mice only at higher dosages of KA (45 mg/kg, i.p.). However, at this high dose of KA, more than 60% of wild-type mice died from continuous tonic clonic convulsions, while all of the JNK3(-/-) mice survived. These results indicate that JNK3(-/-) mice were resistant to the effect of the excitotoxin KA. Further, JNK3(-/-) mice recovered from the drug administration more rapidly than wild-type mice (Fig. 7). Seizure classifications as shown in Figs. 7 and 8 are: 1, arrest of motion; 2, myoclonic jerks of the head and neck, with brief twitching movements; 3, unilateral clonic activity; 4, bilateral forelimb tonic and clonic activity; and 5, generalized tonic-clonic activity with loss of postural tone, often resulting in death.

### Example 4. Resistance to Pentetrazole (PTZ)-Induced Seizures

Since the resistance to KA-induced seizures varied between littermates (+/+ and +/- are less resistant than -/- mice), the observed differential susceptibility cannot be attributed to a difference between mouse strains (Schauwecker and Steward, Proc. Nat. Acad. Sci. USA 94:4103-4108, 1997). However, the resistance of JNK3 deficient mice to KA-induced seizures could be due to decreased drug penetration across the blood-brain barrier or an increased GABA (gamma-aminobutyric acid) inhibitory postsynaptic potential (IPSP), or the ablation of a specific signal transduction pathway mediated by the JNK3 protein kinase. To distinguish between these possibilities, the response of JNK3 (-/-) and wild-type mice to another epileptogenic agent, pentetrazole (PTZ) (Sigma), was examined. PTZ was selected due to its ability to induce seizures by blocking the GABA-IPSPs (Ben-Ari et al., Neurosci. 6:1361-1391, 1981).

JNK3(-/-) mice and wild-type littermates developed seizures of comparable severity at all tested dosages of PTZ (30, 40, 50, 60 mg/kg, i.p.; Fig. 8). Moreover, unlike the slow progression of motor symptoms seen in the KA-induced seizures, PTZ induced abrupt general tonic-clonic seizures within five minutes after injection, presumably reflecting that its epileptogenic mechanism works solely through extracellularly inhibition of the GABA-IPSP. Thus, the differential susceptibility to KA toxicity in JNK3(-/-) mice can neither be explained as a consequence of poor drug delivery to the nervous system nor by potent GABA-IPSPs in the neural circuit. Furthermore, we examined the expression of the kainate-type glutamate receptor subunits GluR5-7 (Pharmingen cat. no. 60006E) by immunocytochemistry using standard methods.

Pyramidal neurons in the hippocampal CA1 subfield were most prominently labeled by the Glu5-7 antibody. Both wild-type and JNK3(-/-) mice showed prominently labeled apical dendrites arising from lightly labeled somata in the CA1 subfield of the hippocampus, a pattern similar to the primate hippocampus (Good et al., Brain Research 624:347-353, 1993). In addition to kainate-type subunit GluR5-7, the expression pattern of the GluR1 subunit that is essential to various glutamate receptor subtypes and the intracellular calcium-binding proteins parvalbumin and calbindin that may buffer the influx of extracellular calcium were also indistinguishable between JNK3(-/-) and wild-type mice. Together, these results indicate no apparent structural abnormality that might be responsible for the resistance of JNK3(-/-) mice to KA-induced excitotoxicity.

### Example 5. Attenuation of KA-Induced Phosphorylation of c-Jun

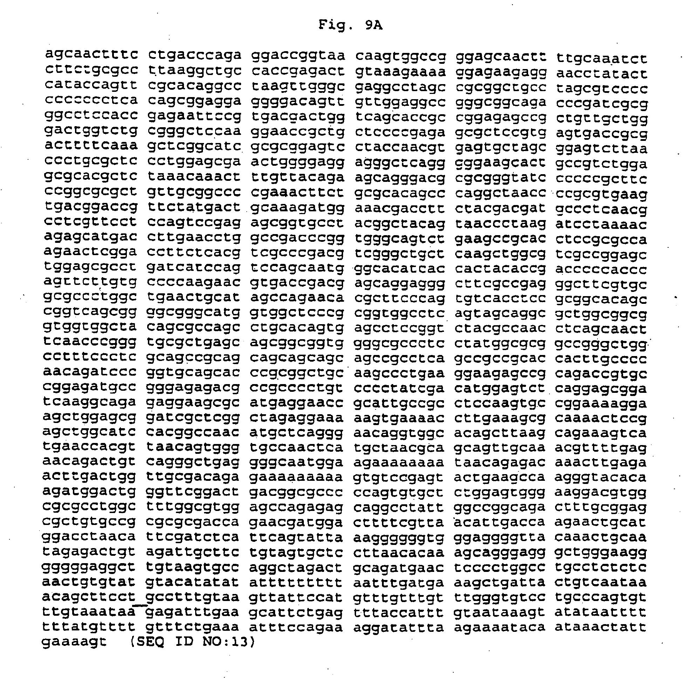

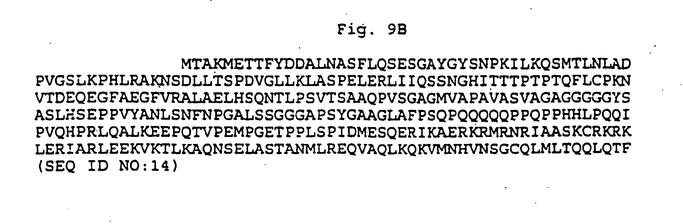

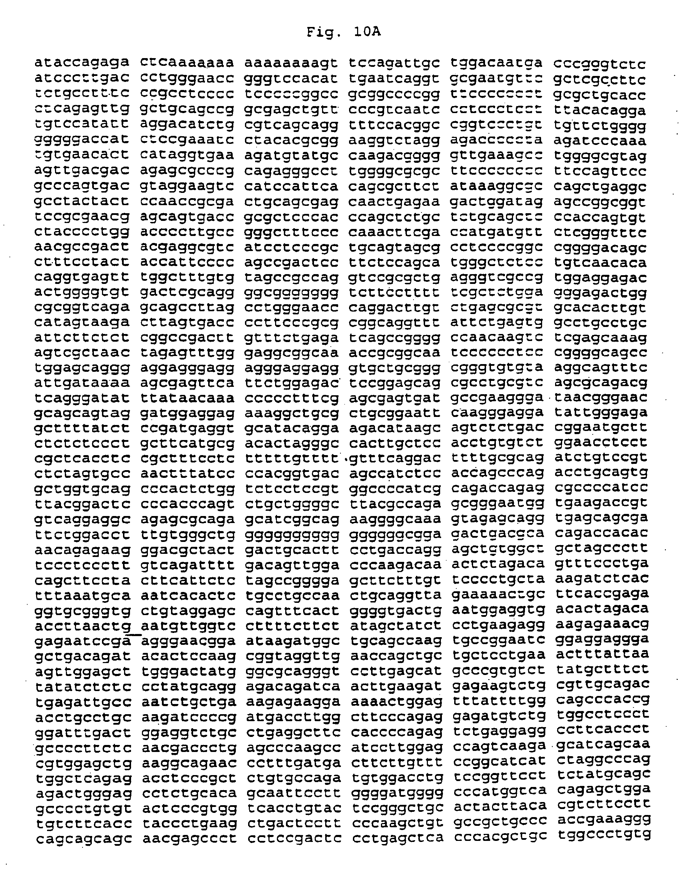

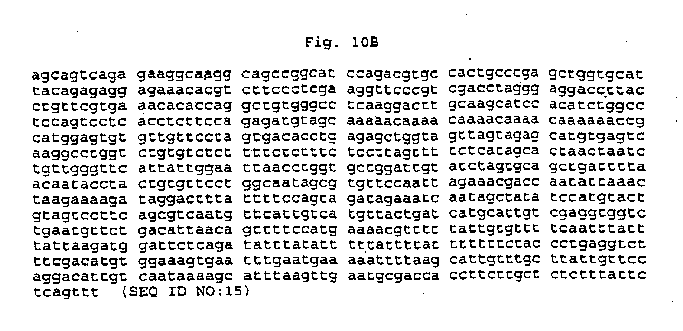

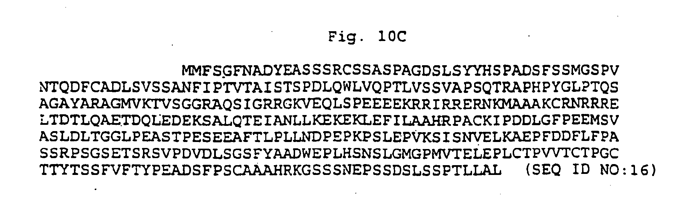

The systemic administration of KA in wild-type mice may induce a stress-response pathway mediated by the JNK3 protein kinase. To explore this possibility, the expression of the immediate-early genes *c-fos* and *c-jun* (Morgan et al., Annu. Rev. Neurosci. 14:421-451, 1991; Smeyne et al., Nature 363:166-169, 1993; Kasof et al., J. Neurosci. 15:4238-4249, 1995) was examined to determine whether KA imposed an equivalent level of noxious stimulation on wild-type and JNK3(-/-) mice. Total RNA was extracted from the hippocampi of mice sacrificed before and at 0.5, 2, 4, or 8 hours after KA injection (30 mg/kg, i.p.), and Northern blots were probed with murine c-fos and c-jun probes. The c-Jun probe was a 207bp fragment corresponding to nucleotides 888-1094 (Fig. 9) of the murine c-Jun cDNA. The c-Fos probe was a 347 bp fragment of the murine c-Fos gene (exon 4; base pairs 2593-2939) (Fig. 10). Both JNK3(-/-) and wild type mice exhibited a comparable level of rapid induction of c-fos and c-jun transcripts, which gradually declined four hours after injection.

To further define this phenomenon, the distribution of KA-induced c-Fos and c-Jun immunoreactivity along the synaptic circuit of the hippocampus was examined. In these experiments, homozygous mutant and control wild-type mice were killed and fixed by transcardial perfusion of 4% paraformaldehyde at 2 or 6 hours after the injection of KA (30 mg/kg, i.p.). Brains from both groups were removed, post-fixed for one hour, and sectioned on a Vibratome (40 mm thick). Tissue sections were processed by immunocytochemistry to detect the expression of c-Jun (Santa Cruz, cat# sc-45), c-Fos (Santa Cruz, cat# sc-52), and phospho-specific c-Jun (Ser-73) (New England Biolabs, #9164S). Sections were floated in a solution of the primary antibody (diluted 200X in PBS) and incubated overnight at room temperature. Secondary antibody incubation, avidin-biotin conjugated peroxidase (Vectastain Elite ABC kit, Vector Lab.), and DAB (3, 3'-diaminobenzidine, Sigma) reactions were performed using standard procedures (Hsu et al., *supra*)*.* In the absence of KA, there was no detectable c-Fos expression and only a few c-Jun-positive cells within the dentate gyrus. Two hours after KA injection (30 mg/kg, i.p.), there was a large increase in c-Fos immunoreactivity throughout the hippocampal region that was the same in both wild-type and JNK3(-/-) mice. Simultaneously, there was an increase in the number of c-Jun-positive cells in the dentate gyrus and the CA3 region of the hippocampus in both wild type and JNK3(-/-) mice. By six hours after KA injection, the expression of c-Jun extended to the CA1 region in both wild-type and JNK3(-/-) mice. The induction of c-Fos and C-Jun is generally accepted as an indicator of neuronal activity following noxious stimulation (Morgan et al., *supra*)*.* The comparable induction level, time-course, and distribution of c-Jun and c-Fos-labeled cells suggests that JNK3(-/-) and wild type mice were subject to an equivalent level of noxious stress by systemic administration of KA.

C-Jun is activated by phosphorylation of the NH₂-terminal activation domain by JNK. The expression of phosphorylated c-Jun provides another measure of whether JNK-like activity was present in JNK3(-/-) mice. The expression of phosphorylated c-Jun was examined using an antibody raised against c-Jun phosphorylated at Ser-73, one of the sites phosphorylated by JNK (Whitmarsh et al., *supra*; Dèrijard et al., *supra*; Kyriakis et al., *supra*). Prior to challenge with KA, no cells were labeled by the antibody in either wild type or JNK3(-/-) mice. By two hours after KA injection, there was a high level of phosphorylated c-Jun in the dentate gyrus and the CA3/CA4 region of the hippocampus in wild-type mice. In contrast, only a trace amount of phosphorylated c-Jun was detected in the JNK3(-/-) mice. Thus, there was either a decreased level or less sustained phosphorylation of c-Jun in the JNK3(-/-) mice.

In addition, there was a dynamic change of the distribution of phosphorylated c-Jun in the wild type mouse hippocampus. By six hours after KA injection, the expression of phosphorylated c-Jun subsided in the dentate gyrus and progressed to a restricted area in the hippocampal CA3 region. Under higher magnification, it was apparent that the expression of phosphorylated c-Jun surrounded a focus of cell destruction. In contrast, no labeling of phosphorylated c-Jun was detected in the JNK3(-/-) mice at the same time point. The hippocampal CA3 region is well documented as the most vulnerable structure to the KA excitotoxicity, presumably due to both a high KA binding affinity (Berger et al., *supra*) and a potent excitatory synaptic connection between CA3 pyramidal neurons (Westbrook et al., Brain Research 273:97-109, 1983). These results indicate that JNK3 is required for the phosphorylation of c-Jun induced by KA.

### Example 6. Attenuation of KA-Induced AP-1

### Transcriptional Activity

Since the phosphorylation of c-Jun is an important initial event during the induction of AP-1 transcriptional activity (Whitemarsh et al., *supra;* Yang et al., *supra*), whether the observed attenuation of c-Jun phosphorylation would lead to decreased induction of AP-1 transcriptional activity in JNK3 (-/-) mice was examined. JNK(-1-) mice were crossed with transgenic AP-1 luciferase (AP1-luc) mice (Rincon et al., Embo. J. 13:4370-4381, 1994) and progeny back crossed. The JNK3(-/-)/API-Luc(-/+) mice were used in experiments with JNK3(+/+) mice to compare the level of KA-induced AP-1 transcriptional activity in the presence or absence of JNK3. The AP1-luc mice contain the firefly luciferase gene under the control of four copies of a consensus AP-1 binding site in the context of the minimal rat prolactin promoter. It has been established that the expression of luciferase in these mice is due to the presence of the AP-1 regulatory element.

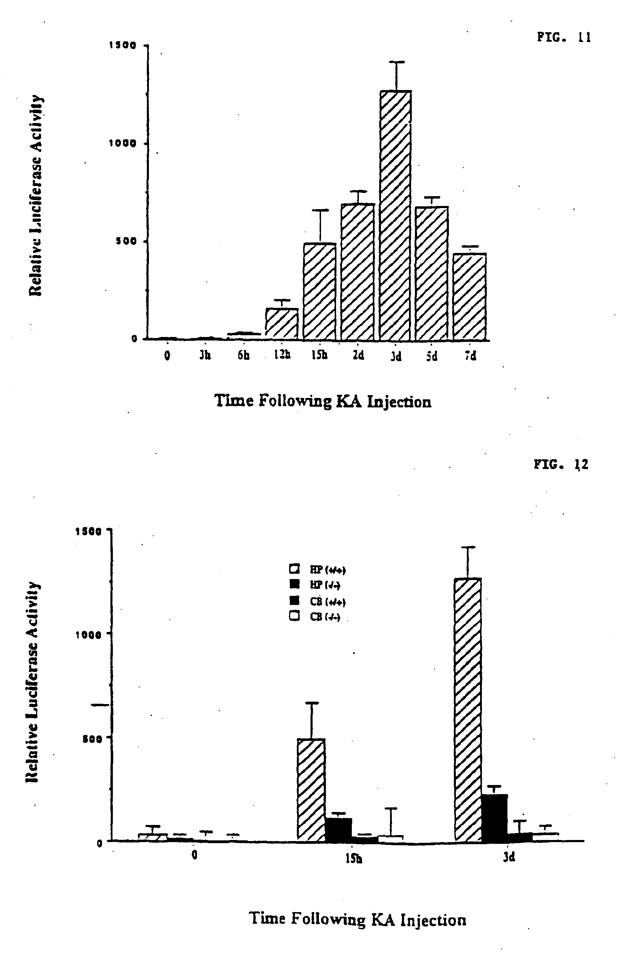

In the luciferase assay, mice were sacrificed at intervals after injection of KA (30 mg/kg, i.p.), and relative luciferase activity compared with that detected in hippocampal lysates obtained from mice injected with vehicle (saline). Mice were decapitated, brains were dissected, and brain tissues were immediately lysed in buffer containing 25mM Hepes pH 7.4, 1% TRITON^{⊙}X-100, 1mM EDTA, 1mM phenylmethyl sulfonyl fluoride, and 10 µg/ml leupeptin (Promega, Madison, WI). Luciferase activity was measured as described in Rincon and Flavell (Embo. J. 13:4370-4381, 1994). The injection of KA (30 mg/kg, i.p.) caused a large induction of AP-1 transcriptional activity in the hippocampus of wild-type mice, as evidenced by the induction of luciferase activity. Luciferase activity in wild type mice was detectable by six hours, gradually increased to the peak at three days, and persisted for at least seven days (Fig. 11). Control experiments demonstrated that the injection of vehicle (saline) did not cause induction of luciferase activity in the AP1-luc mice.

The relative luciferase activity in the hippocampus and cerebellum prepared from wild-type (+/+) and JNK3 (-/-) mice was measured following KA injection. The results are shown in Fig. 12. Each time point represents the mean of three to five (+ SEM) individual animals. The induction of luciferase activity was most prominent in the hippocampus, where a markedly greater induction of phosphorylation of c-Jun was observed, as compared to the cerebellum and the cerebral cortex. The induction of AP-1 activity was significantly reduced in the JNK3 (-/-) mice with the AP1-luc transgene as compared to the wild type mice. At 15 hours after injection of KA, there was approximately four-fold greater AP-1 activity in the hippocampus of wild-type mice compared with JNK3(-/-) mice. At three days after injection, the AP-1 activity was more than six times higher in the hippocampus of wild-type compared with JNK3(-/-) mice. Together, these data demonstrate that the disruption of the JNK3 gene suppressed KA-induced phosphorylation of c-Jun and AP-1 transcription activity in the hippocampus *in vivo.*

### Example 7. Resistance to KA-induced Apoptosis

One unique feature of KA among other epileptogenic agents is its potency in inducing neuronal cell death (Ben-Ari, *supra*; Schwob et al., *supra).* Since this property of cell destruction is paralleled by a sustained level of AP-1 transcriptional activity, it has been suggested that AP-1 mediates KA -induced-neuronal death (Kasof et al., *supra*; Schwarzschild et al., J. of Neurosci. 17:3455-3466, 1997). Wild-type and JNK3(-/-) mouse brains were therefore examined after treatment with KA to determine whether the attenuation of AP-1 transcriptional activity in JNK3 (-/-) mice altered the extent of neuronal damage (Ben-Ari, *supra*; Ben-Ari et al., *supra;* Schwob et al., Neurosci. 5:991-1014, 1980).

These experiments were performed as follows. Wild-type and JNK3 (-/-) mice were killed and fixed by transcardial perfusion of 4% paraformaldehyde and 1.5% glutaraldehyde three days after the injection of KA (30 mg/kg, i.p.). Semithin and thin sections of brain were prepared using a Vibratome and embedded in Epon. Tissue blocks were prepared using a microtome with a diamond tube for 1 µm-thick semithin sections examined by toluidine blue staining, and for ultrathin sections examined by electron microscopy. Nissl's stain was used for initial examination of damage to the hippocampus (Kluver et al., J. Neuropath. Exp. Neuro. 12:400-403, 1953). GFAP immunocytochemistry was also used to assess cell destruction in the hippocampus (Hsu et al., *supra*). Nissl's staining was also performed as described above. TUNEL assays, used to evaluate apoptosis, were performed using cryostat sections (50µm) of cerebral hemispheres that were cryoprotected with sucrose. The TUNEL assay was modified from the terminal deoxynucleotidyl transferase (TdT)-mediated dUTP nick end labeling assay (Gavrieli et al., J. Cell. Biol. 119:493-501, 1992). Briefly, tissue sections, directly mounted on a salinated slide, were permeablized with 2% TRITON^{®} X-100 (20 minutes at room temperature) and then incubated for nick end-labeling for 2 hours at 37°C using 0.32 U/µl TdT (Boehringer Mannheim, cat# 220582) and 2µM digoxigenin-11-dUTP (Boehringer Mannheim, cat# 1573152) in a final volume of 40 µl. The tissues were incubated with anti-digoxigenin antibody (Boehringer Mannheim, cat# 1333062) diluted 500-fold, and processed for immunocytochemistry using standard procedures (Hsu et al., *supra*).

The damage to the hippocampus caused by KA was initially examined by Nissl's stain. The KA-induced cell loss caused either a breach of staining of the pyramidal neurons in the CA3 region or a diffuse sparse staining throughout the CA1 subfield. To corroborate the cell destruction revealed by Nissl's stain, the TUNEL method was applied to detect apoptosis. Groups of small pyknotic nuclei and positively TUNEL-labeled cells were found in the hippocampal CA3 subfield devoid of Nissl's staining. Similarly, a high percentage of pyramidal neurons showing pyknotic nuclei and shattered apical dendrites and numerous strongly TUNEL-labeled cells were located in the hippocampal CA1 subfield which exhibited decreased Nissl's staining. Since the TUNEL method and the pyknosis morphology only indicated the extent of cell damage at one time point of a dynamic process, immunostaining of damage-induced GFAP was also used as an independent assessment of the extent of cell destruction in the hippocampus. Consistent with the patterns of Nissl's, toluidine, and TUNEL staining, an increased number of strongly GFAP-labeled astrocytes was found either in the hippocampal CA3 or CA1 regions. Thus, a combination of Nissl's stain, GFAP immunocytochemistry, TUNEL method, and toluidine stain of semithin sections was used to classify the KA-induced damage in the mouse hippocampus.

A total of 17 wild-type and 18 JNK3(-/-) mice were examined. Results are shown in Table 1 (below). The table was compiled from two sets of data. First, wild-type (n=11) and JNK3 (-/-) (n=10) mice were sacrificed on the fifth day after a single injection of KA (30 mg/kg, i.p.). Second, wild-type (n=6) and JNK3 (-/-) (n=8) mice received an injection of KA (30 mg/kg, i. p.) for five consecutive days and examined two days following the final injection. The severity of the hippocampal damage in wild-type mice was comparable in experiments using both protocols. The ratio of no cell loss/CA3 lesion/CA3+CA1 lesion was 2/7/2 in the single-injection experiments, and 2/2/2 in the multiple injection experiments.

| **TABLE 1 Kainate-induced neuronal damage (number of animals)** | | |
|---|---|---|
| **JNK3 genotype** | **+/+** | **-/-** |
| No perceivable cell loss | 4 | 18 |
| Selective CA3 cell loss | 9 | 0 |
| Including CA1 cell loss | 4 | 0 |

The hippocampal CA3 region was the most susceptible to KA-induced damage in the wild-type mice (9/17; 53%). Cell loss was indicated by decreased crystal violet staining in the CA3 region. Using the TUNEL method (Gavrieli et al., *supra*)*,* which identifies DNA fragmentation in the dying cells, groups of labeled cells were found in the damaged region. A cluster of pyknotic nuclei was found in the CA3 region in toluidine-stained semithin sections. As a result of KA-induced damage, there was selective glial proliferation confined to the CA3 region, as indicated by the strong immunostaining of GFAP. In some wild-type animals, massive cell loss was observed throughout the entire hippocampal CA1 region (4/17; 24%). Similarly, damage to the CA1 region was revealed by decreased crystal violet staining, positively TUNEL-labeled cells, pyknotic nuclei, shattered apical dendrites of pyramidal neurons, and both hypertrophy and proliferation of GFAP-positive astrocytes.

In contrast, there was no apparent hippocampal damage in any of the JNK3(-/-) mice examined (n=18). The pattern of the Nissl's stain, TUNEL assay, toluidine blue staining of semithin sections, and GFAP immunostaining of the hippocampal region in the JNK3(-/-) mice was indistinguishable from that of untreated wild-type mice. Moreover, although JNK2(-/-) mice developed seizures of comparable severity at the sublethal dose of 45 mg/kg KA (a dose that is lethal for more than 60% of wild-type mice due to continuous convulsions), cell damage was nevertheless found in a much smaller percentage of animals (2/15, 13%; p<0.005 by chi-square analysis, d.f.=1).

Methods of assessing apoptosis (e.g., TUNEL assay) can be used to evaluate whether JNK3 modulator is affecting apoptosis.

### Example 8. Electron Microscopic Analysis of Ultrastructural Changes Associated with KA-Induced Neuronal Damage

Cortical neurons *in vitro* undergo either apoptosis or necrosis depending on the extracellular concentration of the glutamate analog N-methyl-D-aspartate (NMDA) (Bonfoco et al., Proc. Natl. Acad. Sci. USA 92:7162-7166, 1995). The distinction between apoptosis versus necrosis in KA-induced neuronal damage is critical since necrosis is generally thought to represent a consequence of acute mechanical insult that is incompatible with an active cell death program involving *de novo* protein synthesis. The TUNEL results (supra) indicate the involvement of apoptosis. To further examine whether the neuronal death *in vivo* due to KA induction was apoptotic or necrotic, electron microscopy was employed to investigate the ultrastructural changes in the degenerated hippocampal neurons. The microscopic analysis suggested a series of morphological changes indicating neuronal damage in the wild-type mouse as a consequence of apoptosis. The initial event after KA injection (30 mg/kg i.p.) appeared to be compaction and segregation of chromatin in pyramidal neurons into electron-dense masses that abutted on the inner surface of the nuclear envelope. In contrast, the nuclei of the hippocampal neurons in the JNK3(-/-) mice following KA injection contained homogeneous electron-lucent euchromatin. At later stages, in wild type mice, there was convolution of the nuclear outline and condensation of the cytoplasm. The double-layered structure of the nuclear envelope in wild type mice remained largely intact in all of these morphological stages. Eventually the degenerated neurons disintegrated resulting in numerous membrane-bounded apoptotic bodies. These morphological features are all consistent with the hallmarks of apoptosis (Kerr et al., Br. J. Cancer 26:239-257, 1972). Thus, it appeared that KA triggered a genetic program within the damaged neuron leading to apoptosis, which was abrogated in JNK3-deficient neurons.

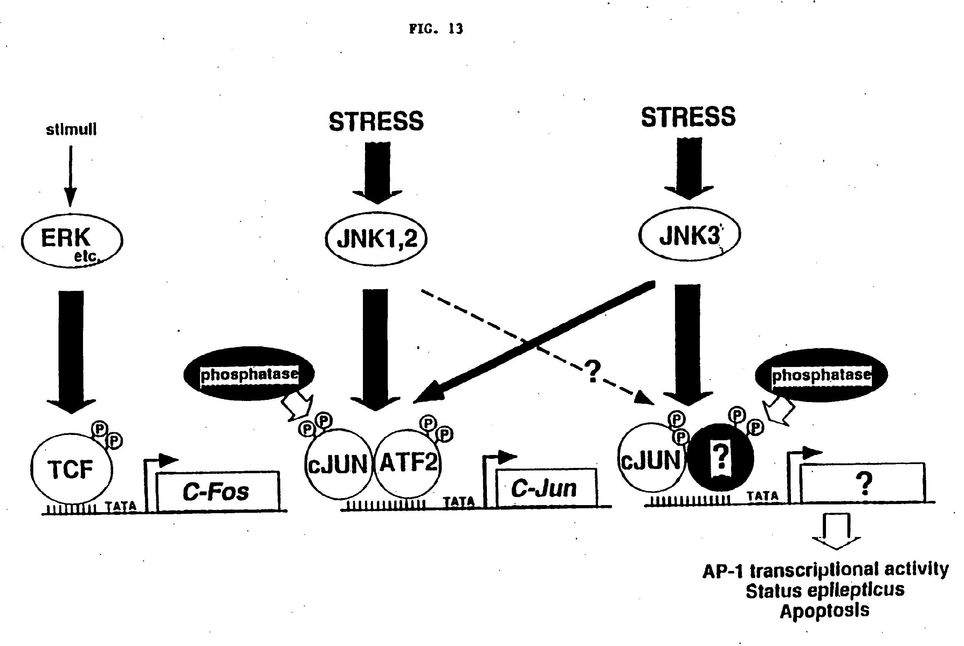

These results suggest that KA-induced phosphorylation of the NH₂-terminal activation domain of c-Jun leads to increased AP-1 transcriptional activity and neuronal apoptosis. Without limitation to a particular theory, a proposed chain of molecular events caused by KA that lead to neuronal apoptosis is shown in Fig. 13.

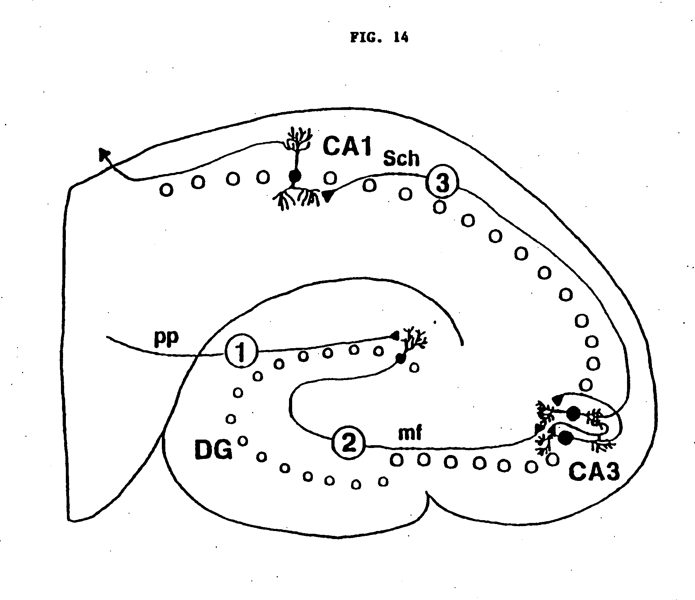

Although systemic administration of KA causes cell damage predominantly localized in the hippocampal CA3 area, the significance of JNK3 in stress-induced neuronal apoptosis is not only restricted to this region. Several lines of evidence indicate that the particular vulnerability of the CA3 hippocampal neurons to KA is due to their unique cellular and synaptic properties. First, the hippocampal CA3 and CA4 regions have the highest density of KA-receptors (Berger et al., Neurosci. Lett. 39:237-242, 1983). Second, the recurrent synaptic excitation is particularly potent in the hippocampal CA3 region (Miles et al., J. Physiol. (London) 373:397-418, 1986). The recurrent excitation of the CA3 pyramidal neurons may sustain JNK3 signaling and therefore rapidly induce KA excitotoxicity. The observed progression of c-Jun phosphorylation from the dentate gyrus to the CA3 region is reminiscent of the synaptic circuitry of the hippocampus. A diagram of the trisynaptic connection within the hippocampal formation is shown in Fig. 14. The first synaptic relay (1) is from the afferent perforant path (pp) onto the granule cell of the dentate gyrus (DG). The second relay (2) follows the mossy fiber (mf) from the dentate gyrus to the CA3 hippocampal neurons. The third relay (3) is from the hippocampal CA3 to the CA1 region along the Schaffer collaterals (Sch). There are recurrent synaptic interactions of pyramidal neurons in the CA3 region.

### Example 9. Assays for Detection of Inhibitors of JNK3 Protein Kinase Activity

Inhibitors of JNK3 can be identified in protein kinase assays. These assays can be performed using JNK3 purified from tissue (e.g., brain) or with recombinant enzyme. The recombinant JNK3 can be isolated from bacteria, yeast, insect, or mammalian cells using standard procedures. Assays of endogenous (natural) JNK3 are known in the art and assays of recombinant JNK3 have been described previously (Gupta et al., EMBO J. 15:2760-2770, 1996).

The protein kinase activity of JNK3 can be measured using ATP and protein substrates for JNK3 in an *in vitro* assay. These substrates include, but are not limited to, the transcription factors ATF2 and Elk-1 (Gupta et al., 1996, *supra*)*.* The incorporation of phosphate into the substrate can be measured by several methods. One example is to measure the incorporation of radioactive phosphate (e.g., ³²P) into the substrate. The incorporation into the substrate can be measured following removal of unincorporated radioactivity by precipitation with trichloroacetic acid and recovery on phosphocellulose paper or by polyacrylamide gel electrophoresis. The radioactivity can be monitored by scintillation counting, phosphorimager analysis, or by autoradiography. In general, methods for automated high throughput screens would not use radioactive materials. For this purpose a method is used to detect the phosphorylated substrate without a radioactive probe. In one approach the electrophoretic mobility of the substrate is examined. For example, ATF2 demonstrates a marked reduction in electrophoretic mobility following phosphorylation by JNK on Thr-69 and Thr-71 (Gupta et al., Science 267:389-393, 1995).

A second approach is to detect the phosphorylation of the substrate using immunochemical methods (e.g. ELISA). Antibodies that bind specifically to the phosphorylated substrates are prepared (monoclonal and polyclonal) and are commercially available (e.g., New England Biolabs, Promega Corp., and Upstate Biotechnology Inc.). The extent of substrate phosphorylation is then measured by standard ELISA assay using secondary antibodies coupled molecules suitable for to spectrophotometric or fluorometric detection using methods known in the art.

Molecules that inhibit JNK3 can be identified in a high throughput screen. A molecule that is a preferred candidate to treat excitotoxic disorders inhibits JNK3, but not other protein kinases, including related MAP kinases. Candidate molecules once identified can be optimized using combinatorial chemical methods or by the synthesis of related molecules. These molecules represent candidate drugs that can be tested for JNK3 therapy.

### Example 10. Assays for Detection of Inhibitors of JNK3 Activation

The JNK protein kinases are activated by dual phosphorylation on Thr and Tyr within protein kinase sub-domain VIII (Davis, Trends Biochem. Sci. 19:470-473, 1994). These sites of activating phosphorylation are conserved in JNK3 (Gupta et al., 1996, *supra*)*.* Molecules that inhibit the activation of JNK3 by interfering with the phosphorylation of JNK3 can be identified by measurement of JNK3 activation in the presence and absence of candidate molecules. Cells expressing JNK3, e.g., neuronal cells, neuroendocrine cells, or cells that are engineered to express recombinant JNK3 (Gupta et al., 1996 *supra*)*,* are exposed to environmental stress (e.g., depolarization, excitotoxic agents, UV radiation, heat, and anoxia) to activate JNK3. The state of JNK3 activation can be assessed by several methods. For example, JNK3 can be isolated, washed free of the candidate inhibitor, and the activation state of JNK3 monitored by protein kinase assay (*supra*)*.* Alternatively, the activation of JNK3 can be probed using immunological methods using antibodies that bind to the Thr and Tyr phosphorylated (activated) form of JNK3. Antibodies that bind specifically to the Thr and Tyr phosphorylated enzyme can be prepared (monoclonal and polyclonal) and are commercially available (e.g., from New England Biolabs and Promega Corp.). The extent of substrate phosphorylation can then be measured by a standard ELISA assay using secondary antibodies coupled to spectrophotometric or fluorometric detection.

### Other Embodiments

It is to be understood that while the invention has been described in conjunction with the detailed description thereof, the foregoing description is intended to illustrate and not limit the scope of the invention, which is defined by the scope of the appended claims.

## Claims

1. A method for identifying a molecule that modulates excitotoxicity, the method comprising:
(a) exposing a cell expressing c-Jun N-terminal kinase (JNK3) to environmental stress in the presence of at least one candidate molecule; and
(b) assessing the state of JNK3 phosphorylation,
wherein a molecule that interferes with phosphorylation of JNK3 modulates excitotoxicity.

2. The method according to claim 1, wherein the cell is a neuronal cell.

3. A method of identifying a compound that modulates excitotoxicity, the method comprising:
incubating a cell that can express a JNK3 protein with a compound under conditions and for a time sufficient for the cell to express a JNK3 protein absent the compound;
incubating a control cell under the same conditions and for the same time absent the compound;
measuring JNK3 expression in the first cell in the presence of the compound;
measuring JNK3 expression in the control cell; and
comparing the amount of JNK3 expression in the presence and absence of the compound,
wherein a difference in the level of expression indicates that the compound modulates excitotoxicity.

4. A method of identifying a compound that modulates excitotoxicity, the method comprising:
incubating a cell that has JNK3 activity with a compound under conditions and for a time sufficient for the cell to express JNK3 activity absent the compound;
incubating a control cell under the same conditions and for the same time absent the compound;
measuring JNK3 activity in the first cell in the presence of the compound;
measuring JNK3 activity in the control cell; and comparing the amount of JNK3 activity in the presence and absence of the compound,
wherein a difference in the level of activity indicates that the compound modulates excitotoxicity.

5. A method of identifying a compound that modulates excitotoxicity, the method comprising:
incubating a cell that can express a JNK3 protein with a compound under conditions of environmental stress for a time sufficient for the cell to express a JNK3 protein absent the compound;
determining JNK3 expression; and
comparing the amount of JNK3 expression in the presence and absence of the compound,
wherein a difference in the level of expression indicates that the compound modulates excitotoxicity.

6. A method of identifying a compound that modulates excitotoxicity, the method comprising:
incubating a cell that has JNK3 activity with a compound under conditions of environmental stress for a time sufficient for the cell to express JNK3 activity absent the compound;
determining JNK3 activity;
and comparing the amount of JNK3 activity in the presence and absence of the compound,
wherein a difference in the level of activity indicates that the compound modulates excitotoxicity.

7. The method according to any one of claims 1-6, wherein the state of JNK3 phosphorylation, JNK3 activation, the JNK3 expression, or the JNK3 activity is measured by determining the level of phosphorylated c-Jun in the cell.

8. The method according to any one of claims 1-6, wherein the state of JNK3 phosphorylation, JNK3 activation, the JNK3 expression, or the JNK3 activity is measured by determining the level of AP-1 transcription activity in the cell.

9. A method of identifying a compound that modulates excitotoxicity, said method comprising comparing the amount of a JNK3 polypeptide bound to a substrate in the presence and absence of a selected compound, wherein a difference in the amount of binding of a JNK3 polypeptide to a substrate indicates that said selected compound modulates excitotoxicity.

## Patentansprüche

1. Ein Verfahren zur Identifikation eines Moleküls das die Excitoxizität moduliert, das Verfahren umfassend:
(a) Aussetzen einer Zelle, die c-Jun N-terminale Kinase (JNK3) exprimiert, gegenüber Umweltstress in der Gegenwart von wenigstens einem Kandidatenmolekül; und
(b) Untersuchung des Status der JNK3-Phosphorylierung,
wobei ein Molekül, das die Phosphorylierung von JNK3 behindert, die Excitoxizität moduliert.

2. Das Verfahren gemäß Anspruch 1, worin die Zelle eine Nervenzelle ist.

3. Ein Verfahren zur Identifikation einer Verbindung, die die Excitoxizität moduliert, das Verfahren umfassend:
Inkubation einer Zelle, die ein JNK3-Protein exprimieren kann, mit einer Verbindung unter Bedingungen und für eine Zeitdauer, die für die Zelle ausreichend sind, um JNK3 in Abwesenheit der Verbindung zu exprimieren;
Inkubation einer Kontrollzelle unter denselben Bedingungen und für dieselbe Zeitdauer in Abwesenheit der Verbindung;
Messen der JNK3-Expression in der ersten Zelle in der Anwesenheit der Verbindung;
Messen der JNK3-Expression in der Kontrollzelle; und
Vergleichen der Menge der JNK3-Expression in der Anwesenheit und Abwesenheit der Verbindung,
worin ein Unterschied im Level der Expression anzeigt, dass die Verbindung die Excitoxizität moduliert.

4. Ein Verfahren zur Identifikation einer Verbindung, die die Excitoxizität moduliert, das Verfahren umfassend:
Inkubation einer Zelle, die JNK3-Aktivität hat, mit einer Verbindung unter Bedingungen und für eine Zeitdauer, die für die Zelle ausreichend sind, um in Abwesenheit der Verbindung JNK3 zu exprimieren;
Inkubation einer Kontrollzelle unter denselben Bedingungen und für dieselbe Zeitdauer in Abwesenheit der Verbindung;
Messen der JNK3-Aktivität in der ersten Zelle in der Anwesenheit der Verbindung;
Messen der JNK3-Aktivität in der Kontrollzelle; und Vergleichen der Menge der JNK3-Aktivität in der Anwesenheit oder Abwesenheit der Verbindung,
worin ein Unterschied im Level der Aktivität anzeigt, dass die Verbindung die Excitoxizität moduliert.

5. Ein Verfahren zur Identifikation einer Verbindung, die die Excitoxizität moduliert, das Verfahren umfassend:
Inkubation einer Zelle, die JNK3-Protein exprimieren kann, mit einer Verbindung unter Bedingungen von Umweltstress, und für eine Zeitdauer, die für die Zelle ausreichend ist, JNK3-Protein in Abwesenheit der Verbindung zu exprimieren;
Bestimmen der JNK3-Expression; und
Vergleichen der Menge der JNK3-Expression in der Anwesenheit und Abwesenheit der Verbindung,
worin ein Unterschied im Level der Expression anzeigt, dass die Verbindung die Excitoxizität moduliert.

6. Ein Verfahren zur Identifikation einer Verbindung, die die Excitoxizität moduliert, das Verfahren umfassend:
Inkubation einer Zelle, die JNK3-Aktivität hat, mit einer Verbindung unter Bedingungen von Umweltstress, und für eine Zeitdauer, die für die Zelle ausreichend ist, JNK3-Protein in Abwesenheit der Verbindung zu exprimieren;
Bestimmen der JNK3-Aktivität; und
Vergleichen der Menge der JNK3-Aktiviät in der Anwesenheit und Abwesenheit der Verbindung, worin ein Unterschied im Level der Aktivität anzeigt, dass die Verbindung die Excitoxizität moduliert.

7. Das Verfahren gemäß eines der Ansprüche 1-6, worin der Status der JNK3-Phosphorylierung, JNK3-Aktivierung, der JNK3-Expression, oder der JNK3-Aktivität durch Bestimmung des Levels von phosphoryliertem c-Jun in der Zelle gemessen wird.

8. Das Verfahren gemäß einem der Ansprüche 1-6, worin der Status der JNK3-Phosphorylierung, JNK3-Aktivierung, der JNK3-Expression, oder der JNK3-Aktivität durch die Bestimmung des Levels der AP-1 Transkriptionsaktivität in der Zelle gemessen wird.

9. Ein Verfahren zur Identifikation einer Verbindung, die die Excitoxizität moduliert, das Verfahren umfassend das Vergleichen der Menge eines JNK3-Polypeptids, das in der Anwesenheit oder Abwesenheit einer ausgewählten Verbindung an ein Substrat gebunden wird, worin ein Unterschied in der Menge der Bindung eines JNK3-Polypeptids an ein Substrat anzeigt, dass die ausgewählte Verbindung die Excitoxizität moduliert.

## Revendications

1. Procédé d'identification d'une molécule qui module l'excitotoxicité, le procédé comprenant :
(a) l'exposition d'une cellule exprimant la c-Jun N-terminal kinase (JNK3) à un stress environnemental en présence d'au moins une molécule candidate ; et
(b) l'évaluation de l'état de phosphorylation de la JNK3,
dans lequel une molécule qui interfère avec la phosphorylation de la JNK3 module l'excitotoxicité.

2. Procédé selon la revendication 1, dans lequel la cellule est une cellule neuronale.

3. Procédé d'identification d'un composé qui module l'excitotoxicité, le procédé comprenant :
l'incubation d'une cellule qui peut exprimer une protéine JNK3 avec un composé dans des conditions et pendant une durée suffisante pour que la cellule exprime une protéine JNK3 sans le composé ;
l'incubation d'une cellule témoin dans les mêmes conditions et pendant la même durée sans le composé ;
la mesure de l'expression de la JNK3 par la première cellule en présence du composé ;
la mesure de l'expression de la JNK3 dans la cellule témoin ; et
la comparaison de la quantité d'expression de JNK3 en présence et en l'absence du composé,
une différence de niveau d'expression indiquant que le composé module l'excitotoxicité.

4. Procédé d'identification d'un composé qui module l'excitotoxicité, le procédé comprenant :
l'incubation d'une cellule qui a une activité JNK3 avec un composé dans des conditions et pendant une durée suffisante pour que la cellule exprime une activité JNK3 sans le composé ;
l'incubation d'une cellule témoin dans les mêmes conditions et pendant la même durée sans le composé ;
la mesure de l'activité JNK3 dans la première cellule en présence du composé ;
la mesure de l'activité JNK3 dans la cellule témoin ; et la comparaison de la quantité d'activité JNK3 en présence et en l'absence du composé,
une différence de niveau d'activité indiquant que le composé module l'excitotoxicité.

5. Procédé d'identification d'un composé qui module l'excitotoxicité, le procédé comprenant :
l'incubation d'une cellule qui peut exprimer une protéine JNK3 avec un composé dans des conditions de stress environnemental pendant une durée suffisante pour que la cellule exprime une protéine JNK3 sans le composé ;
la détermination de l'expression de la JNK3 ; et
la comparaison de la quantité d'expression de la JNK3 en présence et en l'absence du composé,
une différence de niveau d'expression indiquant que le composé module l'excitotoxicité.

6. Procédé d'identification d'un composé qui module l'excitotoxicité, le procédé comprenant :
l'incubation d'une cellule qui a une activité JNK3 avec un composé dans des conditions de stress environnemental pendant une durée suffisante pour que la cellule exprime une activité JNK3 sans le composé ;
la détermination de l'activité JNK3 ; et
la comparaison de la quantité d'activité JNK3 en présence et en l'absence du composé,
une différence de niveau d'activité indiquant que le composé module l'excitotoxicité.

7. Procédé selon l'une quelconque des revendications 1 à 6, dans lequel l'état de phosphorylation de la JNK3, l'activation de la JNK3, l'expression de la JNK3 ou l'activité de la JNK3 est mesuré en déterminant le taux de c-Jun phosphorylé dans la cellule.

8. Procédé selon l'une quelconque des revendications 1 à 6, dans lequel l'état de phosphorylation de la JNK3, l'activation de la JNK3, l'expression de la JNK3 ou l'activité de la JNK3 est mesuré en déterminant le niveau d'activité de transcription de l'AP-1 dans la cellule.

9. Procédé d'identification d'un composé qui module l'excitotoxicité, ledit procédé comprenant la comparaison de la quantité d'un polypeptide JNK3 lié à un substrat en présence ou en l'absence d'un composé sélectionné, une différence de quantité de liaison d'un polypeptide JNK3 à un substrat indiquant que ledit composé sélectionné module l'excitotoxicité.


### SEQUENCE LISTING

<110> University of Massachusetts
<120> JNK3 Modulators and Methods of Use
<130> 116-024T1
<140>
   <141>
<150> EP 98 954 937.3
   <151> 1998-10-05
<150> US 60/060,995
   <151> 1997-10-03
<160> 30
<170> PatentIn version 3.4
<210> 1
   <211> 1505
   <212> DNA
   <213> Homo sapiens
<220>
   <221> CDS
   <222> (68)..(1459)
<400> 1
   ttatgcaaga aactgttgaa ttagacccgt ttcctataga tgagaaacca tacaagctgt      60
   ggtattt atg agc ctc cat ttc tta tac tac tgc agt gaa cca aca ttg       109
           Met Ser Leu His Phe Leu Tyr Tyr Cys Ser Glu Pro Thr Leu
           1               5                   10
   gat gtg aaa att gcc ttt tgt cag gga ttc gat aaa caa gtg gat gtg       157
   Asp Val Lys Ile Ala Phe Cys Gln Gly Phe Asp Lys Gln Val Asp Val
   15                  20                  25                  30
   tca tat att gcc aaa cat tac aac atg agc aaa agc aaa gtt gac aac       205
   Ser Tyr Ile Ala Lys His Tyr Asn Met Ser Lys Ser Lys Val Asp Asn
                   35                  40                  45
   cag ttc tac agt gtg gaa gtg gga gac tca acc ttc aca gtt ctc aag       253
   Gln Phe Tyr Ser Val Glu Val Gly Asp Ser Thr Phe Thr Val Leu Lys
               50                  55                  60
   cgc tac cag aat cta aag cct att ggc tct ggg gct cag ggc ata gtt       301
   Arg Tyr Gln Asn Leu Lys Pro Ile Gly Ser Gly Ala Gln Gly Ile Val
           65                  70                  75
   tgt gcc gcg tat gat gct gtc ctt gac aga aat gtg gcc att aag aag       349
   Cys Ala Ala Tyr Asp Ala Val Leu Asp Arg Asn Val Ala Ile Lys Lys
       80                  85                  90
   ctc agc aga ccc ttt cag aac caa aca cat gcc aag aga gcg tac cgg       397
   Leu Ser Arg Pro Phe Gln Asn Gln Thr His Ala Lys Arg Ala Tyr Arg
   95                  100                 105                 110
   gag ctg gtc ctc atg aag tgt gtg aac cat aaa aac att att agt tta       445
   Glu Leu Val Leu Met Lys Cys Val Asn His Lys Asn Ile Ile Ser Leu
                   115                 120                 125
   tta aat gtc ttc aca ccc cag aaa acg ctg gag gag ttc caa gat gtt       493
   Leu Asn Val Phe Thr Pro Gln Lys Thr Leu Glu Glu Phe Gln Asp Val
               130                 135                 140
   tac tta gta atg gaa ctg atg gat gcc aac tta tgt caa gtg att cag       541
   Tyr Leu Val Met Glu Leu Met Asp Ala Asn Leu Cys Gln Val Ile Gln
           145                 150                 155
   atg gaa tta gac cat gag cga atg tct tac ctg ctg tac caa atg ttg       589
   Met Glu Leu Asp His Glu Arg Met Ser Tyr Leu Leu Tyr Gln Met Leu
       160                 165                 170
   tgt ggc att aag cac ctc cat tct gct gga att att cac agg gat tta       637
   Cys Gly Ile Lys His Leu His Ser Ala Gly Ile Ile His Arg Asp Leu
   175                 180                 185                 190
   aaa cca agt aac att gta gtc aag tct gat tgc aca ttg aaa atc ctg       685
   Lys Pro Ser Asn Ile Val Val Lys Ser Asp Cys Thr Leu Lys Ile Leu
                   195                 200                 205
   gac ttt gga ctg gcc agg aca gca ggc aca agc ttc atg atg act cca       733
   Asp Phe Gly Leu Ala Arg Thr Ala Gly Thr Ser Phe Met Met Thr Pro
               210                 215                 220
   tat gtg gtg aca cgt tat tac aga gcc cct gag gtc atc ctg ggg atg       781
   Tyr Val Val Thr Arg Tyr Tyr Arg Ala Pro Glu Val Ile Leu Gly Met
           225                 230                 235
   ggc tac aag gag aac gtg gat ata tgg tct gtg gga tgc att atg gga       829
   Gly Tyr Lys Glu Asn Val Asp Ile Trp Ser Val Gly Cys Ile Met Gly
       240                 245                 250
   gaa atg gtt cgc cac aaa atc ctc ttt cca gga agg gac tat att gac       877
   Glu Met Val Arg His Lys Ile Leu Phe Pro Gly Arg Asp Tyr Ile Asp
   255                 260                 265                 270
   cag tgg aat aag gta att gaa caa cta gga aca cca tgt cca gaa ttc       925
   Gln Trp Asn Lys Val Ile Glu Gln Leu Gly Thr Pro Cys Pro Glu Phe
                   275                 280                 285
   atg aag aaa ttg caa ccc aca gta aga aac tat gtg gag aat cgg ccc       973
   Met Lys Lys Leu Gln Pro Thr Val Arg Asn Tyr Val Glu Asn Arg Pro
               290                 295                 300
   aag tat gcg gga ctc acc ttc ccc aaa ctc ttc cca gat tcc ctc ttc      1021
   Lys Tyr Ala Gly Leu Thr Phe Pro Lys Leu Phe Pro Asp Ser Leu Phe
           305                 310                 315
   cca gcg gac tcc gag cac aat aaa ctc aaa gcc agc caa gcc agg gac      1069
   Pro Ala Asp Ser Glu His Asn Lys Leu Lys Ala Ser Gln Ala Arg Asp
       320                 325                 330
   ttg ttg tca aag atg cta gtg att gac cca gca aaa aga ata tca gtg     1117
   Leu Leu Ser Lys Met Leu Val Ile Asp Pro Ala Lys Arg Ile Ser Val
   335                 340                 345                 350
   gac gac gcc tta cag cat ccc tac atc aac gtc tgg tat gac cca gcc     1165
   Asp Asp Ala Leu Gln His Pro Tyr Ile Asn Val Trp Tyr Asp Pro Ala
                   355                 360                 365
   gaa gtg gag gcg cct cca cct cag ata tat gac aag cag ttg gat gaa     1213
   Glu Val Glu Ala Pro Pro Pro Gln Ile Tyr Asp Lys Gln Leu Asp Glu
               370                 375                 380
   aga gaa cac aca att gaa gaa tgg aaa gaa ctt atc tac aag gaa gta     1261
   Arg Glu His Thr Ile Glu Glu Trp Lys Glu Leu Ile Tyr Lys Glu Val
           385                 390                 395
   atg aat tca gaa gaa aag act aaa aat ggt gta gta aaa gga cag cct     1309
   Met Asn Ser Glu Glu Lys Thr Lys Asn Gly Val Val Lys Gly Gln Pro
       400                 405                 410
   tct cct tca ggt gca gca gtg aac agc agt gag agt ctc cct cca tcc     1357
   Ser Pro Ser Gly Ala Ala Val Asn Ser Ser Glu Ser Leu Pro Pro Ser
   415                 420                 425                 430
   tcg tct gtc aat gac atc tcc tcc atg tcc acc gac cag acc ctg gca     1405
   Ser Ser Val Asn Asp Ile Ser Ser Met Ser Thr Asp Gln Thr Leu Ala
                   435                 440                 445
   tct gac act gac agc agc ctg gaa gcc tcg gca gga ccc ctg ggt tgt     1453
   Ser Asp Thr Asp Ser Ser Leu Glu Ala Ser Ala Gly Pro Leu Gly Cys
               450                 455                 460
   tgc agg tgactagccg cctgcctgcg aaacccagcg ttcttcagga gatgat          1505
   Cys Arg
<210> 2
   <211> 464
   <212> PRT
   <213> Homo sapiens
<400> 2
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305                 310                 315                 320
   Asp Ser Glu His Asn Lys Leu Lys Ala Ser Gln Ala Arg Asp Leu Leu
                   325                 330                 335
   Ser Lys Met Leu Val Ile Asp Pro Ala Lys Arg Ile Ser Val Asp Asp
               340                 345                 350
   Ala Leu Gln His Pro Tyr Ile Asn Val Trp Tyr Asp Pro Ala Glu Val
           355                 360                 365
   Glu Ala Pro Pro Pro Gln Ile Tyr Asp Lys Gln Leu Asp Glu Arg Glu
       370                 375                 380
   His Thr Ile Glu Glu Trp Lys Glu Leu Ile Tyr Lys Glu Val Met Asn
   385                 390                 395                 400
   Ser Glu Glu Lys Thr Lys Asn Gly Val Val Lys Gly Gln Pro Ser Pro
                   405                 410                 415
   Ser Gly Ala Ala Val Asn Ser Ser Glu Ser Leu Pro Pro Ser Ser Ser
               420                 425                 430
   Val Asn Asp Ile Ser Ser Met Ser Thr Asp Gln Thr Leu Ala Ser Asp
           435                 440                 445
   Thr Asp Ser Ser Leu Glu Ala Ser Ala Gly Pro Leu Gly Cys Cys Arg
       450                 455                 460
<210> 3
   <211> 464
   <212> PRT
   <213> Homo sapiens
<400> 3
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65                  70                  75                  80
   Ala Tyr Asp Ala Val Leu Asp Arg Asn Val Ala Ile Lys Lys Leu Ser
                   85                  90                  95
   Arg Pro Phe Gln Asn Gln Thr His Ala Lys Arg Ala Tyr Arg Glu Leu
               100                 105                 110
   Val Leu Met Lys Cys Val Asn His Lys Asn Ile Ile Ser Leu Leu Asn
           115                 120                 125
   Val Phe Thr Pro Gln Lys Thr Leu Glu Glu Phe Gln Asp Val Tyr Leu
       130                 135                 140
   Val Met Glu Leu Met Asp Ala Asn Leu Cys Gln Val Ile Gln Met Glu
   145                 150                 155                 160
   Leu Asp His Glu Arg Met Ser Tyr Leu Leu Tyr Gln Met Leu Cys Gly
                   165                 170                 175
   Ile Lys His Leu His Ser Ala Gly Ile Ile His Arg Asp Leu Lys Pro
               180                 185                 190
   Ser Asn Ile Val Val Lys Ser Asp Cys Thr Leu Lys Ile Leu Asp Phe
           195                 200                 205
   Gly Leu Ala Arg Thr Ala Gly Thr Ser Phe Met Met Thr Pro Tyr Val
       210                 215                 220
   Val Thr Arg Tyr Tyr Arg Ala Pro Glu Val Ile Leu Gly Met Gly Tyr
   225                 230                 235                 240
   Lys Glu Asn Val Asp Ile Trp Ser Val Gly Cys Ile Met Gly Glu Met
                   245                 250                 255
   Val Arg His Lys Ile Leu Phe Pro Gly Arg Asp Tyr Ile Asp Gln Trp
               260                 265                 270
   Asn Lys Val Ile Glu Gln Leu Gly Thr Pro Cys Pro Glu Phe Met Lys
           275                 280                 285
   Lys Leu Gln Pro Thr Val Arg Asn Tyr Val Glu Asn Arg Pro Lys Tyr
       290                 295                 300
   Ala Gly Leu Thr Phe Pro Lys Leu Phe Pro Asp Ser Leu Phe Pro Ala
   305                 310                 315                 320
   Asp Ser Glu His Asn Lys Leu Lys Ala Ser Gln Ala Arg Asp Leu Leu
                   325                 330                 335
   Ser Lys Met Leu Val Ile Asp Pro Ala Lys Arg Ile Ser Val Asp Asp
               340                 345                 350
   Ala Leu Gln His Pro Tyr Ile Asn Val Trp Tyr Asp Pro Ala Glu Val
           355                 360                 365
   Glu Ala Pro Pro Pro Gln Ile Tyr Asp Lys Gln Leu Asp Glu Arg Glu
       370                 375                 380
   His Thr Ile Glu Glu Trp Lys Glu Leu Ile Tyr Lys Glu Val Met Asn
   385                 390                 395                 400
   Ser Glu Glu Lys Thr Lys Asn Gly Val Val Lys Gly Gln Pro Ser Pro
                   405                 410                 415
   Ser Gly Ala Ala Val Asn Ser Ser Glu Ser Leu Pro Pro Ser Ser Ser
               420                 425                 430
   Val Asn Asp Ile Ser Ser Met Ser Thr Asp Gln Thr Leu Ala Ser Asp
           435                 440                 445
   Thr Asp Ser Ser Leu Glu Ala Ser Ala Gly Pro Leu Gly Cys Cys Arg
       450                 455                 460
<210> 4
   <211> 2366
   <212> DNA
   <213> Homo sapiens
<400> 4
   gagaaatggc gtggcagggg acccagcgag cccagaggga ttttgccgct gcttcctcta      60
   cccctgtatt tcacgcagct ctctaaattg actcagctcc aggctagtgt gagaaacacc     120
   acagcaggcc catctcagat cttcactatg gcaacttatg caagaaactg ttgaattaga     180
   cccgtttcct atagatgaga aaccatacaa gctgtggtat ttatgagcct ccatttctta    240
   tactactgca gtgaaccaac attggatgtg aaaattgcct tttgtcaggg attcgataaa    300
   caagtggatg tgtcatatat tgccaaacat tacaacatga gcaaaagcaa agttgacaac    360
   cagttctaca gtgtggaagt gggagactca accttcacag ttctcaagcg ctaccagaat    420
   ctaaagccta ttggctctgg ggctcagggc atagtttgtg ccgcgtatga tgctgtcctt    480
   gacagaaatg tggccattaa gaagctcagc agaccctttc agaaccaaac acatgccaag    540
   agagcgtacc gggagctggt cctcatgaag tgtgtgaacc ataaaaacat tattagttta    600
   ttaaatgtct tcacacccca gaaaacgctg gaggagttcc aagatgttta cttagtaatg    660
   gaactgatgg atgccaactt atgtcaagtg attcagatgg aattagacca tgagcgaatg    720
   tcttacctgc tgtaccaaat gttgtgtggc attaagcacc tccattctgc tggaattatt    780
   cacagggatt taaaaccaag taacattgta gtcaagtctg attgcacatt gaaaatcctg    840
   gactttggac tggccaggac agcaggcaca agcttcatga tgactccata tgtggtgaca    900
   cgttattaca gagcccctga ggtcatcctg gggatgggct acaaggagaa cgtggatata    960
   tggtctgtgg gatgcattat gggagaaatg gttcgccaca aaatcctctt tccaggaagg   1020
   gactatattg accagtggaa taaggtaatt gaacaactag gaacaccatg tccagaattc   1080
   atgaagaaat tgcaacccac agtaagaaac tatgtggaga atcggcccaa gtatgcggga   1140
   ctcaccttcc ccaaactctt cccagattcc ctcttcccag cggactccga gcacaataaa   1200
   ctcaaagcca gccaagccag ggacttgttg tcaaagatgc tagtgattga cccagcaaaa   1260
   agaatatcag tggacgacgc cttacagcat ccctacatca acgtctggta tgacccagcc   1320
   gaagtggagg cgcctccacc tcagatatat gacaagcagt tggatgaaag agaacacaca   1380
   attgaagaat ggaaagaact tatctacaag gaagtaatga attcagaaga aaagactaaa   1440
   aatggtgtag taaaaggaca gccttctcct tcaggtgcag cagtgaacag cagtgagagt   1500
   ctccctccat cctcgtctgt caatgacatc tcctccatgt ccaccgacca gaccctggca   1560
   tctgacactg acagcagcct ggaagcctcg gcaggacccc tgggttgttg caggtgacta   1620
   gccgcctgcc tgcgaaaccc agcgttcttc aggagatgat gtgatggaac acacacacac   1680
   gcagacacac acacacacac aaatgcagac acacaacatc aagaaaacag caagggagag   1740
   aatccaagcc taaaattaaa taaatctttc agcctgcttc ttccccaggg ttctgtattg   1800
   cagctaagct caaatgtata tttaacttct agttgctctt gctttggtct tcttccaatg   1860
   atgcttacta cagaaagcaa atcagacaca attagagaag ccttttccat aaagtgtaat   1920
   tttaatggct gcaaaaccgg caacctgtaa ctgccctttt aaatggcatg acaaggtgtg   1980
   cagtggcccc atccagcatg tgtgtgtctc tatcttgcat ctacctgctc cttggcctag   2040
   tcagatggat gtagatacag atccgcatgt gtctgtattc atacagcact acttacttag   2100
   agatgctact ctcagtgtcc tcagggctct accaagacat aatgcactgg ggtaccacat   2160
   ggtccatttc atgtgatcta ttactctgac ataaacccat ctgtaatata ttgccagtat   2220
   ataagctgtt tagtttgtta attgattaaa ctgtatgtct tataagaaaa catgtaaagg   2280
   gggaatatat tgggggagtg agctctctca gacccttgaa gatgtagctt ccaaatttga   2340
   atggattaaa tggcacctgt atacca                                        2366
<210> 5
   <211> 1773
   <212> DNA
   <213> Homo sapiens
<220>
   <221> CDS
   <222> (92)..(1357)
<400> 5
   atctcagatc ttcactatgg caacttatgc aagaaactgt tgaattagac ccgtttccta      60
   tagatgagaa accatacaag ctgtggtatt t atg agc ctc cat ttc tta tac        112
                                      Met Ser Leu His Phe Leu Tyr
                                      1               5
   tac tgc agt gaa cca aca ttg gat gtg aaa att gcc ttt tgt cag gga       160
   Tyr Cys Ser Glu Pro Thr Leu Asp Val Lys Ile Ala Phe Cys Gln Gly
           10                  15                  20
   ttc gat aaa caa gtg gat gtg tca tat att gcc aaa cat tac aac atg       208
   Phe Asp Lys Gln Val Asp Val Ser Tyr Ile Ala Lys His Tyr Asn Met
       25                  30                  35
   agc aaa agc aaa gtt gac aac cag ttc tac agt gtg gaa gtg gga gac       256
   Ser Lys Ser Lys Val Asp Asn Gln Phe Tyr Ser Val Glu Val Gly Asp
   40                  45                  50                  55
   tca acc ttc aca gtt ctc aag cgc tac cag aat cta aag cct att ggc       304
   Ser Thr Phe Thr Val Leu Lys Arg Tyr Gln Asn Leu Lys Pro Ile Gly
                   60                  65                  70
   tct ggg gct cag ggc ata gtt tgt gcc gcg tat gat gct gtc ctt gac       352
   Ser Gly Ala Gln Gly Ile Val Cys Ala Ala Tyr Asp Ala Val Leu Asp
               75                  80                  85
   aga aat gtg gcc att aag aag ctc agc aga ccc ttt cag aac caa aca       400
   Arg Asn Val Ala Ile Lys Lys Leu Ser Arg Pro Phe Gln Asn Gln Thr
           90                  95                  100
   cat gcc aag aga gcg tac cgg gag ctg gtc ctc atg aag tgt gtg aac       448
   His Ala Lys Arg Ala Tyr Arg Glu Leu Val Leu Met Lys Cys Val Asn
       105                 110                 115
   cat aaa aac att att agt tta tta aat gtc ttc aca ccc cag aaa acg       496
   His Lys Asn Ile Ile Ser Leu Leu Asn Val Phe Thr Pro Gln Lys Thr
   120                 125                 130                 135
   ctg gag gag ttc caa gat gtt tac tta gta atg gaa ctg atg gat gcc       544
   Leu Glu Glu Phe Gln Asp Val Tyr Leu Val Met Glu Leu Met Asp Ala
                   140                 145                 150
   aac tta tgt caa gtg att cag atg gaa tta gac cat gag cga atg tct       592
   Asn Leu Cys Gln Val Ile Gln Met Glu Leu Asp His Glu Arg Met Ser
               155                 160                 165
   tac ctg ctg tac caa atg ttg tgt ggc att aag cac ctc cat tct gct       640
   Tyr Leu Leu Tyr Gln Met Leu Cys Gly Ile Lys His Leu His Ser Ala
           170                 175                 180
   gga att att cac agg gat tta aaa cca agt aac att gta gtc aag tct       688
   Gly Ile Ile His Arg Asp Leu Lys Pro Ser Asn Ile Val Val Lys Ser
       185                 190                 195
   gat tgc aca ttg aaa atc ctg gac ttt gga ctg gcc agg aca gca ggc       736
   Asp Cys Thr Leu Lys Ile Leu Asp Phe Gly Leu Ala Arg Thr Ala Gly
   200                 205                 210                 215
   aca agc ttc atg atg act cca tat gtg gtg aca cgt tat tac aga gcc       784
   Thr Ser Phe Met Met Thr Pro Tyr Val Val Thr Arg Tyr Tyr Arg Ala
                   220                 225                 230
   cct gag gtc atc ctg ggg atg ggc tac aag gag aac gtg gat ata tgg       832
   Pro Glu Val Ile Leu Gly Met Gly Tyr Lys Glu Asn Val Asp Ile Trp
               235                 240                 245
   tct gtg gga tgc att atg gga gaa atg gtt cgc cac aaa atc ctc ttt       880
   Ser Val Gly Cys Ile Met Gly Glu Met Val Arg His Lys Ile Leu Phe
           250                 255                 260
   cca gga agg gac tat att gac cag tgg aat aag gta att gaa caa cta       928
   Pro Gly Arg Asp Tyr Ile Asp Gln Trp Asn Lys Val Ile Glu Gln Leu
       265                 270                 275
   gga aca cca tgt cca gaa ttc atg aag aaa ttg caa ccc aca gta aga       976
   Gly Thr Pro Cys Pro Glu Phe Met Lys Lys Leu Gln Pro Thr Val Arg
   280                 285                 290                 295
   aac tat gtg gag aat cgg ccc aag tat gcg gga ctc acc ttc ccc aaa      1024
   Asn Tyr Val Glu Asn Arg Pro Lys Tyr Ala Gly Leu Thr Phe Pro Lys
                   300                 305                 310
   ctc ttc cca gat tcc ctc ttc cca gcg gac tcc gag cac aat aaa ctc      1072
   Leu Phe Pro Asp Ser Leu Phe Pro Ala Asp Ser Glu His Asn Lys Leu
               315                 320                 325
   aaa gcc agc caa gcc agg gac ttg ttg tca aag atg cta gtg att gac      1120
   Lys Ala Ser Gln Ala Arg Asp Leu Leu Ser Lys Met Leu Val Ile Asp
           330                 335                 340
   cca gca aaa aga ata tca gtg gac gac gcc tta cag cat ccc tac atc      1168
   Pro Ala Lys Arg Ile Ser Val Asp Asp Ala Leu Gln His Pro Tyr Ile
       345                 350                 355
   aac gtc tgg tat gac cca gcc gaa gtg gag gcg cct cca cct cag ata      1216
   Asn Val Trp Tyr Asp Pro Ala Glu Val Glu Ala Pro Pro Pro Gln Ile
   360                 365                 370                 375
   tat gac aag cag ttg gat gaa aga gaa cac aca att gaa gaa tgg aaa      1264
   Tyr Asp Lys Gln Leu Asp Glu Arg Glu His Thr Ile Glu Glu Trp Lys
                   380                 385                 390
   gaa ctt atc tac aag gaa gta atg aat tca gaa gaa aag act aaa aat      1312
   Glu Leu Ile Tyr Lys Glu Val Met Asn Ser Glu Glu Lys Thr Lys Asn
               395                 400                 405
   ggt gta gta aaa gga cag cct tct cct tca gca cag gtg cag cag          1357
   Gly Val Val Lys Gly Gln Pro Ser Pro Ser Ala Gln Val Gln Gln
           410                 415                 420
   tgaacagcag tgagagtctc cctccatcct cgtctgtcaa tgacatctcc tccatgtcca    1417
   ccgaccagac cctggcatct gacactgaca gcagcctgga agcctcggca ggacccctgg    1477
   gttgttgcag gtgactagcc gcctgcctgc gaaacccagc gttcttcagg agatgatgtg    1537
   atggaacaca cacacacgca gacacacaca cacacacaaa tgcagacaca caacatcaag    1597
   aaaacagcaa gggagagaat ccaagcctaa aattaaataa atctttcagc ctgcttcttc    1657
   cccagggttc tgtattgcag ctaagctcaa atgtatattt aacttctagt tgctcttgct    1717
   ttggtcttct tccaatgatg cttactacag aaagcaaatc agacacaatt agagaa        1773
<210> 6
   <211> 422
   <212> PRT
   <213> Homo sapiens
<400> 6
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65                  70                  75                  80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Ala Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Gly Gln Pro Ser Pro
   405 410 415
   Ser Ala Gln Val Gln Gln
   420
<210> 7
   <211> 422
   <212> PRT
   <213> Homo sapiens
<400> 7
   Met Ser Leu His Phe Leu Tyr Tyr Cys Ser Glu Pro Thr Leu Asp Val
   1 5 10 15
   Lys Ile Ala Phe Cys Gln Gly Phe Asp Lys Gln Val Asp Val Ser Tyr
   20 25 30
   Ile Ala Lys His Tyr Asn Met Ser Lys Ser Lys Val Asp Asn Gln Phe
   35 40 45
   Tyr Ser Val Glu Val Gly Asp Ser Thr Phe Thr Val Leu Lys Arg Tyr
   50 55 60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Ala Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Gly Gln Pro Ser Pro
   405 410 415
   Ser Ala Gln Val Gln Gln
   420
<210> 8
   <211> 2372
   <212> DNA
   <213> Homo sapiens
<400> 8
   gagaaatggc gtggcagggg acccagcgag cccagaggga ttttgccgct gcttcctcta 60
   cccctgtatt tcacgcagct ctctaaattg actcagctcc aggctagtgt gagaaacacc 120
   aacagcaggc ccatctcaga tcttcactat ggcaacttat gcaagaaact gttgaattag 180
   acccgtttcc tatagatgag aaaccataca agctgtggta tttatgagcc tccatttctt 240
   atactactgc agtgaaccaa cattggatgt gaaaattgcc ttttgtcagg gattcgataa 300
   acaagtggat gtgtcatata ttgccaaaca ttacaacatg agcaaaagca aagttgacaa 360
   ccagttctac agtgtggaag tgggagactc aaccttcaca gttctcaagc gctaccagaa 420
   tctaaagcct attggctctg gggctcaggg catagtttgt gccgcgtatg atgctgtcct 480
   tgacagaaat gtggccatta agaagctcag cagacccttt cagaaccaaa cacatgccaa 540
   gagagcgtac cgggagctgg tcctcatgaa gtgtgtgaac cataaaaaca ttattagttt 600
   attaaatgtc ttcacacccc agaaaacgct ggaggagttc caagatgttt acttagtaat 660
   ggaactgatg gatgccaact tatgtcaagt gattcagatg gaattagacc atgagcgaat 720
   gtcttacctg ctgtaccaaa tgttgtgtgg cattaagcac ctccattctg ctggaattat 780
   tcacagggat ttaaaaccaa gtaacattgt agtcaagtct gattgcacat tgaaaatcct     840
   ggactttgga ctggccagga cagcaggcac aagcttcatg atgactccat atgtggtgac     900
   acgttattac agagcccctg aggtcatcct ggggatgggc tacaaggaga acgtggatat     960
   atggtctgtg ggatgcatta tgggagaaat ggttcgccac aaaatcctct ttccaggaag    1020
   ggactatatt gaccagtgga ataaggtaat tgaacaacta ggaacaccat gtccagaatt    1080
   catgaagaaa ttgcaaccca cagtaagaaa ctatgtggag aatcggccca agtatgcggg    1140
   actcaccttc cccaaactct tcccagattc cctcttccca gcggactccg agcacaataa    1200
   actcaaagcc agccaagcca gggacttgtt gtcaaagatg ctagtgattg acccagcaaa    1260
   aagaatatca gtggacgacg ccttacagca tccctacatc aacgtctggt atgacccagc    1320
   cgaagtggag gcgcctccac ctcagatata tgacaagcag ttggatgaaa gagaacacac    1380
   aattgaagaa tggaaagaac ttatctacaa ggaagtaatg aattcagaag aaaagactaa    1440
   aaatggtgta gtaaaaggac agccttctcc ttcagcacag gtgcagcagt gaacagcagt    1500
   gagagtctcc ctccatcctc gtctgtcaat gacatctcct ccatgtccac cgaccagacc    1560
   ctggcatctg acactgacag cagcctggaa gcctcggcag gacccctggg ttgttgcagg    1620
   tgactagccg cctgcctgcg aaacccagcg ttcttcagga gatgatgtga tggaacacac    1680
   acacacgcag acacacacac acacacaaat gcagacacac aacatcaaga aaacagcaag    1740
   ggagagaatc caagcctaaa attaaataaa tctttcagcc tgcttcttcc ccagggttct    1800
   gtattgcagc taagctcaaa tgtatattta acttctagtt gctcttgctt tggtcttctt    1860
   ccaatgatgc ttactacaga aagcaaatca gacacaatta gagaagcctt ttccataaag    1920
   tgtaatttta atggctgcaa aaccggcaac ctgtaactgc ccttttaaat ggcatgacaa    1980
   ggtgtgcagt ggccccatcc agcatgtgtg tgtctctatc ttgcatctac ctgctccttg    2040
   gcctagtcag atggatgtag atacagatcc gcatgtgtct gtattcatac agcactactt    2100
   acttagagat gctactctca gtgtcctcag ggctctacca agacataatg cactggggta    2160
   ccacatggtc catttcatgt gatctattac tctgacataa acccatctgt aatatattgc    2220
   cagtatataa gctgtttagt ttgttaattg attaaactgt atgtcttata agaaaacatg    2280
   taaaggggga atatattggg ggagtgagct ctctcagacc cttgaagatg tagcttccaa    2340
   atttgaatgg attaaatggc acctgtatac ca                                  2372
<210> 9
   <211> 2372
   <212> DNA
   <213> Homo sapiens
<220>
   <221> CDS
   <222> (224)..(1489)
<400> 9
   gagaaatggc gtggcagggg acccagcgag cccagaggga ttttgccgct gcttcctcta      60
   cccctgtatt tcacgcagct ctctaaattg actcagctcc aggctagtgt gagaaacacc     120
   aacagcaggc ccatctcaga tcttcactat ggcaacttat gcaagaaact gttgaattag     180
   acccgtttcc tatagatgag aaaccataca agctgtggta ttt atg agc ctc cat       235
                                                   Met Ser Leu His
                                                   1
   ttc tta tac tac tgc agt gaa cca aca ttg gat gtg aaa att gcc ttt       283
   Phe Leu Tyr Tyr Cys Ser Glu Pro Thr Leu Asp Val Lys Ile Ala Phe
   5                   10                  15                  20
   tgt cag gga ttc gat aaa caa gtg gat gtg tca tat att gcc aaa cat       331
   Cys Gln Gly Phe Asp Lys Gln Val Asp Val Ser Tyr Ile Ala Lys His
                   25                  30                  35
   tac aac atg agc aaa agc aaa gtt gac aac cag ttc tac agt gtg gaa       379
   Tyr Asn Met Ser Lys Ser Lys Val Asp Asn Gln Phe Tyr Ser Val Glu
               40                  45                  50
   gtg gga gac tca acc ttc aca gtt ctc aag cgc tac cag aat cta aag       427
   Val Gly Asp Ser Thr Phe Thr Val Leu Lys Arg Tyr Gln Asn Leu Lys
           55                  60                  65
   cct att ggc tct ggg gct cag ggc ata gtt tgt gcc gcg tat gat gct       475
   Pro Ile Gly Ser Gly Ala Gln Gly Ile Val Cys Ala Ala Tyr Asp Ala
       70                  75                  80
   gtc ctt gac aga aat gtg gcc att aag aag ctc agc aga ccc ttt cag       523
   Val Leu Asp Arg Asn Val Ala Ile Lys Lys Leu Ser Arg Pro Phe Gln
   85                  90                  95                  100
   aac caa aca cat gcc aag aga gcg tac cgg gag ctg gtc ctc atg aag       571
   Asn Gln Thr His Ala Lys Arg Ala Tyr Arg Glu Leu Val Leu Met Lys
                   105                 110                 115
   tgt gtg aac cat aaa aac att att agt tta tta aat gtc ttc aca ccc       619
   Cys Val Asn His Lys Asn Ile Ile Ser Leu Leu Asn Val Phe Thr Pro
               120                 125                 130
   cag aaa acg ctg gag gag ttc caa gat gtt tac tta gta atg gaa ctg       667
   Gln Lys Thr Leu Glu Glu Phe Gln Asp Val Tyr Leu Val Met Glu Leu
           135                 140                 145
   atg gat gcc aac tta tgt caa gtg att cag atg gaa tta gac cat gag       715
   Met Asp Ala Asn Leu Cys Gln Val Ile Gln Met Glu Leu Asp His Glu
       150                 155                 160
   cga atg tct tac ctg ctg tac caa atg ttg tgt ggc att aag cac ctc       763
   Arg Met Ser Tyr Leu Leu Tyr Gln Met Leu Cys Gly Ile Lys His Leu
   165                 170                 175                 180
   cat tct gct gga att att cac agg gat tta aaa cca agt aac att gta      811
   His Ser Ala Gly Ile Ile His Arg Asp Leu Lys Pro Ser Asn Ile Val
                   185                 190                 195
   gtc aag tct gat tgc aca ttg aaa atc ctg gac ttt gga ctg gcc agg      859
   Val Lys Ser Asp Cys Thr Leu Lys Ile Leu Asp Phe Gly Leu Ala Arg
               200                 205                 210
   aca gca ggc aca agc ttc atg atg act cca tat gtg gtg aca cgt tat      907
   Thr Ala Gly Thr Ser Phe Met Met Thr Pro Tyr Val Val Thr Arg Tyr
           215                 220                 225
   tac aga gcc cct gag gtc atc ctg ggg atg ggc tac aag gag aac gtg      955
   Tyr Arg Ala Pro Glu Val Ile Leu Gly Met Gly Tyr Lys Glu Asn Val
       230                 235                 240
   gat ata tgg tct gtg gga tgc att atg gga gaa atg gtt cgc cac aaa     1003
   Asp Ile Trp Ser Val Gly Cys Ile Met Gly Glu Met Val Arg His Lys
   245                 250                 255                 260
   atc ctc ttt cca gga agg gac tat att gac cag tgg aat aag gta att     1051
   Ile Leu Phe Pro Gly Arg Asp Tyr Ile Asp Gln Trp Asn Lys Val Ile
                   265                 270                 275
   gaa caa cta gga aca cca tgt cca gaa ttc atg aag aaa ttg caa ccc     1099
   Glu Gln Leu Gly Thr Pro Cys Pro Glu Phe Met Lys Lys Leu Gln Pro
               280                 285                 290
   aca gta aga aac tat gtg gag aat cgg ccc aag tat gcg gga ctc acc     1147
   Thr Val Arg Asn Tyr Val Glu Asn Arg Pro Lys Tyr Ala Gly Leu Thr
           295                 300                 305
   ttc ccc aaa ctc ttc cca gat tcc ctc ttc cca gcg gac tcc gag cac     1195
   Phe Pro Lys Leu Phe Pro Asp Ser Leu Phe Pro Ala Asp Ser Glu His
       310                 315                 320
   aat aaa ctc aaa gcc agc caa gcc agg gac ttg ttg tca aag atg cta     1243
   Asn Lys Leu Lys Ala Ser Gln Ala Arg Asp Leu Leu Ser Lys Met Leu
   325                 330                 335                 340
   gtg att gac cca gca aaa aga ata tca gtg gac gac gcc tta cag cat     1291
   Val Ile Asp Pro Ala Lys Arg Ile Ser Val Asp Asp Ala Leu Gln His
                   345                 350                 355
   ccc tac atc aac gtc tgg tat gac cca gcc gaa gtg gag gcg cct cca     1339
   Pro Tyr Ile Asn Val Trp Tyr Asp Pro Ala Glu Val Glu Ala Pro Pro
               360                 365                 370
   cct cag ata tat gac aag cag ttg gat gaa aga gaa cac aca att gaa     1387
   Pro Gln Ile Tyr Asp Lys Gln Leu Asp Glu Arg Glu His Thr Ile Glu
           375                 380                 385
   gaa tgg aaa gaa ctt atc tac aag gaa gta atg aat tca gaa gaa aag     1435
   Glu Trp Lys Glu Leu Ile Tyr Lys Glu Val Met Asn Ser Glu Glu Lys
       390                 395                 400
   act aaa aat ggt gta gta aaa gga cag cct tct cct tca gca cag gtg 1483
   Thr Lys Asn Gly Val Val Lys Gly Gln Pro Ser Pro Ser Ala Gln Val
   405 410 415 420
   cag cag tgaacagcag tgagagtctc cctccatcct cgtctgtcaa tgacatctcc 1539
   Gln Gln
   tccatgtcca ccgaccagac cctggcatct gacactgaca gcagcctgga agcctcggca 1599
   ggacccctgg gttgttgcag gtgactagcc gcctgcctgc gaaacccagc gttcttcagg 1659
   agatgatgtg atggaacaca cacacacgca gacacacaca cacacacaaa tgcagacaca 1719
   caacatcaag aaaacagcaa gggagagaat ccaagcctaa aattaaataa atctttcagc 1779
   ctgcttcttc cccagggttc tgtattgcag ctaagctcaa atgtatattt aacttctagt 1839
   tgctcttgct ttggtcttct tccaatgatg cttactacag aaagcaaatc agacacaatt 1899
   agagaagcct tttccataaa gtgtaatttt aatggctgca aaaccggcaa cctgtaactg 1959
   cccttttaaa tggcatgaca aggtgtgcag tggccccatc cagcatgtgt gtgtctctat 2019
   cttgcatcta cctgctcctt ggcctagtca gatggatgta gatacagatc cgcatgtgtc 2079
   tgtattcata cagcactact tacttagaga tgctactctc agtgtcctca gggctctacc 2139
   aagacataat gcactggggt accacatggt ccatttcatg tgatctatta ctctgacata 2199
   aacccatctg taatatattg ccagtatata agctgtttag tttgttaatt gattaaactg 2259
   tatgtcttat aagaaaacat gtaaaggggg aatatattgg gggagtgagc tctctcagac 2319
   ccttgaagat gtagcttcca aatttgaatg gattaaatgg cacctgtata cca 2372
<210> 10
   <211> 422
   <212> PRT
   <213> Homo sapiens
<400> 10
   Met Ser Leu His Phe Leu Tyr Tyr Cys Ser Glu Pro Thr Leu Asp Val
   1 5 10 15
   Lys Ile Ala Phe Cys Gln Gly Phe Asp Lys Gln Val Asp Val Ser Tyr
   20 25 30
   Ile Ala Lys His Tyr Asn Met Ser Lys Ser Lys Val Asp Asn Gln Phe
   35 40 45
   Tyr Ser Val Glu Val Gly Asp Ser Thr Phe Thr Val Leu Lys Arg Tyr
   50 55 60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Ala Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Gly Gln Pro Ser Pro
   405 410 415
   Ser Ala Gln Val Gln Gln
   420
<210> 11
   <211> 422
   <212> PRT
   <213> Homo sapiens
<400> 11
   Met Ser Leu His Phe Leu Tyr Tyr Cys Ser Glu Pro Thr Leu Asp Val
   1               5                   10                  15
   Lys Ile Ala Phe Cys Gln Gly Phe Asp Lys Gln Val Asp Val Ser Tyr
               20                  25                  30
   Ile Ala Lys His Tyr Asn Met Ser Lys Ser Lys Val Asp Asn Gln Phe
           35                  40                  45
   Tyr Ser Val Glu Val Gly Asp Ser Thr Phe Thr Val Leu Lys Arg Tyr
       50                  55                  60
   Gln Asn Leu Lys Pro Ile Gly Ser Gly Ala Gln Gly Ile Val Cys Ala
   65                  70                  75                  80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Asp Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Ala Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Gly Gln Pro Ser Pro
   405 410 415
   Ser Ala Gln Val Gln Gln
   420
<210> 12
   <211> 1975
   <212> DNA
   <213> Rattus norvegicus
<220>
   <221> CDS
   <222> (364)..(1641)
<400> 12
   ccctccttat tccggtttgg aatgtggcta atgaaagccc agtaggagga tttctggggc     60
   aaacaggtgg accaggatcc tggttctcag gcacggaatg gctattgtga gagcgccacc    120
   agcaggacca tcgcagatct tggttatggc tgctcacgca agaggctgtt gatgtagacc    180
   ccctttcccg tagatgagaa atcacacgag cagtggtatt tatgagcctc catttcttat    240
   actactgcag tgaaccaacc ttggatgtga aaattgcctt ttgtcaggtg tgtgttcctt    300
   acaggtaaaa caaagggatt cgacaaacac gtggatgtgt cttctgttgt caaacattac    360
   aac atg agc aaa agc aag gta gat aac cag ttc tac agt gtg gaa gtg      408
       Met Ser Lys Ser Lys Val Asp Asn Gln Phe Tyr Ser Val Glu Val
       1               5                   10                  15
   gga gac tca acc ttc aca gtt cta aag cgc tac cag aac ctg aag ccg      456
   Gly Asp Ser Thr Phe Thr Val Leu Lys Arg Tyr Gln Asn Leu Lys Pro
                   20                  25                  30
   atc ggc tct ggg gct cag gga ata gtt tgt gct gcg tat gac gct gtc      504
   Ile Gly Ser Gly Ala Gln Gly Ile Val Cys Ala Ala Tyr Asp Ala Val
               35                  40                  45
   ctc gac aga aat gtg gcc att aag aag ctc agc aga ccc ttc cag aac      552
   Leu Asp Arg Asn Val Ala Ile Lys Lys Leu Ser Arg Pro Phe Gln Asn
           50                  55                  60
   caa act cat gcc aag agg gct tac cgg gag ctg gtc ctc atg aag tgt      600
   Gln Thr His Ala Lys Arg Ala Tyr Arg Glu Leu Val Leu Met Lys Cys
       65                  70                  75
   gtg aac cat aaa aac att att agc tta tta aat gtc ttt aca ccc cag      648
   Val Asn His Lys Asn Ile Ile Ser Leu Leu Asn Val Phe Thr Pro Gln
   80                  85                  90                  95
   aaa aca ctg gag gag ttc caa gat gtt tac tta gtg atg gaa ctg atg      696
   Lys Thr Leu Glu Glu Phe Gln Asp Val Tyr Leu Val Met Glu Leu Met
                   100                 105                 110
   gac gcc aac ttg tgt cag gtg att cag atg gag ctg gac cac gag cgg      744
   Asp Ala Asn Leu Cys Gln Val Ile Gln Met Glu Leu Asp His Glu Arg
               115                 120                 125
   atg tcg tac ttg ctg tac cag atg ctg tcg gcg atc aaa cac ctc cac      792
   Met Ser Tyr Leu Leu Tyr Gln Met Leu Ser Ala Ile Lys His Leu His
           130                 135                 140
   tcc gct ggg atc atc cac agg gac tta aaa ccc agt aac atc gta gtc      840
   Ser Ala Gly Ile Ile His Arg Asp Leu Lys Pro Ser Asn Ile Val Val
       145                 150                 155
   aag tct gat tgc aca ctg aaa atc ctg gac ttt gga ctg gcc agg aca      888
   Lys Ser Asp Cys Thr Leu Lys Ile Leu Asp Phe Gly Leu Ala Arg Thr
   160                 165                 170                 175
   gcg ggc aca agc ttc atg atg act ccg tat gtg gtg acg aga tat tac      936
   Ala Gly Thr Ser Phe Met Met Thr Pro Tyr Val Val Thr Arg Tyr Tyr
                   180                 185                 190
   aga gcc ccc gag gtc atc ctg ggc atg ggc tac aag gag aac gtg gac      984
   Arg Ala Pro Glu Val Ile Leu Gly Met Gly Tyr Lys Glu Asn Val Asp
               195                 200                 205
   ata tgg tct gtg ggc tgc atc atg gga gaa atg gtt cgt cac aaa atc     1032
   Ile Trp Ser Val Gly Cys Ile Met Gly Glu Met Val Arg His Lys Ile
           210                 215                 220
   ctc ttt ccc gga agg gac tat att gac cag tgg aac aaa gtc ata gag     1080
   Leu Phe Pro Gly Arg Asp Tyr Ile Asp Gln Trp Asn Lys Val Ile Glu
       225                 230                 235
   cag cta gga act ccg tgt cca gaa ttc atg aag aaa ttg cag ccc acc     1128
   Gln Leu Gly Thr Pro Cys Pro Glu Phe Met Lys Lys Leu Gln Pro Thr
   240                 245                 250                 255
   gtc aga aac tac gtg gag aac cgg ccc aag tat gca ggc ctc acc ttc     1176
   Val Arg Asn Tyr Val Glu Asn Arg Pro Lys Tyr Ala Gly Leu Thr Phe
                   260                     265                 270
   ccc aag ctc ttt cca gat tcc ctc ttc cca gcg gat tcc gag cac aat     1224
   Pro Lys Leu Phe Pro Asp Ser Leu Phe Pro Ala Asp Ser Glu His Asn
               275                     280                 285
   aaa ctt aaa gcc agc caa gcc agg gac ttg ttg tca aag atg tta gtg     1272
   Lys Leu Lys Ala Ser Gln Ala Arg Asp Leu Leu Ser Lys Met Leu Val
           290                     295                 300
   att gac cca gcg aag agg ata tcg gtg gat gac gca ttg cag cat ccg     1320
   Ile Asp Pro Ala Lys Arg Ile Ser Val Asp Asp Ala Leu Gln His Pro
       305                     310                 315
   tac atc aac gtt tgg tac gac cct gct gaa gtg gag gcg cct ccg cct     1368
   Tyr Ile Asn Val Trp Tyr Asp Pro Ala Glu Val Glu Ala Pro Pro Pro
   320                     325                 330                 335
   cag ata tat gac aag caa ttg gat gaa agg gag cac acc atc gaa gaa     1416
   Gln Ile Tyr Asp Lys Gln Leu Asp Glu Arg Glu His Thr Ile Glu Glu
                   340                     345                 350
   tgg aaa gaa ctc atc tac aag gaa gta atg aac tca gaa gag aag act     1464
   Trp Lys Glu Leu Ile Tyr Lys Glu Val Met Asn Ser Glu Glu Lys Thr
               355                     360                 365
   aag aac ggc gta gtc aaa ggc cag ccc tca cct tca ggt gca gca gtg     1512
   Lys Asn Gly Val Val Lys Gly Gln Pro Ser Pro Ser Gly Ala Ala Val
           370                     375                 380
   aac agc agt gag agt ctc cct cca tcc tca tct gtc aac gac atc tcc     1560
   Asn Ser Ser Glu Ser Leu Pro Pro Ser Ser Ser Val Asn Asp Ile Ser
       385                     390                 395
   tcc atg tcc acc gac cag acc ctc gca tcc gac act gac agc agc ctg     1608
   Ser Met Ser Thr Asp Gln Thr Leu Ala Ser Asp Thr Asp Ser Ser Leu
   400                     405                 410                 415
   gaa gcc tcg gcg gga ccg ctg ggt tgt tgc agg tgactagccg cctgcctgcg   1661
   Glu Ala Ser Ala Gly Pro Leu Gly Cys Cys Arg
                   420                     425
   aaacccagcg ttcttcagga gatgacgcca tgatagaaca cagcgcacat gcacacacac   1721
   agagcttgta cacacacaca cacacacaca cacacgcacg cacgcacgca cgcaagcacg   1781
   cacgcacgca caaatgcact cacgcaatgt caagaaaaaa aaaagtagcg agagagagcg   1841
   agagagccaa cgtaaaacta agttaaatct ttctgcgtgc ttctccagag ttctgtatcg   1901
   cagctgagct gaaatgtata cttaacttct agtcgcgctc gctcgacttt ggtctccctc   1961
   cggcagtgct tact                                                     1975
<210> 13
   <211> 426
   <212> PRT
   <213> Rattus norvegicus
<400> 13
   Met Ser Lys Ser Lys Val Asp Asn Gln Phe Tyr Ser Val Glu Val Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Val Leu
           35                  40                  45
   Asp Arg Asn Val Ala Ile Lys Lys Leu Ser Arg Pro Phe Gln Asn Gln
       50                  55                  60
   Thr His Ala Lys Arg Ala Tyr Arg Glu Leu Val Leu Met Lys Cys Val
   65                  70                  75                  80
   Asn His Lys Asn Ile Ile Ser Leu Leu Asn Val Phe Thr Pro Gln Lys
                   85                  90                  95
   Thr Leu Glu Glu Phe Gln Asp Val Tyr Leu Val Met Glu Leu Met Asp
               100                 105                 110
   Ala Asn Leu Cys Gln Val Ile Gln Met Glu Leu Asp His Glu Arg Met
           115                 120                 125
   Ser Tyr Leu Leu Tyr Gln Met Leu Ser Ala Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Gly Thr Ser Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Ile
           195                 200                 205
   Trp Ser Val Gly Cys Ile Met Gly Glu Met Val Arg His Lys Ile Leu
       210                 215                 220
   Phe Pro Gly Arg Asp Tyr Ile Asp Gln Trp Asn Lys Val Ile Glu Gln
   225 230 235 240
   Leu Gly Thr Pro Cys Pro Glu Phe Met Lys Lys Leu Gln Pro Thr Val
   245 250 255
   Arg Asn Tyr Val Glu Asn Arg Pro Lys Tyr Ala Gly Leu Thr Phe Pro
   260 265 270
   Lys Leu Phe Pro Asp Ser Leu Phe Pro Ala Asp Ser Glu His Asn Lys
   275 280 285
   Leu Lys Ala Ser Gln Ala Arg Asp Leu Leu Ser Lys Met Leu Val Ile
   290 295 300
   Asp Pro Ala Lys Arg Ile Ser Val Asp Asp Ala Leu Gln His Pro Tyr
   305 310 315 320
   Ile Asn Val Trp Tyr Asp Pro Ala Glu Val Glu Ala Pro Pro Pro Gln
   325 330 335
   Ile Tyr Asp Lys Gln Leu Asp Glu Arg Glu His Thr Ile Glu Glu Trp
   340 345 350
   Lys Glu Leu Ile Tyr Lys Glu Val Met Asn Ser Glu Glu Lys Thr Lys
   355 360 365
   Asn Gly Val Val Lys Gly Gln Pro Ser Pro Ser Gly Ala Ala Val Asn
   370 375 380
   Ser Ser Glu Ser Leu Pro Pro Ser Ser Ser Val Asn Asp Ile Ser Ser
   385 390 395 400
   Met Ser Thr Asp Gln Thr Leu Ala Ser Asp Thr Asp Ser Ser Leu Glu
   405 410 415
   Ala Ser Ala Gly Pro Leu Gly Cys Cys Arg
   420 425
<210> 14
   <211> 426
   <212> PRT
   <213> Rattus norvegicus
<400> 14
   Met Ser Lys Ser Lys Val Asp Asn Gln Phe Tyr Ser Val Glu Val Gly
   1               5                   10                  15
   Asp Ser Thr Phe Thr Val Leu Lys Arg Tyr Gln Asn Leu Lys Pro Ile
               20                  25                  30
   Gly Ser Gly Ala Gln Gly Ile Val Cys Ala Ala Tyr Asp Ala Val Leu
           35                  40                  45
   Asp Arg Asn Val Ala Ile Lys Lys Leu Ser Arg Pro Phe Gln Asn Gln
       50                  55                  60
   Thr His Ala Lys Arg Ala Tyr Arg Glu Leu Val Leu Met Lys Cys Val
   65                  70                  75                  80
   Asn His Lys Asn Ile Ile Ser Leu Leu Asn Val Phe Thr Pro Gln Lys
                   85                  90                  95
   Thr Leu Glu Glu Phe Gln Asp Val Tyr Leu Val Met Glu Leu Met Asp
               100                 105                 110
   Ala Asn Leu Cys Gln Val Ile Gln Met Glu Leu Asp His Glu Arg Met
           115                 120                 125
   Ser Tyr Leu Leu Tyr Gln Met Leu Ser Ala Ile Lys His Leu His Ser
       130                 135                 140
   Ala Gly Ile Ile His Arg Asp Leu Lys Pro Ser Asn Ile Val Val Lys
   145                 150                 155                 160
   Ser Asp Cys Thr Leu Lys Ile Leu Asp Phe Gly Leu Ala Arg Thr Ala
                   165                 170                 175
   Gly Thr Ser Phe Met Met Thr Pro Tyr Val Val Thr Arg Tyr Tyr Arg
               180                 185                 190
   Ala Pro Glu Val Ile Leu Gly Met Gly Tyr Lys Glu Asn Val Asp Ile
           195                 200                 205
   Trp Ser Val Gly Cys Ile Met Gly Glu Met Val Arg His Lys Ile Leu
       210                 215                 220
   Phe Pro Gly Arg Asp Tyr Ile Asp Gln Trp Asn Lys Val Ile Glu Gln
   225                 230                 235                 240
   Leu Gly Thr Pro Cys Pro Glu Phe Met Lys Lys Leu Gln Pro Thr Val
                   245                 250                 255
   Arg Asn Tyr Val Glu Asn Arg Pro Lys Tyr Ala Gly Leu Thr Phe Pro
               260                 265                 270
   Lys Leu Phe Pro Asp Ser Leu Phe Pro Ala Asp Ser Glu His Asn Lys
           275                 280                 285
   Leu Lys Ala Ser Gln Ala Arg Asp Leu Leu Ser Lys Met Leu Val Ile
       290                 295                 300
   Asp Pro Ala Lys Arg Ile Ser Val Asp Asp Ala Leu Gln His Pro Tyr
   305                 310                 315                 320
   Ile Asn Val Trp Tyr Asp Pro Ala Glu Val Glu Ala Pro Pro Pro Gln
                   325                 330                 335
   Ile Tyr Asp Lys Gln Leu Asp Glu Arg Glu His Thr Ile Glu Glu Trp
               340                 345                 350
   Lys Glu Leu Ile Tyr Lys Glu Val Met Asn Ser Glu Glu Lys Thr Lys
           355                 360                 365
   Asn Gly Val Val Lys Gly Gln Pro Ser Pro Ser Gly Ala Ala Val Asn
       370                 375                 380
   Ser Ser Glu Ser Leu Pro Pro Ser Ser Ser Val Asn Asp Ile Ser Ser
   385                 390                 395                 400
   Met Ser Thr Asp Gln Thr Leu Ala Ser Asp Thr Asp Ser Ser Leu Glu
                   405                 410                 415
   Ala Ser Ala Gly Pro Leu Gly Cys Cys Arg
               420                 425
<210> 15
   <211> 2522
   <212> DNA
   <213> Mus musculus
<220>
   <221> CDS
   <222> (310)..(1575)
<400> 15
   ggggcttgag tgagctaaag attgggtctt cttggaaatc acctgtctgt tattattttt     60
   aaacaatcgc tacacctcca aagactctgc tccttactcc ggtttggaat gtggctaatg    120
   actacccagt agggaggatt tctggggcaa acagccggac caggatccta gttctcaggc    180
   acggaatggc tattgtgaga acagcaccag caggatcatc gcagatcttg gttatggcca    240
   ctcaggcaag acgctgttga gttaagaccc ctttcccata gatgagaagc cacagaagca    300
   gtggtattt atg agc ctc cat ttc tta tac tac tgc agt gaa cca acc ttg    351
             Met Ser Leu His Phe Leu Tyr Tyr Cys Ser Glu Pro Thr Leu
             1               5                   10
   gat gtg aaa att gcc ttt tgt cag gga ttc gat aaa cac gtg gat gtg      399
   Asp Val Lys Ile Ala Phe Cys Gln Gly Phe Asp Lys His Val Asp Val
   15                  20                  25                  30
   tca tct att gcc aaa cat tac aac atg agc aaa agc aag gtg gac aac      447
   Ser Ser Ile Ala Lys His Tyr Asn Met Ser Lys Ser Lys Val Asp Asn
                   35                  40                  45
   cag ttc tac agt gtg gaa gtg ggg gac tca acc ttc acc gtt ctt aag      495
   Gln Phe Tyr Ser Val Glu Val Gly Asp Ser Thr Phe Thr Val Leu Lys
               50                  55                  60
   cgc tac cag aac ctg aag cca att ggc tct ggg gct cag gga ata gtc      543
   Arg Tyr Gln Asn Leu Lys Pro Ile Gly Ser Gly Ala Gln Gly Ile Val
           65                  70                  75
   tgt gct gcg tac gac gct gtc ctt gac aga aat gtg gcc att aag aag      591
   Cys Ala Ala Tyr Asp Ala Val Leu Asp Arg Asn Val Ala Ile Lys Lys
       80                  85                  90
   ctc agc aga ccc ttc cag aac caa act cac gcc aag agg gct tac cgg      639
   Leu Ser Arg Pro Phe Gln Asn Gln Thr His Ala Lys Arg Ala Tyr Arg
   95                  100                 105                 110
   gag ctg gtg ctc atg aag tgt gtg aac cat aaa aac att att agc tta      687
   Glu Leu Val Leu Met Lys Cys Val Asn His Lys Asn Ile Ile Ser Leu
                   115                 120                 125
   tta aat gtt ttt aca ccc cag aaa acg ctg gag gag ttc caa gat gtc      735
   Leu Asn Val Phe Thr Pro Gln Lys Thr Leu Glu Glu Phe Gln Asp Val
               130                 135                 140
   tac tta gtg atg gaa ctg atg gac gcc aac ctg tgt cag gtg att cag      783
   Tyr Leu Val Met Glu Leu Met Asp Ala Asn Leu Cys Gln Val Ile Gln
           145                 150                 155
   atg gag ctg gac cac gag cgg atg tct tac ttg ctg tac cag atg ctg      831
   Met Glu Leu Asp His Glu Arg Met Ser Tyr Leu Leu Tyr Gln Met Leu
       160                 165                 170
   tgt ggc atc aag cac ctc cac tcc gct ggg atc atc cac agg gac tta      879
   Cys Gly Ile Lys His Leu His Ser Ala Gly Ile Ile His Arg Asp Leu
   175                 180                 185                 190
   aaa ccc agt aac att gta gtc aag tct gat tgc aca ctg aaa atc ctc        927
   Lys Pro Ser Asn Ile Val Val Lys Ser Asp Cys Thr Leu Lys Ile Leu
                   195                 200                 205
   gac ttc gga ctg gcc agg aca gcg ggt aca agc ttc atg atg act ccg        975
   Asp Phe Gly Leu Ala Arg Thr Ala Gly Thr Ser Phe Met Met Thr Pro
               210                 215                 220
   tat gtg gtg acg cga tat tac aga gcc cct gag gtc atc ctg ggc atg       1023
   Tyr Val Val Thr Arg Tyr Tyr Arg Ala Pro Glu Val Ile Leu Gly Met
           225                 230                 235
   ggc tac aag gag aac gtg gac ata tgg tct gtg gga tgc atc atg gga       1071
   Gly Tyr Lys Glu Asn Val Asp Ile Trp Ser Val Gly Cys Ile Met Gly
       240                 245                 250
   gaa atg gtt cgc cac aaa atc ctc ttt ccc gga agg agc tat att gac       1119
   Glu Met Val Arg His Lys Ile Leu Phe Pro Gly Arg Ser Tyr Ile Asp
   255                 260                 265                 270
   cag tgg aac aaa gtc atc gag cag cta gga act ccg tgt cca gag ttc       1167
   Gln Trp Asn Lys Val Ile Glu Gln Leu Gly Thr Pro Cys Pro Glu Phe
                   275                 280                 285
   atg aag aaa ttg cag ccc aca gtc aga aac tac gtg gag aat cgg ccc       1215
   Met Lys Lys Leu Gln Pro Thr Val Arg Asn Tyr Val Glu Asn Arg Pro
               290                 295                 300
   aag tac gca gga ctc acc ttc ccc aag ctc ttt cca gat tcc ctc ttc       1263
   Lys Tyr Ala Gly Leu Thr Phe Pro Lys Leu Phe Pro Asp Ser Leu Phe
           305                 310                 315
   cca gcg gat tct gag cac aat aaa ctt aaa gcc agc caa gcc agg gat       1311
   Pro Ala Asp Ser Glu His Asn Lys Leu Lys Ala Ser Gln Ala Arg Asp
       320                 325                 330
   ttg ttg tct aag atg tta gtg att gac cca gtg aag agg ata tcg gtg       1359
   Leu Leu Ser Lys Met Leu Val Ile Asp Pro Val Lys Arg Ile Ser Val
   335                 340                 345                 350
   gac gac gca ctg cag cat ccg tac atc aac gtt tgg tac gac ccg gct       1407
   Asp Asp Ala Leu Gln His Pro Tyr Ile Asn Val Trp Tyr Asp Pro Ala
                   355                 360                 365
   gaa gtg gag gcg cct ccg cct cag ata tat gat aag cag ctg gat gaa       1455
   Glu Val Glu Ala Pro Pro Pro Gln Ile Tyr Asp Lys Gln Leu Asp Glu
               370                 375                 380
   agg gag cac acc atc gaa gaa tgg aaa gaa ctt atc tac aag gag gta       1503
   Arg Glu His Thr Ile Glu Glu Trp Lys Glu Leu Ile Tyr Lys Glu Val
           385                 390                 395
   atg aac tca gaa gag aag act aag aat ggc gta gtc aaa agc cag ccc       1551
   Met Asn Ser Glu Glu Lys Thr Lys Asn Gly Val Val Lys Ser Gln Pro
       400                 405                 410
   tcg cct tca gca cag gtg cag cag tgaacagcag tgagagtctc cctccatcct      1605
   Ser Pro Ser Ala Gln Val Gln Gln
   415 420
   cggctgtcaa cgacatctcc tccatgtcca ccgaccagac cctcgcatct gacactgaca 1665
   gcagcctgga ggcctcggcg ggaccgttgg gttgttgcag gtgactagcc gcctgcctgc 1725
   gaaacccagc gttcttcagg agatgacgcg atagaacaca gcacacatgc acacacacag 1785
   cttgctctca cacacactca gcttgctcac acacacacac acacatacac acaaacacac 1845
   actgtctctc tctcacacac acacactgtc acaacgcact cacgaaaggt caagaaaaaa 1905
   ataacaatag agagatccaa cataaaatta agttaaattt ttctgcgtgc ttctccaaag 1965
   ttctgtatca cagctgagct gaaatgtata cttaacttct agttgcgctc gctttggttt 2025
   ccctccagca gtgcttacta cacaagacaa atcagacaca attagagaaa cctttcccta 2085
   aagtgtaact taagtggctg cagaaccagc aacctgtaac tgcccttcaa atggcatgag 2145
   gaggtgggca cgggtcccgc ccagcatgtg tgtgtctcta tctcgcgtct acctgctctt 2205
   ccggcctagt cagatggatg tagatacaga tcccgcatgt gtctgtattc aaacagcact 2265
   tagagatgct cctgtcagtg tcctccaggc tccaccaaga cacacaccgg ggtaccacat 2325
   ggtccatttc atgtgatcta ttactctgac ataaatccat ctgtaatata ttgccagtat 2385
   ataagctgtt tagtttgtta attgcttaag ctgtatgtct tataagagac tatgtaaagg 2445
   gggaaaatgg aggcgtgaac tctcagaccc ttgaagatgt agcttccgaa tttgaccgtt 2505
   aaatggcacc gtatacc 2522
<210> 16
   <211> 422
   <212> PRT
   <213> Mus musculus
<400> 16
   Met Ser Leu His Phe Leu Tyr Tyr Cys Ser Glu Pro Thr Leu Asp Val
   1 5 10 15
   Lys Ile Ala Phe Cys Gln Gly Phe Asp Lys His Val Asp Val Ser Ser
   20 25 30
   Ile Ala Lys His Tyr Asn Met Ser Lys Ser Lys Val Asp Asn Gln Phe
   35 40 45
   Tyr Ser Val Glu Val Gly Asp Ser Thr Phe Thr Val Leu Lys Arg Tyr
   50 55 60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Ser Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Val Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Ser Gln Pro Ser Pro
   405 410 415
   Ser Ala Gln Val Gln Gln
   420
<210> 17
   <211> 422
   <212> PRT
   <213> Mus musculus
<400> 17
   Met Ser Leu His Phe Leu Tyr Tyr Cys Ser Glu Pro Thr Leu Asp Val
   1 5 10 15
   Lys Ile Ala Phe Cys Gln Gly Phe Asp Lys His Val Asp Val Ser Ser
   20 25 30
   Ile Ala Lys His Tyr Asn Met Ser Lys Ser Lys Val Asp Asn Gln Phe
   35 40 45
   Tyr Ser Val Glu Val Gly Asp Ser Thr Phe Thr Val Leu Lys Arg Tyr
   50 55 60
   Gln Asn Leu Lys Pro Ile Gly Ser Gly Ala Gln Gly Ile Val Cys Ala
   65 70 75 80
   Ala Tyr Asp Ala Val Leu Asp Arg Asn Val Ala Ile Lys Lys Leu Ser
   85 90 95
   Arg Pro Phe Gln Asn Gln Thr His Ala Lys Arg Ala Tyr Arg Glu Leu
   100 105 110
   Val Leu Met Lys Cys Val Asn His Lys Asn Ile Ile Ser Leu Leu Asn
   115 120 125
   Val Phe Thr Pro Gln Lys Thr Leu Glu Glu Phe Gln Asp Val Tyr Leu
   130 135 140
   Val Met Glu Leu Met Asp Ala Asn Leu Cys Gln Val Ile Gln Met Glu
   145 150 155 160
   Leu Asp His Glu Arg Met Ser Tyr Leu Leu Tyr Gln Met Leu Cys Gly
   165 170 175
   Ile Lys His Leu His Ser Ala Gly Ile Ile His Arg Asp Leu Lys Pro
   180 185 190
   Ser Asn Ile Val Val Lys Ser Asp Cys Thr Leu Lys Ile Leu Asp Phe
   195 200 205
   Gly Leu Ala Arg Thr Ala Gly Thr Ser Phe Met Met Thr Pro Tyr Val
   210 215 220
   Val Thr Arg Tyr Tyr Arg Ala Pro Glu Val Ile Leu Gly Met Gly Tyr
   225 230 235 240
   Lys Glu Asn Val Asp Ile Trp Ser Val Gly Cys Ile Met Gly Glu Met
   245 250 255
   Val Arg His Lys Ile Leu Phe Pro Gly Arg Ser Tyr Ile Asp Gln Trp
   260 265 270
   Asn Lys Val Ile Glu Gln Leu Gly Thr Pro Cys Pro Glu Phe Met Lys
   275 280 285
   Lys Leu Gln Pro Thr Val Arg Asn Tyr Val Glu Asn Arg Pro Lys Tyr
   290 295 300
   Ala Gly Leu Thr Phe Pro Lys Leu Phe Pro Asp Ser Leu Phe Pro Ala
   305 310 315 320
   Asp Ser Glu His Asn Lys Leu Lys Ala Ser Gln Ala Arg Asp Leu Leu
   325 330 335
   Ser Lys Met Leu Val Ile Asp Pro Val Lys Arg Ile Ser Val Asp Asp
   340 345 350
   Ala Leu Gln His Pro Tyr Ile Asn Val Trp Tyr Asp Pro Ala Glu Val
   355 360 365
   Glu Ala Pro Pro Pro Gln Ile Tyr Asp Lys Gln Leu Asp Glu Arg Glu
   370 375 380
   His Thr Ile Glu Glu Trp Lys Glu Leu Ile Tyr Lys Glu Val Met Asn
   385 390 395 400
   Ser Glu Glu Lys Thr Lys Asn Gly Val Val Lys Ser Gln Pro Ser Pro
   405 410 415
   Ser Ala Gln Val Gln Gln
   420
<210> 18
   <211> 2347
   <212> DNA
   <213> Mus musculus
<220>
   <221> CDS
   <222> (615)..(1616)
<400> 18
   agcaactttc ctgacccaga ggaccggtaa caagtggccg ggagcaactt ttgcaaatct 60
   cttctgcgcc ttaaggctgc caccgagact gtaaagaaaa ggagaagagg aacctatact 120
   cataccagtt cgcacaggcc taagttgggc gaggcctagc cgcggctgcc tagcgtcccc 180
   ccccccctca cagcggagga ggggacagtt gttggaggcc gggcggcaga cccgatcgcg 240
   ggcctccacc gagaattccg tgacgactgg tcagcaccgc cggagagccg ctgttgctgg 300
   gactggtctg cgggctccaa ggaaccgctg ctccccgaga gcgctccgtg agtgaccgcg 360
   acttttcaaa gctcggcatc gcgcggagtc ctaccaacgt gagtgctagc ggagtcttaa 420
   ccctgcgctc cctggagcga actggggagg agggctcagg gggaagcact gccgtctgga 480
   gcgcacgctc taaacaaact ttgttacaga agcagggacg cgcgggtatc cccccgcttc 540
   ccggcgcgct gttgcggccc cgaaacttct gcgcacagcc caggctaacc ccgcgtgaag     600
   tgacggaccg ttct atg act gca aag atg gaa acg acc ttc tac gac gat       650
                   Met Thr Ala Lys Met Glu Thr Thr Phe Tyr Asp Asp
                   1               5                   10
   gcc ctc aac gcc tcg ttc ctc cag tcc gag agc ggt gcc tac ggc tac       698
   Ala Leu Asn Ala Ser Phe Leu Gln Ser Glu Ser Gly Ala Tyr Gly Tyr
           15                  20                  25
   agt aac cct aag atc cta aaa cag agc atg acc ttg aac ctg gcc gac       746
   Ser Asn Pro Lys Ile Leu Lys Gln Ser Met Thr Leu Asn Leu Ala Asp
       30                  35                  40
   ccg gtg ggc agt ctg aag ccg cac ctc cgc gcc aag aac tcg gac ctt       794
   Pro Val Gly Ser Leu Lys Pro His Leu Arg Ala Lys Asn Ser Asp Leu
   45                  50                  55                  60
   ctc acg tcg ccc gac gtc ggg ctg ctc aag ctg gcg tcg ccg gag ctg       842
   Leu Thr Ser Pro Asp Val Gly Leu Leu Lys Leu Ala Ser Pro Glu Leu
                   65                  70                  75
   gag cgc ctg atc atc cag tcc agc aat ggg cac atc acc act aca ccg       890
   Glu Arg Leu Ile Ile Gln Ser Ser Asn Gly His Ile Thr Thr Thr Pro
               80                  85                  90
   acc ccc acc cag ttc ttg tgc ccc aag aac gtg acc gac gag cag gag       938
   Thr Pro Thr Gln Phe Leu Cys Pro Lys Asn Val Thr Asp Glu Gln Glu
           95                  100                 105
   ggc ttc gcc gag ggc ttc gtg cgc gcc ctg gct gaa ctg cat agc cag       986
   Gly Phe Ala Glu Gly Phe Val Arg Ala Leu Ala Glu Leu His Ser Gln
       110                 115                 120
   aac acg ctt ccc agt gtc acc tcc gcg gca cag ccg gtc agc ggg gcg      1034
   Asn Thr Leu Pro Ser Val Thr Ser Ala Ala Gln Pro Val Ser Gly Ala
   125                 130                 135                 140
   ggc atg gtg gct ccc gcg gtg gcc tca gta gca ggc gct ggc ggc ggt      1082
   Gly Met Val Ala Pro Ala Val Ala Ser Val Ala Gly Ala Gly Gly Gly
                   145                 150                 155
   ggt ggc tac agc gcc agc ctg cac agt gag cct ccg gtc tac gcc aac      1130
   Gly Gly Tyr Ser Ala Ser Leu His Ser Glu Pro Pro Val Tyr Ala Asn
               160                 165                 170
   ctc agc aac ttc aac ccg ggt gcg ctg agc agc ggc ggt ggg gcg ccc      1178
   Leu Ser Asn Phe Asn Pro Gly Ala Leu Ser Ser Gly Gly Gly Ala Pro
           175                 180                 185
   tcc tat ggc gcg gcc ggg ctg gcc ttt ccc tcg cag ccg cag cag cag      1226
   Ser Tyr Gly Ala Ala Gly Leu Ala Phe Pro Ser Gln Pro Gln Gln Gln
       190                 195                 200
   cag cag ccg cct cag ccg ccg cac cac ttg ccc caa cag atc ccg gtg      1274
   Gln Gln Pro Pro Gln Pro Pro His His Leu Pro Gln Gln Ile Pro Val
   205                 210                 215                 220
   cag cac ccg cgg ctg caa gcc ctg aag gaa gag ccg cag acc gtg ccg      1322
   Gln His Pro Arg Leu Gln Ala Leu Lys Glu Glu Pro Gln Thr Val Pro
                   225                 230                 235
   gag atg ccg gga gag acg ccg ccc ctg tcc cct atc gac atg gag tct      1370
   Glu Met Pro Gly Glu Thr Pro Pro Leu Ser Pro Ile Asp Met Glu Ser
               240                 245                 250
   cag gag cgg atc aag gca gag agg aag cgc atg agg aac cgc att gcc      1418
   Gln Glu Arg Ile Lys Ala Glu Arg Lys Arg Met Arg Asn Arg Ile Ala
           255                 260                 265
   gcc tcc aag tgc cgg aaa agg aag ctg gag cgg atc gct cgg cta gag      1466
   Ala Ser Lys Cys Arg Lys Arg Lys Leu Glu Arg Ile Ala Arg Leu Glu
       270                 275                 280
   gaa aaa gtg aaa acc ttg aaa gcg caa aac tcc gag ctg gca tcc acg      1514
   Glu Lys Val Lys Thr Leu Lys Ala Gln Asn Ser Glu Leu Ala Ser Thr
   285                 290                 295                 300
   gcc aac atg ctc agg gaa cag gtg gca cag ctt aag cag aaa gtc atg      1562
   Ala Asn Met Leu Arg Glu Gln Val Ala Gln Leu Lys Gln Lys Val Met
                   305                 310                 315
   aac cac gtt aac agt ggg tgc caa ctc atg cta acg cag cag ttg caa      1610
   Asn His Val Asn Ser Gly Cys Gln Leu Met Leu Thr Gln Gln Leu Gln
               320                 325                 330
   acg ttt tgagaacaga ctgtcagggc tgaggggcaa tggaagaaaa aaaataacag       1666
   Thr Phe
   agacaaactt gagaacttga ctggttgcga cagagaaaaa aaaagtgtcc gagtactgaa   1726
   gccaagggta cacaagatgg actgggttcg gactgacggc gcccccagtg tgctctggag   1786
   tgggaaggac gtggcgcgcc tggctttggc gtggagccag agagcaggcc tattggccgg   1846
   cagactttgc ggagcgctgt gccgcgcgcg accagaacga tggacttttc gttaacattg   1906
   accaagaact gcatggacct aacattcgat ctcattcagt attaaagggg ggtgggaggg   1966
   gttacaaact gcaatagaga ctgtagattg cttctgtagt gctccttaac acaaagcagg   2026
   gagggctggg aaggggggga ggcttgtaag tgccaggcta gactgcagat gaactcccct   2086
   ggcctgcctc tctcaactgt gtatgtacat atatattttt ttttaatttg atgaaagctg   2146
   attactgtca ataaacagct tcctgccttt gtaagttatt ccatgtttgt ttgtttgggt   2206
   gtcctgccca gtgtttgtaa ataagagatt tgaagcattc tgagtttacc atttgtaata   2266
   aagtatataa tttttttatg ttttgtttct gaaaatttcc agaaaggata tttaagaaaa   2326
   tacaataaac tattgaaaag t                                             2347
<210> 19
   <211> 334
   <212> PRT
   <213> Mus musculus
<400> 19
   Met Thr Ala Lys Met Glu Thr Thr Phe Tyr Asp Asp Ala Leu Asn Ala
   1 5 10 15
   Ser Phe Leu Gln Ser Glu Ser Gly Ala Tyr Gly Tyr Ser Asn Pro Lys
   20 25 30
   Ile Leu Lys Gln Ser Met Thr Leu Asn Leu Ala Asp Pro Val Gly Ser
   35 40 45
   Leu Lys Pro His Leu Arg Ala Lys Asn Ser Asp Leu Leu Thr Ser Pro
   50 55 60
   Asp Val Gly Leu Leu Lys Leu Ala Ser Pro Glu Leu Glu Arg Leu Ile
   65 70 75 80
   Ile Gln Ser Ser Asn Gly His Ile Thr Thr Thr Pro Thr Pro Thr Gln
   85 90 95
   Phe Leu Cys Pro Lys Asn Val Thr Asp Glu Gln Glu Gly Phe Ala Glu
   100 105 110
   Gly Phe Val Arg Ala Leu Ala Glu Leu His Ser Gln Asn Thr Leu Pro
   115 120 125
   Ser Val Thr Ser Ala Ala Gln Pro Val Ser Gly Ala Gly Met Val Ala
   130 135 140
   Pro Ala Val Ala Ser Val Ala Gly Ala Gly Gly Gly Gly Gly Tyr Ser
   145 150 155 160
   Ala Ser Leu His Ser Glu Pro Pro Val Tyr Ala Asn Leu Ser Asn Phe
   165 170 175
   Asn Pro Gly Ala Leu Ser Ser Gly Gly Gly Ala Pro Ser Tyr Gly Ala
   180 185 190
   Ala Gly Leu Ala Phe Pro Ser Gln Pro Gln Gln Gln Gln Gln Pro Pro
   195 200 205
   Gln Pro Pro His His Leu Pro Gln Gln Ile Pro Val Gln His Pro Arg
   210 215 220
   Leu Gln Ala Leu Lys Glu Glu Pro Gln Thr Val Pro Glu Met Pro Gly
   225 230 235 240
   Glu Thr Pro Pro Leu Ser Pro Ile Asp Met Glu Ser Gln Glu Arg Ile
   245 250 255
   Lys Ala Glu Arg Lys Arg Met Arg Asn Arg Ile Ala Ala Ser Lys Cys
   260 265 270
   Arg Lys Arg Lys Leu Glu Arg Ile Ala Arg Leu Glu Glu Lys Val Lys
   275 280 285
   Thr Leu Lys Ala Gln Asn Ser Glu Leu Ala Ser Thr Ala Asn Met Leu
   290 295 300
   Arg Glu Gln Val Ala Gln Leu Lys Gln Lys Val Met Asn His Val Asn
   305 310 315 320
   Ser Gly Cys Gln Leu Met Leu Thr Gln Gln Leu Gln Thr Phe
   325 330
<210> 20
   <211> 334
   <212> PRT
   <213> Mus musculus
<400> 20
   Met Thr Ala Lys Met Glu Thr Thr Phe Tyr Asp Asp Ala Leu Asn Ala
   1               5                   10                  15
   Ser Phe Leu Gln Ser Glu Ser Gly Ala Tyr Gly Tyr Ser Asn Pro Lys
               20                  25                  30
   Ile Leu Lys Gln Ser Met Thr Leu Asn Leu Ala Asp Pro Val Gly Ser
           35                  40                  45
   Leu Lys Pro His Leu Arg Ala Lys Asn Ser Asp Leu Leu Thr Ser Pro
       50                  55                  60
   Asp Val Gly Leu Leu Lys Leu Ala Ser Pro Glu Leu Glu Arg Leu Ile
   65                  70                  75                  80
   Ile Gln Ser Ser Asn Gly His Ile Thr Thr Thr Pro Thr Pro Thr Gln
                   85                  90                  95
   Phe Leu Cys Pro Lys Asn Val Thr Asp Glu Gln Glu Gly Phe Ala Glu
   100 105 110
   Gly Phe Val Arg Ala Leu Ala Glu Leu His Ser Gln Asn Thr Leu Pro
   115 120 125
   Ser Val Thr Ser Ala Ala Gln Pro Val Ser Gly Ala Gly Met Val Ala
   130 135 140
   Pro Ala Val Ala Ser Val Ala Gly Ala Gly Gly Gly Gly Gly Tyr Ser
   145 150 155 160
   Ala Ser Leu His Ser Glu Pro Pro Val Tyr Ala Asn Leu Ser Asn Phe
   165 170 175
   Asn Pro Gly Ala Leu Ser Ser Gly Gly Gly Ala Pro Ser Tyr Gly Ala
   180 185 190
   Ala Gly Leu Ala Phe Pro Ser Gln Pro Gln Gln Gln Gln Gln Pro Pro
   195 200 205
   Gln Pro Pro His His Leu Pro Gln Gln Ile Pro Val Gln His Pro Arg
   210 215 220
   Leu Gln Ala Leu Lys Glu Glu Pro Gln Thr Val Pro Glu Met Pro Gly
   225 230 235 240
   Glu Thr Pro Pro Leu Ser Pro Ile Asp Met Glu Ser Gln Glu Arg Ile
   245 250 255
   Lys Ala Glu Arg Lys Arg Met Arg Asn Arg Ile Ala Ala Ser Lys Cys
   260 265 270
   Arg Lys Arg Lys Leu Glu Arg Ile Ala Arg Leu Glu Glu Lys Val Lys
   275 280 285
   Thr Leu Lys Ala Gln Asn Ser Glu Leu Ala Ser Thr Ala Asn Met Leu
   290 295 300
   Arg Glu Gln Val Ala Gln Leu Lys Gln Lys Val Met Asn His Val Asn
   305 310 315 320
   Ser Gly Cys Gln Leu Met Leu Thr Gln Gln Leu Gln Thr Phe
   325 330
<210> 21
   <211> 3967
   <212> DNA
   <213> Mus musculus
<400> 21
   ataccagaga ctcaaaaaaa aaaaaaaagt tccagattgc tggacaatga cccgggtctc     60
   atcccttgac cctgggaacc gggtccacat tgaatcaggt gcgaatgttc gctcgccttc    120
   tctgcctttc ccgcctcccc tcccccggcc gcggccccgg ttccccccct gcgctgcacc   180
   ctcagagttg gctgcagccg gcgagctgtt cccgtcaatc cctccctcct ttacacagga    240
   tgtccatatt aggacatctg cgtcagcagg tttccacggc cggtccctgt tgttctgggg    300
   gggggaccat ctccgaaatc ctacacgcgg aaggtctagg agacccccta agatcccaaa    360
   tgtgaacact cataggtgaa agatgtatgc caagacgggg gttgaaagcc tggggcgtag    420
   agttgacgac agagcgcccg cagagggcct tggggcgcgc ttcccccccc ttccagttcc    480
   gcccagtgac gtaggaagtc catccattca cagcgcttct ataaaggcgc cagctgaggc    540
   gcctactact ccaaccgcga ctgcagcgag caactgagaa gactggatag agccggcggt    600
   tccgcgaacg agcagtgacc gcgctcccac ccagctctgc tctgcagctc ccaccagtgt    660
   ctacccctgg accccttgcc gggctttccc caaacttcga ccatgatgtt ctcgggtttc    720
   aacgccgact acgaggcgtc atcctcccgc tgcagtagcg cctccccggc cggggacagc    780
   ctttcctact accattcccc agccgactcc ttctccagca tgggctctcc tgtcaacaca    840
   caggtgagtt tggctttgtg tagccgccag gtccgcgctg agggtcgccg tggaggagac    900
   actggggtgt gactcgcagg ggcggggggg tcttcctttt tcgctctgga gggagactgg    960
   cgcggtcaga gcagccttag cctgggaacc caggacttgt ctgagcgcgt gcacacttgt   1020
   catagtaaga cttagtgacc ccttcccgcg cggcaggttt attctgagtg gcctgcctgc   1080
   attcttctct cggccgactt gtttctgaga tcagccgggg ccaacaagtc tcgagcaaag   1140
   agtcgctaac tagagtttgg gaggcggcaa accgcggcaa tcccccctcc cggggcagcc  1200
   tggagcaggg aggagggagg agggaggagg gtgctgcggg cgggtgtgta aggcagtttc   1260
   attgataaaa agcgagttca ttctggagac tccggagcag cgcctgcgtc agcgcagacg   1320
   tcagggatat ttataacaaa ccccctttcg agcgagtgat gccgaaggga taacgggaac    1380
   gcagcagtag gatggaggag aaaggctgcg ctgcggaatt caagggagga tattgggaga   1440
   gcttttatct ccgatgaggt gcatacagga agacataagc agtctctgac cggaatgctt   1500
   ctctctccct gcttcatgcg acactagggc cacttgctcc acctgtgtct ggaacctcct   1560
   cgctcacctc cgctttcctc tttttgtttt gtttcaggac ttttgcgcag atctgtccgt 1620
   ctctagtgcc aactttatcc ccacggtgac agccatctcc accagcccag acctgcagtg 1680
   gctggtgcag cccactctgg tctcctccgt ggccccatcg cagaccagag cgccccatcc 1740
   ttacggactc cccacccagt ctgctggggc ttacgccaga gcgggaatgg tgaagaccgt 1800
   gtcaggaggc agagcgcaga gcatcggcag aaggggcaaa gtagagcagg tgagcagcga 1860
   ttctggacct ttgtgggctg gggggggggg ggggggcgga gactgacgca cagaccacac 1920
   aacagagaag ggacgctact gactgcactt cctgaccagg agctgtggct gctagccctt 1980
   tccctccctt gtcagatttt gacagttgga cccaagacaa actctagaca gtttccctga 2040
   cagcttccta cttcattctc tagccgggga gcttctttgt tcccctgcta aagatctcac 2100
   tttaaatgca aatcacactc tgcctgccaa ctgcaggtta gaaaaactgc ttcaccgaga 2160
   ggtgcgggtg ctgtaggagc cagtttcact ggggtgactg aatggaggtg acactagaca 2220
   accttaactg aatgttggtc cttttcttct atagctatct cctgaagagg aagagaaacg 2280
   gagaatccga agggaacgga ataagatggc tgcagccaag tgccggaatc ggaggaggga 2340
   gctgacagat acactccaag cggtaggttg aaccagctgc tgctcctgaa actttattaa 2400
   agttggagct tgggactatg ggcgcagggt ccttgagcat gcccgtgtct tatgctttct 2460
   tatatctctc cctatgcagg agacagatca acttgaagat gagaagtctg cgttgcagac 2520
   tgagattgcc aatctgctga aagagaagga aaaactggag tttattttgg cagcccaccg 2580
   acctgcctgc aagatccccg atgaccttgg cttcccagag gagatgtctg tggcctccct 2640
   ggatttgact ggaggtctgc ctgaggcttc caccccagag tctgaggagg ccttcaccct 2700
   gccccttctc aacgaccctg agcccaagcc atccttggag ccagtcaaga gcatcagcaa 2760
   cgtggagctg aaggcagaac cctttgatga cttcttgttt ccggcatcat ctaggcccag 2820
   tggctcagag acctcccgct ctgtgccaga tgtggacctg tccggttcct tctatgcagc 2880
   agactgggag cctctgcaca gcaattcctt ggggatgggg cccatggtca cagagctgga 2940
   gcccctgtgt actcccgtgg tcacctgtac tccgggctgc actacttaca cgtcttcctt 3000
   tgtcttcacc taccctgaag ctgactcctt cccaagctgt gccgctgccc accgaaaggg 3060
   cagcagcagc aacgagccct cctccgactc cctgagctca cccacgctgc tggccctgtg 3120
   agcagtcaga gaaggcaagg cagccggcat ccagacgtgc cactgcccga gctggtgcat 3180
   tacagagagg agaaacacgt cttccctcga aggttcccgt cgacctaggg aggaccttac 3240
   ctgttcgtga aacacaccag gctgtgggcc tcaaggactt gcaagcatcc acatctggcc 3300
   tccagtcctc acctcttcca gagatgtagc aaaaacaaaa caaaacaaaa caaaaaaccg   3360
   catggagtgt gttgttccta gtgacacctg agagctggta gttagtagag catgtgagtc   3420
   aaggcctggt ctgtgtctct tttctctttc tccttagttt tctcatagca ctaactaatc   3480
   tgttgggttc attattggaa ttaacctggt gctggattgt atctagtgca gctgatttta   3540
   acaataccta ctgtgttcct ggcaatagcg tgttccaatt agaaacgacc aatattaaac   3600
   taagaaaaga taggacttta ttttccagta gatagaaatc aatagctata tccatgtact   3660
   gtagtccttc agcgtcaatg ttcattgtca tgttactgat catgcattgt cgaggtggtc   3720
   tgaatgttct gacattaaca gttttccatg aaaacgtttt tattgtgttt tcaatttatt   3780
   tattaagatg gattctcaga tatttatatt tttattttat ttttttctac cctgaggtct   3840
   ttcgacatgt ggaaagtgaa tttgaatgaa aaattttaag cattgtttgc ttattgttcc   3900
   aggacattgt caataaaagc atttaagttg aatgcgacca ccttcttgct ctctttattc   3960
   tcagttt                                                             3967
<210> 22
   <211> 380
   <212> PRT
   <213> Mus musculus
<400> 22
   Met Met Phe Ser Gly Phe Asn Ala Asp Tyr Glu Ala Ser Ser Ser Arg
   1               5                   10                  15
   Cys Ser Ser Ala Ser Pro Ala Gly Asp Ser Leu Ser Tyr Tyr His Ser
               20                  25                  30
   Pro Ala Asp Ser Phe Ser Ser Met Gly Ser Pro Val Asn Thr Gln Asp
           35                  40                  45
   Phe Cys Ala Asp Leu Ser Val Ser Ser Ala Asn Phe Ile Pro Thr Val
       50                  55                  60
   Thr Ala Ile Ser Thr Ser Pro Asp Leu Gln Trp Leu Val Gln Pro Thr
   65                  70                  75                  80
   Leu Val Ser Ser Val Ala Pro Ser Gln Thr Arg Ala Pro His Pro Tyr
                   85                  90                  95
   Gly Leu Pro Thr Gln Ser Ala Gly Ala Tyr Ala Arg Ala Gly Met Val
               100                 105                 110
   Lys Thr Val Ser Gly Gly Arg Ala Gln Ser Ile Gly Arg Arg Gly Lys
   115 120 125
   Val Glu Gln Leu Ser Pro Glu Glu Glu Glu Lys Arg Arg Ile Arg Arg
   130 135 140
   Glu Arg Asn Lys Met Ala Ala Ala Lys Cys Arg Asn Arg Arg Arg Glu
   145 150 155 160
   Leu Thr Asp Thr Leu Gln Ala Glu Thr Asp Gln Leu Glu Asp Glu Lys
   165 170 175
   Ser Ala Leu Gln Thr Glu Ile Ala Asn Leu Leu Lys Glu Lys Glu Lys
   180 185 190
   Leu Glu Phe Ile Leu Ala Ala His Arg Pro Ala Cys Lys Ile Pro Asp
   195 200 205
   Asp Leu Gly Phe Pro Glu Glu Met Ser Val Ala Ser Leu Asp Leu Thr
   210 215 220
   Gly Gly Leu Pro Glu Ala Ser Thr Pro Glu Ser Glu Glu Ala Phe Thr
   225 230 235 240
   Leu Pro Leu Leu Asn Asp Pro Glu Pro Lys Pro Ser Leu Glu Pro Val
   245 250 255
   Lys Ser Ile Ser Asn Val Glu Leu Lys Ala Glu Pro Phe Asp Asp Phe
   260 265 270
   Leu Phe Pro Ala Ser Ser Arg Pro Ser Gly Ser Glu Thr Ser Arg Ser
   275 280 285
   Val Pro Asp Val Asp Leu Ser Gly Ser Phe Tyr Ala Ala Asp Trp Glu
   290 295 300
   Pro Leu His Ser Asn Ser Leu Gly Met Gly Pro Met Val Thr Glu Leu
   305 310 315 320
   Glu Pro Leu Cys Thr Pro Val Val Thr Cys Thr Pro Gly Cys Thr Thr
   325 330 335
   Tyr Thr Ser Ser Phe Val Phe Thr Tyr Pro Glu Ala Asp Ser Phe Pro
   340 345 350
   Ser Cys Ala Ala Ala His Arg Lys Gly Ser Ser Ser Asn Glu Pro Ser
   355 360 365
   Ser Asp Ser Leu Ser Ser Pro Thr Leu Leu Ala Leu
   370 375 380
<210> 23
   <211> 30
   <212> DNA
   <213> Artificial
<220>
   <223> primer
<400> 23
   aagaaatgga ggctcataaa taccacagct 30
<210> 24
   <211> 30
   <212> DNA
   <213> Artificial
<220>
   <223> primer
<400> 24
   attggaagaa gaccaaagca agagcaacta 30
<210> 25
   <211> 30
   <212> DNA
   <213> Artificial
<220>
   <223> primer
<400> 25
   taagtaagta gtgctgtatg aatacagaca 30
<210> 26
   <211> 30
   <212> DNA
   <213> Artificial
<220>
   <223> primer
<400> 26
   tactggcaat atattacaga tgggtttatg 30
<210> 27
   <211> 28
   <212> DNA
   <213> Artificial
<220>
   <223> primer
<400> 27
   gtgtgcagct tatgatgcta ttcttgaa 28
<210> 28
   <211> 24
   <212> DNA
   <213> Artificial
<220>
   <223> primer
<400> 28
   cgcgtcacca catacggagt catc 24
<210> 29
   <211> 25
   <212> DNA
   <213> Artificial
<220>
   <223> primer
<400> 29
   ctggaggagt tccaagatgt ctact 25
<210> 30
   <211> 24
   <212> .DNA
   <213> Artificial
<220>
   <223> primer
<400> 30
   tggaaagagc ttggggaagg tgag 24